# EUROPEAN PATENT SPECIFICATION

(11) **EP 4 097 478 B1**
(45) Date of publication and mention of the grant of the patent: **27.08.2025**
(21) Application number: 21709817.7
(22) Date of filing: 29.01.2021
(51) Int. Cl.: G01N 33/574, G01N 33/68

(54) **BIOMARKERS FOR DIAGNOSING OVARIAN CANCER**
BIOMARKER ZUR DIAGNOSE EINES OVARIALKARZINOMS
BIOMARQUEURS POUR LE DIAGNOSTIC DU CANCER DE L'OVAIRE

(30) Priority: 31.01.2020 US 202062968941 P
(43) Date of publication of application: 07.12.2022
(73) Proprietor: Venn Biosciences Corporation, South San Francisco, CA 94080 (US)
(72) Inventor: XU, Gege, San Francisco, California 94080 (US); SHEN, Ling, San Francisco, California 94080 (US); XU, Hui, San Francisco, California 94080 (US); SERIE, Daniel, San Francisco, California 94080 (US)
(74) Representative: WSL Patentanwälte Partnerschaft mbB
(86) International application number: PCT/US2021/015915
(87) International publication number: WO 2021/155300

(56) References cited:
- MIYAMOTO SUZANNE ET AL: "Multiple Reaction Monitoring for the Quantitation of Serum Protein Glycosylation Profiles: Application to Ovarian Cancer", JOURNAL OF PROTEOME RESEARCH, vol. 17, no. 1, 5 January 2018 (2018-01-05), pages 222 - 233, XP055809570, ISSN: 1535-3893, Retrieved from the Internet <URL:https://pubs.acs.org/doi/pdf/10.1021/acs.jproteome.7b00541> DOI: 10.1021/acs.jproteome.7b00541
- RUHAAK L. RENEE ET AL: "Protein-Specific Differential Glycosylation of Immunoglobulins in Serum of Ovarian Cancer Patients", JOURNAL OF PROTEOME RESEARCH, vol. 15, no. 3, 4 March 2016 (2016-03-04), pages 1002 - 1010, XP055809787, ISSN: 1535-3893, Retrieved from the Internet <URL:https://pubs.acs.org/doi/pdf/10.1021/acs.jproteome.5b01071> DOI: 10.1021/acs.jproteome.5b01071
- QIONGYU LI ET AL: "Site-Specific Glycosylation Quantitation of 50 Serum Glycoproteins Enhanced by Predictive Glycopeptidomics for Improved Disease Biomarker Discovery", ANALYTICAL CHEMISTRY, vol. 91, no. 8, 18 March 2019 (2019-03-18), US, pages 5433 - 5445, XP055689784, ISSN: 0003-2700, DOI: 10.1021/acs.analchem.9b00776
- ZHOU SHIYUE ET AL: "Quantitation of PermethylatedN-Glycans through Multiple-Reaction Monitoring (MRM) LC-MS/MS", JOURNAL OF THE AMERICAN SOCIETY FOR MASS SPECTROMETRY, ELSEVIER SCIENCE INC, US, vol. 26, no. 4, 20 February 2015 (2015-02-20), pages 596 - 603, XP035469428, ISSN: 1044-0305, [retrieved on 20150220], DOI: 10.1007/S13361-014-1054-1
- VIVEKANANDA SHETTY ET AL: "Investigation of ovarian cancer associated sialylation changes in N-linked glycopeptides by quantitative proteomics", CLINICAL PROTEOMICS, BIOMED CENTRAL LTD, LONDON, UK, vol. 9, no. 1, 2 August 2012 (2012-08-02), pages 10, XP021121965, ISSN: 1559-0275, DOI: 10.1186/1559-0275-9-10

### SEQUENCE LISTING

<110> DANIEL SERIE
   VENN BIOSCIENCES CORPORATION
<120> BIOMARKERS FOR DIAGNOSING OVARIAN CANCER
<130> 119759.00018
<160> 76
<170> PatentIn version 3.5
<210> 1
   <211> 16
   <212> PRT
   <213> Artificial Sequence
<220>
   <223> Synthetic: A1AT-GP001_271_5402
<400> 1
   Tyr Leu Gly Asn Ala Thr Ala Ile Phe Phe Leu Pro Asp Glu Gly Lys
   1               5                   10                  15
<210> 2
   <211> 16
   <212> PRT
   <213> Artificial Sequence
<220>
   <223> Synthetic: A1AT-GP001_271_5412
<400> 2
   Tyr Leu Gly Asn Ala Thr Ala Ile Phe Phe Leu Pro Asp Glu Gly Lys
   1               5                   10                  15
<210> 3
   <211> 31
   <212> PRT
   <213> Artificial Sequence
<220>
   <223> Synthetic: A1AT-GP001_271MC_5402
<400> 3
   Tyr Leu Gly Asn Ala Thr Ala Ile Phe Phe Leu Pro Asp Glu Gly Lys
   1               5                   10                  15
   Leu Gln His Leu Glu Asn Glu Leu Thr His Asp Ile Ile Thr Lys
               20                  25                  30
<210> 4
   <211> 33
   <212> PRT
   <213> Artificial Sequence
<220>
   <223> Synthetic: A1BG-GP002_179_5421/5402
<400> 4
   Glu Gly Asp His Glu Phe Leu Glu Val Pro Glu Ala Gln Glu Asp Val
   1               5                   10                  15
   Glu Ala Thr Phe Pro Val His Gln Pro Gly Asn Tyr Ser Cys Ser Tyr
               20                  25                  30
   Arg
<210> 5
   <211> 19
   <212> PRT
   <213> Artificial Sequence
<220>
   <223> Synthetic: A2MG-GP004_1424_5402
<400> 5
   Val Ser Asn Gln Thr Leu Ser Leu Phe Phe Thr Val Leu Gln Asp Val
   1               5                   10                  15
   Pro Val Arg
<210> 6
   <211> 19
   <212> PRT
   <213> Artificial Sequence
<220>
   <223> Synthetic: A2MG-GP004_1424_5411
<400> 6
   Val Ser Asn Gln Thr Leu Ser Leu Phe Phe Thr Val Leu Gln Asp Val
   1 5 10 15
   Pro Val Arg
<210> 7
   <211> 22
   <212> PRT
   <213> Artificial Sequence
<220>
   <223> Synthetic: A2MG-GP004_55_5401
<400> 7
   Gly Cys Val Leu Leu Ser Tyr Leu Asn Glu Thr Val Thr Val Ser Ala
   1 5 10 15
   Ser Leu Glu Ser Val Arg
   20
<210> 8
   <211> 22
   <212> PRT
   <213> Artificial Sequence
<220>
   <223> Synthetic: A2MG-GP004_55_5402
<400> 8
   Gly Cys Val Leu Leu Ser Tyr Leu Asn Glu Thr Val Thr Val Ser Ala
   1 5 10 15
   Ser Leu Glu Ser Val Arg
   20
<210> 9
   <211> 22
   <212> PRT
   <213> Artificial Sequence
<220>
   <223> Synthetic: A2MG-GP004_55_5411
<400> 9
   Gly Cys Val Leu Leu Ser Tyr Leu Asn Glu Thr Val Thr Val Ser Ala
   1 5 10 15
   Ser Leu Glu Ser Val Arg
   20
<210> 10
   <211> 32
   <212> PRT
   <213> Artificial Sequence
<220>
   <223> Synthetic: A2MG-GP004_869_5200
<400> 10
   Ser Leu Gly Asn Val Asn Phe Thr Val Ser Ala Glu Ala Leu Glu Ser
   1 5 10 15
   Gln Glu Leu Cys Gly Thr Glu Val Pro Ser Val Pro Glu His Gly Arg
   20 25 30
<210> 11
   <211> 32
   <212> PRT
   <213> Artificial Sequence
<220>
   <223> Synthetic: A2MG-GP004_869_5401
<400> 11
   Ser Leu Gly Asn Val Asn Phe Thr Val Ser Ala Glu Ala Leu Glu Ser
   1 5 10 15
   Gln Glu Leu Cys Gly Thr Glu Val Pro Ser Val Pro Glu His Gly Arg
   20 25 30
<210> 12
   <211> 32
   <212> PRT
   <213> Artificial Sequence
<220>
   <223> Synthetic: A2MG-GP004_869_6301
<400> 12
   Ser Leu Gly Asn Val Asn Phe Thr Val Ser Ala Glu Ala Leu Glu Ser
   1 5 10 15
   Gln Glu Leu Cys Gly Thr Glu Val Pro Ser Val Pro Glu His Gly Arg
   20 25 30
<210> 13
   <211> 10
   <212> PRT
   <213> Artificial Sequence
<220>
   <223> Synthetic: AFAM-GP006_33_5402
<400> 13
   Asp Ile Glu Asn Phe Asn Ser Thr Gln Lys
   1 5 10
<210> 14
   <211> 24
   <212> PRT
   <213> Artificial Sequence
<220>
   <223> Synthetic: AGP12-GP007&008_72MC_6503
<400> 14
   Ser Val Gln Glu Ile Gln Ala Thr Phe Phe Tyr Phe Thr Pro Asn Lys
   1 5 10 15
   Thr Glu Asp Thr Ile Phe Leu Arg
   20
<210> 15
   <211> 24
   <212> PRT
   <213> Artificial Sequence
<220>
   <223> Synthetic: AGP12-GP007&008_72MC_6513
<400> 15
   Ser Val Gln Glu Ile Gln Ala Thr Phe Phe Tyr Phe Thr Pro Asn Lys
   1 5 10 15
   Thr Glu Asp Thr Ile Phe Leu Arg
   20
<210> 16
   <211> 24
   <212> PRT
   <213> Artificial Sequence
<220>
   <223> Synthetic: AGP12-GP007&008_72MC_7601
<400> 16
   Ser Val Gln Glu Ile Gln Ala Thr Phe Phe Tyr Phe Thr Pro Asn Lys
   1 5 10 15
   Thr Glu Asp Thr Ile Phe Leu Arg
   20
<210> 17
   <211> 24
   <212> PRT
   <213> Artificial Sequence
<220>
   <223> Synthetic: AGP12-GP007&008 72MC 7602
<400> 17
   Ser Val Gln Glu Ile Gln Ala Thr Phe Phe Tyr Phe Thr Pro Asn Lys
   1 5 10 15
   Thr Glu Asp Thr Ile Phe Leu Arg
   20
<210> 18
   <211> 24
   <212> PRT
   <213> Artificial Sequence
<220>
   <223> Synthetic: AGP12-GP007&008_72MC_7603
<400> 18
   Ser Val Gln Glu Ile Gln Ala Thr Phe Phe Tyr Phe Thr Pro Asn Lys
   1 5 10 15
   Thr Glu Asp Thr Ile Phe Leu Arg
   20
<210> 19
   <211> 24
   <212> PRT
   <213> Artificial Sequence
<220>
   <223> Synthetic: AGP12-GP007&008_72MC_7613
<400> 19
   Ser Val Gln Glu Ile Gln Ala Thr Phe Phe Tyr Phe Thr Pro Asn Lys
   1 5 10 15
   Thr Glu Asp Thr Ile Phe Leu Arg
   20
<210> 20
   <211> 24
   <212> PRT
   <213> Artificial Sequence
<220>
   <223> Synthetic: AGP1-GP007_33_5402
<400> 20
   Gln Ile Pro Leu Cys Ala Asn Leu Val Pro Val Pro Ile Thr Asn Ala
   1 5 10 15
   Thr Leu Asp Gln Ile Thr Gly Lys
   20
<210> 21
   <211> 15
   <212> PRT
   <213> Artificial Sequence
<220>
   <223> Synthetic: AGP1-GP007_93_6503/6522
<400> 21
   Gln Asp Gln Cys Ile Tyr Asn Thr Thr Tyr Leu Asn Val Gln Arg
   1 5 10 15
<210> 22
   <211> 15
   <212> PRT
   <213> Artificial Sequence
<220>
   <223> Synthetic: AGP1-GP007_93_6513
<400> 22
   Gln Asp Gln Cys Ile Tyr Asn Thr Thr Tyr Leu Asn Val Gln Arg
   1 5 10 15
<210> 23
   <211> 15
   <212> PRT
   <213> Artificial Sequence
<220>
   <223> Synthetic: AGP1-GP007_93_7603/7622
<400> 23
   Gln Asp Gln Cys Ile Tyr Asn Thr Thr Tyr Leu Asn Val Gln Arg
   1 5 10 15
<210> 24
   <211> 15
   <212> PRT
   <213> Artificial Sequence
<220>
   <223> Synthetic: AGP1-GP007_93_7613
<400> 24
   Gln Asp Gln Cys Ile Tyr Asn Thr Thr Tyr Leu Asn Val Gln Arg
   1 5 10 15
<210> 25
   <211> 19
   <212> PRT
   <213> Artificial Sequence
<220>
   <223> Synthetic: APOC3-GP012_74_1102
<400> 25
   Phe Ser Glu Phe Trp Asp Leu Asp Pro Glu Val Arg Pro Thr Ser Ala
   1               5                   10                  15
   Val Ala Ala
<210> 26
   <211> 21
   <212> PRT
   <213> Artificial Sequence
<220>
   <223> Synthetic: APOC3-GP012_74MC_1102
<400> 26
   Asp Lys Phe Ser Glu Phe Trp Asp Leu Asp Pro Glu Val Arg Pro Thr
   1               5                   10                  15
   Ser Ala Val Ala Ala
               20
<210> 27
   <211> 11
   <212> PRT
   <213> Artificial Sequence
<220>
   <223> Synthetic: APOH-GP015_253_5401
<400> 27
   Leu Gly Asn Trp Ser Ala Met Pro Ser Cys Lys
   1               5                   10
<210> 28
   <211> 16
   <212> PRT
   <213> Artificial Sequence
<220>
   <223> Synthetic: CERU-GP023_138_5412
<400> 28
   Glu His Glu Gly Ala Ile Tyr Pro Asp Asn Thr Thr Asp Phe Gln Arg
   1               5                   10                  15
<210> 29
   <211> 16
   <212> PRT
   <213> Artificial Sequence
<220>
   <223> Synthetic: CERU-GP023_138_5421/5402
<400> 29
   Glu His Glu Gly Ala Ile Tyr Pro Asp Asn Thr Thr Asp Phe Gln Arg
   1               5                   10                  15
<210> 30
   <211> 16
   <212> PRT
   <213> Artificial Sequence
<220>
   <223> Synthetic: CERU-GP023_138_6503/6522
<400> 30
   Glu His Glu Gly Ala Ile Tyr Pro Asp Asn Thr Thr Asp Phe Gln Arg
   1               5                   10                  15
<210> 31
   <211> 18
   <212> PRT
   <213> Artificial Sequence
<220>
   <223> Synthetic: CFAH-GP024 882 5421/5402
<400> 31
   Ile Pro Cys Ser Gln Pro Pro Gln Ile Glu His Gly Thr Ile Asn Ser
   1               5                   10                  15
   Ser Arg
<210> 32
   <211> 21
   <212> PRT
   <213> Artificial Sequence
<220>
   <223> Synthetic: CO3-GP028_85_5200
<400> 32
   Thr Val Leu Thr Pro Ala Thr Asn His Met Gly Asn Val Thr Phe Thr
   1               5                   10                  15
   Ile Pro Ala Asn Arg
               20
<210> 33
   <211> 21
   <212> PRT
   <213> Artificial Sequence
<220>
   <223> Synthetic: CO3-GP028_85_6200
<400> 33
   Thr Val Leu Thr Pro Ala Thr Asn His Met Gly Asn Val Thr Phe Thr
   1               5                   10                  15
   Ile Pro Ala Asn Arg
               20
<210> 34
   <211> 11
   <212> PRT
   <213> Artificial Sequence
<220>
   <223> Synthetic: CO4A&CO4B-GP029&030_1328_5402
<400> 34
   Gly Leu Asn Val Thr Leu Ser Ser Thr Gly Arg
   1               5                   10
<210> 35
   <211> 15
   <212> PRT
   <213> Artificial Sequence
<220>
   <223> Synthetic: FETUA-GP036_156_5402/5421
<400> 35
   Val Cys Gln Asp Cys Pro Leu Leu Ala Pro Leu Asn Asp Thr Arg
   1               5                   10                  15
<210> 36
   <211> 22
   <212> PRT
   <213> Artificial Sequence
<220>
   <223> Synthetic: FETUA-GP036_176_6513
<400> 36
   Ala Ala Leu Ala Ala Phe Asn Ala Gln Asn Asn Gly Ser Asn Phe Gln
   1               5                   10                  15
   Leu Glu Glu Ile Ser Arg
               20
<210> 37
   <211> 21
   <212> PRT
   <213> Artificial Sequence
<220>
   <223> Synthetic: FETUA-GP036_346_1101
<400> 37
   Thr Val Val Gln Pro Ser Val Gly Ala Ala Ala Gly Pro Val Val Pro
   1               5                   10                  15
   Pro Cys Pro Gly Arg
               20
<210> 38
   <211> 13
   <212> PRT
   <213> Artificial Sequence
<220>
   <223> Synthetic: HEMO-GP042_187_5412/5431
<400> 38
   Ser Trp Pro Ala Val Gly Asn Cys Ser Ser Ala Leu Arg
   1               5                   10
<210> 39
   <211> 16
   <212> PRT
   <213> Artificial Sequence
<220>
   <223> Synthetic: HEMO-GP042_453_5420/5401
<400> 39
   Ala Leu Pro Gln Pro Gln Asn Val Thr Ser Leu Leu Gly Cys Thr His
   1               5                   10                  15
<210> 40
   <211> 24
   <212> PRT
   <213> Artificial Sequence
<220>
   <223> Synthetic: HPT-GP044_184_6502
<400> 40
   Met Val Ser His His Asn Leu Thr Thr Gly Ala Thr Leu Ile Asn Glu
   1               5                   10                  15
   Gln Trp Leu Leu Thr Thr Ala Lys
               20
<210> 41
   <211> 13
   <212> PRT
   <213> Artificial Sequence
<220>
   <223> Synthetic: HPT-GP044_207_10803
<400> 41
   Asn Leu Phe Leu Asn His Ser Glu Asn Ala Thr Ala Lys
   1               5                   10
<210> 42
   <211> 13
   <212> PRT
   <213> Artificial Sequence
<220>
   <223> Synthetic: HPT-GP044_207_10804
<400> 42
   Asn Leu Phe Leu Asn His Ser Glu Asn Ala Thr Ala Lys
   1               5                   10
<210> 43
   <211> 13
   <212> PRT
   <213> Artificial Sequence
<220>
   <223> Synthetic: HPT-GP044_207_11904
<400> 43
   Asn Leu Phe Leu Asn His Ser Glu Asn Ala Thr Ala Lys
   1 5 10
<210> 44
   <211> 13
   <212> PRT
   <213> Artificial Sequence
<220>
   <223> Synthetic: HPT-GP044_207_11914
<400> 44
   Asn Leu Phe Leu Asn His Ser Glu Asn Ala Thr Ala Lys
   1 5 10
<210> 45
   <211> 13
   <212> PRT
   <213> Artificial Sequence
<220>
   <223> Synthetic: HPT-GP044_207_11915
<400> 45
   Asn Leu Phe Leu Asn His Ser Glu Asn Ala Thr Ala Lys
   1 5 10
<210> 46
   <211> 16
   <212> PRT
   <213> Artificial Sequence
<220>
   <223> Synthetic: HPT-GP044_241_5401/5420
<400> 46
   Val Val Leu His Pro Asn Tyr Ser Gln Val Asp Ile Gly Leu Ile Lys
   1 5 10 15
<210> 47
   <211> 16
   <212> PRT
   <213> Artificial Sequence
<220>
   <223> Synthetic: HPT-GP044_241_5412
<400> 47
   Val Val Leu His Pro Asn Tyr Ser Gln Val Asp Ile Gly Leu Ile Lys
   1               5                   10                  15
<210> 48
   <211> 16
   <212> PRT
   <213> Artificial Sequence
<220>
   <223> Synthetic: HPT-GP044_241_6501
<400> 48
   Val Val Leu His Pro Asn Tyr Ser Gln Val Asp Ile Gly Leu Ile Lys
   1               5                   10                  15
<210> 49
   <211> 16
   <212> PRT
   <213> Artificial Sequence
<220>
   <223> Synthetic: HPT-GP044_241_6502
<400> 49
   Val Val Leu His Pro Asn Tyr Ser Gln Val Asp Ile Gly Leu Ile Lys
   1               5                   10                  15
<210> 50
   <211> 16
   <212> PRT
   <213> Artificial Sequence
<220>
   <223> Synthetic: HPT-GP044_241_6511
<400> 50
   Val Val Leu His Pro Asn Tyr Ser Gln Val Asp Ile Gly Leu Ile Lys
   1               5                   10                  15
<210> 51
   <211> 16
   <212> PRT
   <213> Artificial Sequence
<220>
   <223> Synthetic: HPT-GP044_241_6513
<400> 51
   Val Val Leu His Pro Asn Tyr Ser Gln Val Asp Ile Gly Leu Ile Lys
   1 5 10 15
<210> 52
   <211> 27
   <212> PRT
   <213> Artificial Sequence
<220>
   <223> Synthetic: IgA12-GP046&047_144_5501
<400> 52
   Leu Ser Leu His Arg Pro Ala Leu Glu Asp Leu Leu Leu Gly Ser Glu
   1 5 10 15
   Ala Asn Leu Thr Cys Thr Leu Thr Gly Leu Arg
   20 25
<210> 53
   <211> 9
   <212> PRT
   <213> Artificial Sequence
<220>
   <223> Synthetic: IgA2-GP047_205_4510
<400> 53
   Thr Pro Leu Thr Ala Asn Ile Thr Lys
   1 5
<210> 54
   <211> 9
   <212> PRT
   <213> Artificial Sequence
<220>
   <223> Synthetic: IgA2-GP047_205_5412
<400> 54
   Thr Pro Leu Thr Ala Asn Ile Thr Lys
   1 5
<210> 55
   <211> 9
   <212> PRT
   <213> Artificial Sequence
<220>
   <223> Synthetic: IgA2-GP047_205_5510
<400> 55
   Thr Pro Leu Thr Ala Asn Ile Thr Lys
   1 5
<210> 56
   <211> 9
   <212> PRT
   <213> Artificial Sequence
<220>
   <223> Synthetic: IgG1-GP048_297_3410
<400> 56
   Glu Glu Gln Tyr Asn Ser Thr Tyr Arg
   1 5
<210> 57
   <211> 9
   <212> PRT
   <213> Artificial Sequence
<220>
   <223> Synthetic: IgG1-GP048_297_4400
<400> 57
   Glu Glu Gln Tyr Asn Ser Thr Tyr Arg
   1 5
<210> 58
   <211> 9
   <212> PRT
   <213> Artificial Sequence
<220>
   <223> Synthetic: IgG1-GP048_297_4510
<400> 58
   Glu Glu Gln Tyr Asn Ser Thr Tyr Arg
   1 5
<210> 59
   <211> 9
   <212> PRT
   <213> Artificial Sequence
<220>
   <223> Synthetic: IgG1-GP048_297_5400
<400> 59
   Glu Glu Gln Tyr Asn Ser Thr Tyr Arg
   1               5
<210> 60
   <211> 9
   <212> PRT
   <213> Artificial Sequence
<220>
   <223> Synthetic: IgG1-GP048_297_5410
<400> 60
   Glu Glu Gln Tyr Asn Ser Thr Tyr Arg
   1               5
<210> 61
   <211> 9
   <212> PRT
   <213> Artificial Sequence
<220>
   <223> Synthetic: IgG1-GP048_297_5411
<400> 61
   Glu Glu Gln Tyr Asn Ser Thr Tyr Arg
   1               5
<210> 62
   <211> 9
   <212> PRT
   <213> Artificial Sequence
<220>
   <223> Synthetic: IgG1-GP048_297_5510
<400> 62
   Glu Glu Gln Tyr Asn Ser Thr Tyr Arg
   1               5
<210> 63
   <211> 9
   <212> PRT
   <213> Artificial Sequence
<220>
   <223> Synthetic: IgG1-GP048_297_nonglycosylated
<400> 63
   Glu Glu Gln Tyr Asn Ser Thr Tyr Arg
   1               5
<210> 64
   <211> 9
   <212> PRT
   <213> Artificial Sequence
<220>
   <223> Synthetic: IgG2-GP049_297_3510
<400> 64
   Glu Glu Gln Phe Asn Ser Thr Phe Arg
   1               5
<210> 65
   <211> 9
   <212> PRT
   <213> Artificial Sequence
<220>
   <223> Synthetic: IgG2-GP049_297_4411
<400> 65
   Glu Glu Gln Phe Asn Ser Thr Phe Arg
   1               5
<210> 66
   <211> 9
   <212> PRT
   <213> Artificial Sequence
<220>
   <223> Synthetic: IgG2-GP049_297_4510
<400> 66
   Glu Glu Gln Phe Asn Ser Thr Phe Arg
   1               5
<210> 67
   <211> 11
   <212> PRT
   <213> Artificial Sequence
<220>
   <223> Synthetic: IgJ-GP052 71 5401
<400> 67
   Glu Asn Ile Ser Asp Pro Thr Ser Pro Leu Arg
   1               5                   10
<210> 68
   <211> 22
   <212> PRT
   <213> Artificial Sequence
<220>
   <223> Synthetic: IgM-GP053_439_6200
<400> 68
   Ser Thr Gly Lys Pro Thr Leu Tyr Asn Val Ser Leu Val Met Ser Asp
   1               5                   10                  15
   Thr Ala Gly Thr Cys Tyr
               20
<210> 69
   <211> 11
   <212> PRT
   <213> Artificial Sequence
<220>
   <223> Synthetic: IgM-GP053_46_4311
<400> 69
   Tyr Lys Asn Asn Ser Asp Ile Ser Ser Thr Arg
   1               5                   10
<210> 70
   <211> 12
   <212> PRT
   <213> Artificial Sequence
<220>
   <223> Synthetic: KNG1-GP057_205_6503
<400> 70
   Ile Thr Tyr Ser Ile Val Gln Thr Asn Cys Ser Lys
   1               5                   10
<210> 71
   <211> 13
   <212> PRT
   <213> Artificial Sequence
<220>
   <223> Synthetic: TRFE-GP064_432_5402
<400> 71
   Cys Gly Leu Val Pro Val Leu Ala Glu Asn Tyr Asn Lys
   1               5                   10
<210> 72
   <211> 21
   <212> PRT
   <213> Artificial Sequence
<220>
   <223> Synthetic: TRFE-GP064_630_5401
<400> 72
   Gln Gln Gln His Leu Phe Gly Ser Asn Val Thr Asp Cys Ser Gly Asn
   1               5                   10                  15
   Phe Cys Leu Phe Arg
               20
<210> 73
   <211> 21
   <212> PRT
   <213> Artificial Sequence
<220>
   <223> Synthetic: TRFE-GP064_630_6513
<400> 73
   Gln Gln Gln His Leu Phe Gly Ser Asn Val Thr Asp Cys Ser Gly Asn
   1               5                   10                  15
   Phe Cys Leu Phe Arg
               20
<210> 74
   <211> 8
   <212> PRT
   <213> Artificial Sequence
<220>
   <223> Synthetic: VTNC-GP067_169_5401
<400> 74
   Asn Gly Ser Leu Phe Ala Phe Arg
   1               5
<210> 75
   <211> 27
   <212> PRT
   <213> Artificial Sequence
<220>
   <223> Synthetic: VTNC-GP067_242_6503
<400> 75
   Asn Ile Ser Asp Gly Phe Asp Gly Ile Pro Asp Asn Val Asp Ala Ala
   1               5                   10                  15
   Leu Ala Leu Pro Ala His Ser Tyr Ser Gly Arg
               20                  25
<210> 76
   <211> 23
   <212> PRT
   <213> Artificial Sequence
<220>
   <223> Synthetic: VTNC-GP067_86_5421
<400> 76
   Asn Asn Ala Thr Val His Glu Gln Val Gly Gly Pro Ser Leu Thr Ser
   1               5                   10                  15
   Asp Leu Gln Ala Gln Ser Lys
               20

## Description

### FIELD

The instant disclosure is directed to glycoproteomic biomarkers including, but not limited to, glycans, peptides, and glycopeptides, as well as to methods of using these biomarkers with mass spectroscopy and in clinical applications.

### BACKGROUND

Changes in glycosylation have been described in relationship to disease states such as cancer. *See, e.g.,* Dube, D. H.; Bertozzi, C. R. Glycans in Cancer and Inflammation - Potential for Therapeutics and Diagnostics. Nature Rev. Drug Disc. 2005, 4, 477-88. However, clinically relevant, non-invasive assays for diagnosing cancer, such as ovarian cancer, in a patient based on glycosylation changes in a sample from that patient are still needed.

Conventional clinical assays for diagnosing ovarian cancer, for example, include measuring the amount of the protein CA 125 (cancer antigen 125) in a patient's blood by an enzyme-linked immunosorbent assay (ELISA). However, ELISA has limited sensitivity and precision. ELISA, for example, only measures CA 125 at concentrations in the ng/mL range. This narrow measurement range limits the relevance of this assay by failing to measure biomarkers at concentrations substantially above or below this concentration range. Also, the
CA 125 ELISA assay is limited with respect to the types of samples which can be assayed. As a consequence of the lack of more precise and sensitive tests, patients who might otherwise be diagnosed with ovarian cancer are not and thereby fail to receive proper follow-up medical attention.

As an alternative, mass spectroscopy (MS) offers sensitive and precise measurement of cancer-specific biomarkers including glycopeptides. *See,* for example, Ruhaak, L.R., et al., Protein-Specific Differential Glycosylation of Immunoglobulins in Serum of Ovarian Cancer Patients DOI: 10.1021/acs.jproteome.5b01071; J. Proteome Res., 2016, 15, 1002-1010 (2016); also Miyamoto, S., et al., Multiple Reaction Monitoring for the Quantitation of Serum Protein Glycosylation Profiles: Application to Ovarian Cancer, DOI: 10.1021/acs.jproteome.7b00541, J. Proteome Res. 2018, 17, 222-233 (2017). Li, Q, et al. (Site-Specific Glycosylation Quantitation of 50 Serum Glycoproteins Enhanced by Predictive Glycopeptidomics for Improved Disease Biomarker Discovery) Analytical Chemistry 2019 91 (8), 5433-5445, describes using multiple reaction monitoring (MRM) for the quantitation of serum glycoproteins. Shiyue, Z, et al. (Quantitation of Permethylated N-Glycans through Multiple-Reaction Monitoring (MRM) LC-MS/MS) J Am Soc Mass Spectrom. 2015 26 (4), 596-603, describes using MRM for quantitation of glycopeptide expression in human serum and cancer cells. Shetty, V, et al 2010 (Investigation of ovarian cancer associated sialylation changes in N-linked glycopeptides by quantitative proteomics) Clin Proteomics. 2012 Aug 2;9(1):10, describes N-linked glycopeptide proteomic quantification in samples from ovarian cancer patients. However, using MS to diagnose cancer, generally, or ovarian cancer specifically, has not been demonstrated to date in a clinically relevant manner.

What is needed are new biomarkers and new methods of using MS to diagnose disease states such as cancer using these biomarkers. Set forth herein in the disclosure below are such biomarkers comprising glycans, peptides, and glycopeptides, as well as fragments thereof, and methods of using the biomarkers with MS to diagnose ovarian cancer.

### SUMMARY

The invention provides a method for diagnosing a patient having ovarian cancer; the method comprising: performing mass spectroscopy of a biological sample that has been obtained from the patient, using MRM-MS with a QQQ and/or qTOF spectrometer to detect MRM transitions selected from the group consisting of transitions 1, 2 or 3, wherein each MRM transition is indicative of a corresponding alpha-1-antitrypsin (A1AT) glycopeptide in Table 1, wherein the analyzing the detected glycopeptides comprises using a machine learning algorithm; inputting the quantification of the MRM transitions into a trained model to generate an output probability; determining if the output probability is above or below a threshold for a classification; and identifying a diagnostic classification for the patient based on whether the output probability is above or below a threshold for a classification; and diagnosing the patient as having ovarian cancer based on the diagnostic classification.

### BRIEF DESCRIPTIONS OF THE DRAWINGS

Figures 1 through 14 illustrate glycan chemical structures, using the Symbol Nomenclature for Glycans (SNFG) system. Each glycan structure is associated with a glycan reference code number.
Figures 15 and 16 show work flows for detecting transitions 1-76 by mass spectroscopy.
Figure 17 is a plot of intensity by retention time (RT) obtained by liquid chromatography / mass spectrometry (LC/MS) detection of a biomarker analyte. The top plot shows predicted probabilities from the PB-Net algorithm, and final (RT) start and stop prediction for the integrated peak.
Figure 18 shows LC retention time analysis.

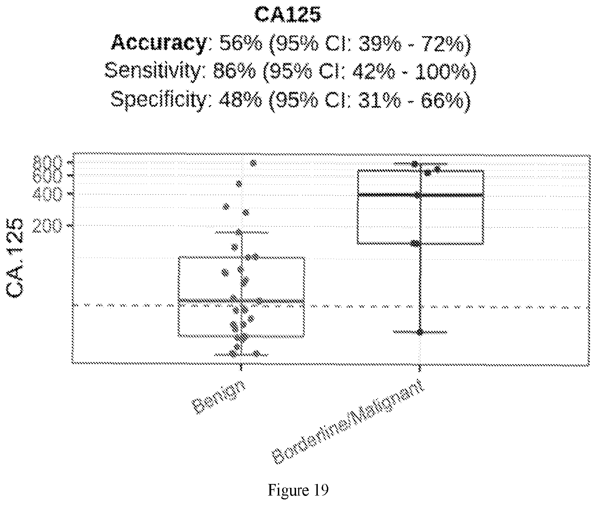

Figure 19 is plot of ELISA results for measuring CA 125 protein in benign and malignant ovarian cancer samples, as set forth in Example 3.
Figure 20 is a plot of probability of having cancer in benign and malignant ovarian cancer samples, as set forth in Example 4.

The patent or application file contains at least one drawing executed in color. Copies of this patent or patent application publication with color drawing(s) will be provided by the Office upon request and payment of the necessary fee.

### DETAILED DESCRIPTION

The following description is presented to enable one of ordinary skill in the art to make and use the invention and to incorporate it in the context of particular applications. Various modifications, as well as a variety of uses in different applications will be readily apparent to those skilled in the art, and the general principles defined herein may be applied to a wide range of embodiments..

All the features disclosed in this specification, (including any accompanying claims, abstract, and drawings) may be replaced by alternative features serving the same, equivalent or similar purpose, unless expressly stated otherwise. Thus, unless expressly stated otherwise, each feature disclosed is one example only of a generic series of equivalent or similar features.

Please note, if used, the labels left, right, front, back, top, bottom, forward, reverse, clockwise and counter clockwise have been used for convenience purposes only and are not intended to imply any particular fixed direction. Instead, they are used to reflect relative locations and/or directions between various portions of an object.

### I. GENERAL

Disclosed are methods and compositions for the profiling, detecting, and/or quantifying of glycans in a biological sample. Glycan and glycopeptide panels are described for diagnosing and screening patients having ovarian cancer. Glycan and glycopeptide panels are described for diagnosing and screening patients having cancer, an autoimmune disease, or fibrosis. The references to methods of treatment in this description are to be interpreted as reference to the compounds, pharmaceutical compositions, and medicaments of the present disclosure for use in those methods.

Certain techniques for analyzing biological samples using mass spectroscopy are known. *See,* for example, International PCT Patent Application Publication No. WO2019079639A1, filed October 18, 2018 as International Patent Application No. PCT/US2018/56574, and titled IDENTIFICATION AND USE OF BIOLOGICAL PARAMETERS FOR DIAGNOSIS AND TREATMENT MONITORING. *See,* also, US Patent Application Publication No. US20190101544A1, filed August 31, 2018 as US Patent Application No. 16/120,016, and titled IDENTIFICATION AND USE OF GLYCOPEPTIDES AS BIOMARKERS FOR DIAGNOSIS AND TREATMENT MONITORING.

### II. DEFINITIONS

As used herein, the singular forms "a," "an" and "the" include plural referents unless the context clearly dictates otherwise.

As used herein, the phrase "biological sample," refers to a sample derived from, obtained by, generated from, provided from, take from, or removed from an organism; or from fluid or tissue from the organism. Biological samples include, but are not limited to synovial fluid, whole blood, blood serum, blood plasma, urine, sputum, tissue, saliva, tears, spinal fluid, tissue section(s) obtained by biopsy; cell(s) that are placed in or adapted to tissue culture; sweat, mucous, fecal material, gastric fluid, abdominal fluid, amniotic fluid, cyst fluid, peritoneal fluid, pancreatic juice, breast milk, lung lavage, marrow, gastric acid, bile, semen, pus, aqueous humor, transudate, and the like including derivatives, portions and combinations of the foregoing. In some examples, biological samples include, but are not limited, to blood and/or plasma. In some examples, biological samples include, but are not limited, to urine or stool. Biological samples include, but are not limited, to saliva. Biological samples include, but are not limited, to tissue dissections and tissue biopsies. Biological samples include, but are not limited, any derivative or fraction of the aforementioned biological samples.

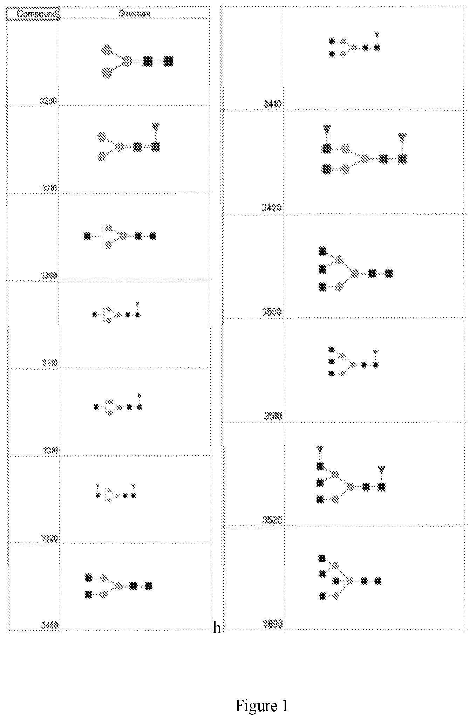

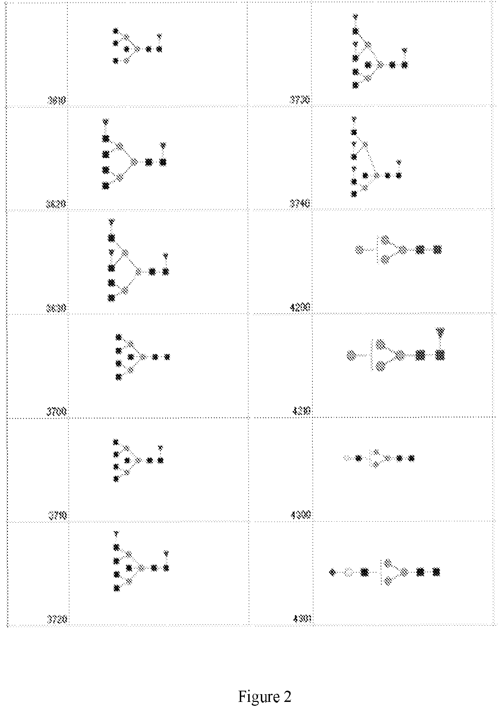

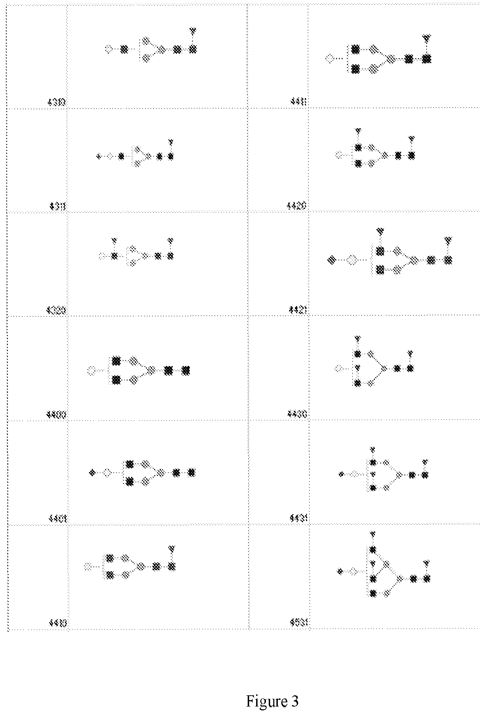

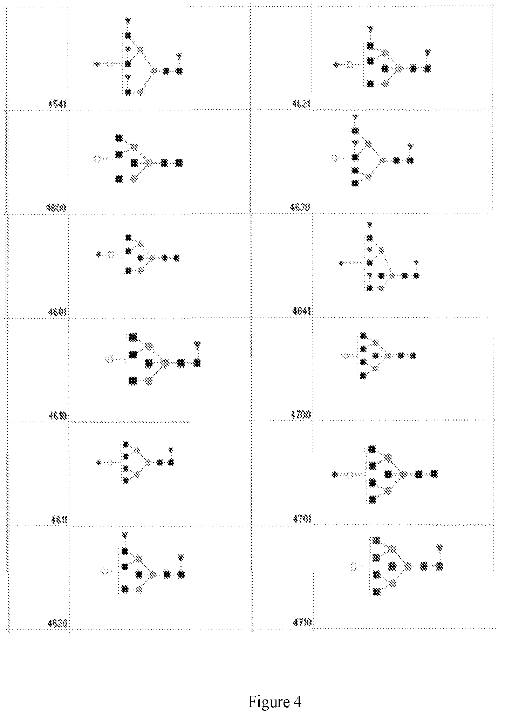

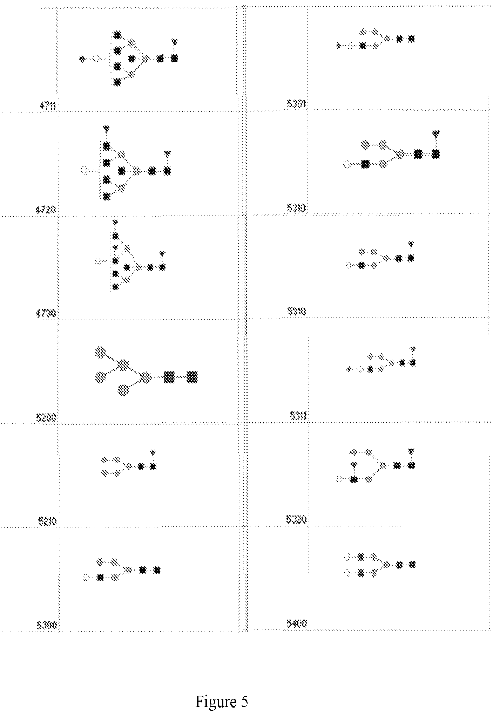

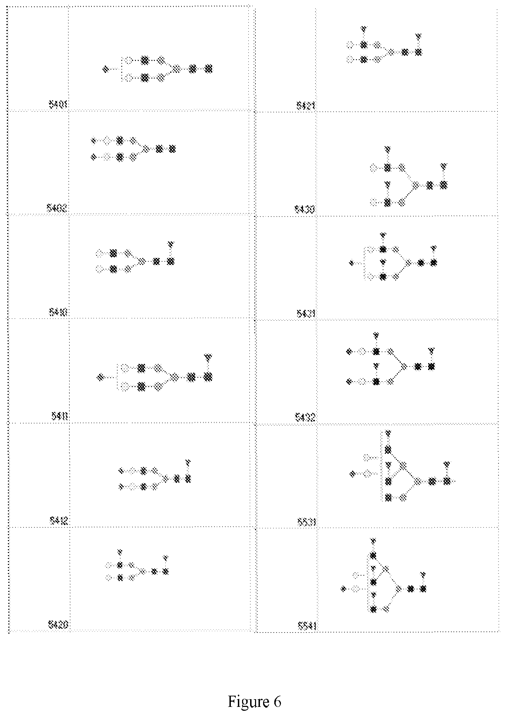

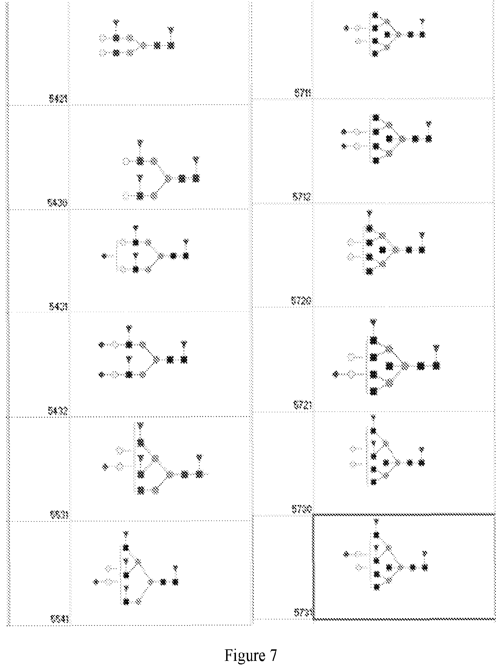

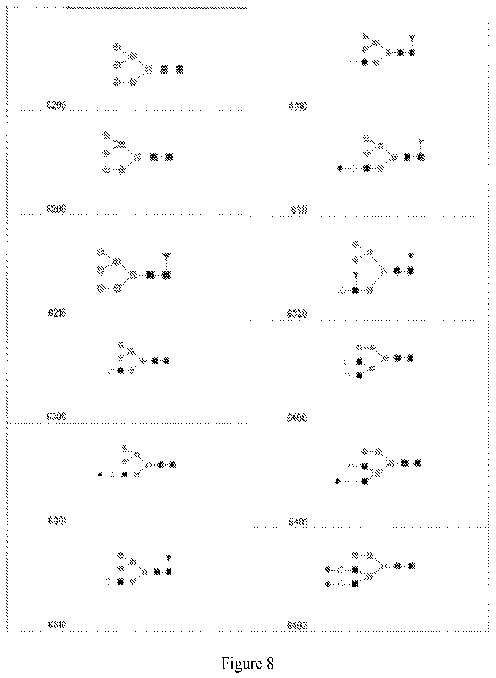

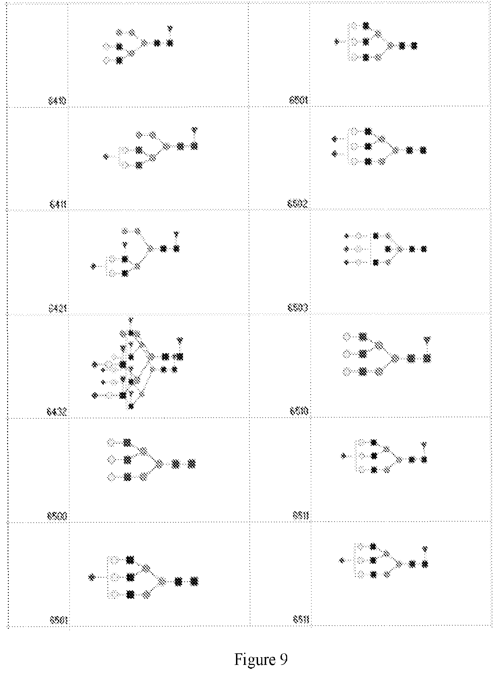

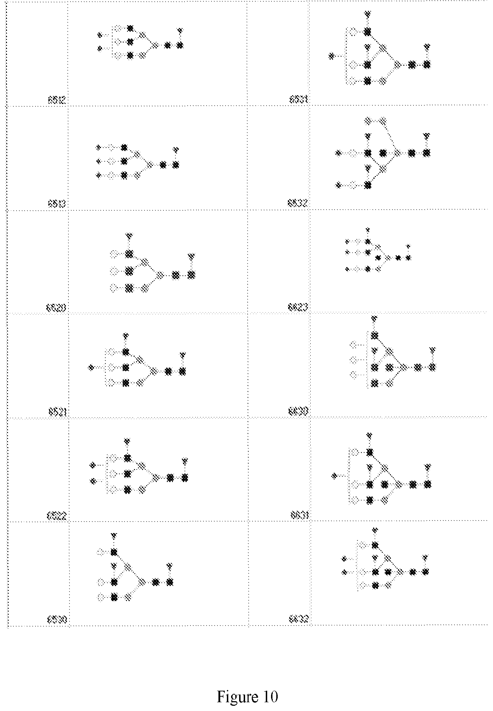

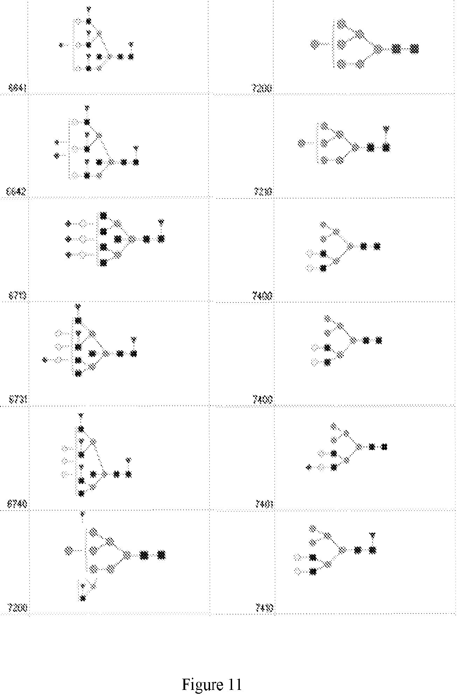

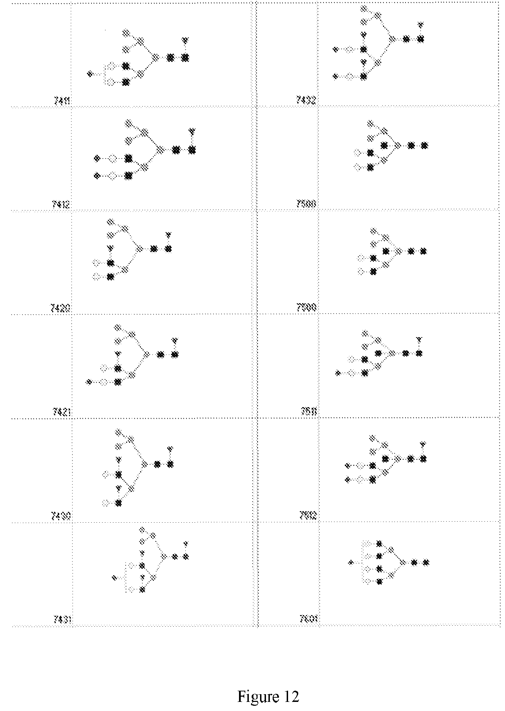

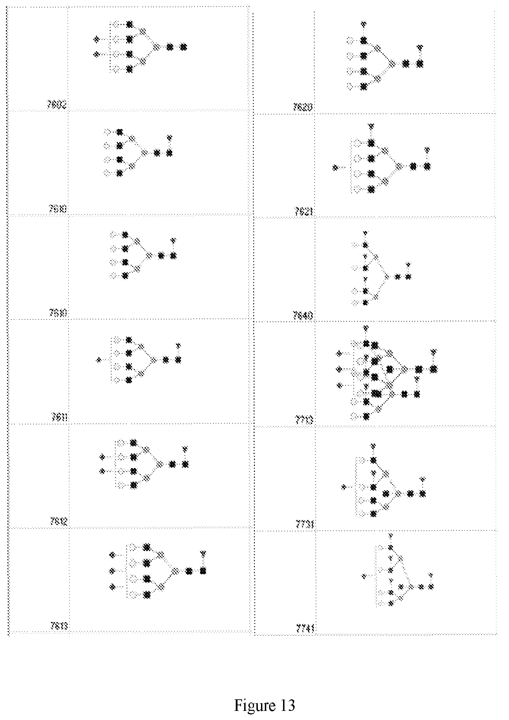

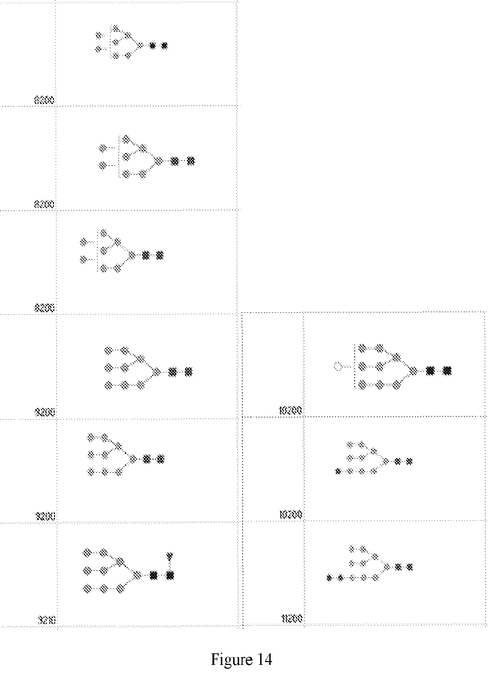

As used herein, the term "glycan" refers to the carbohydrate residue of a glycoconjugate, such as the carbohydrate portion of a glycopeptide, glycoprotein, glycolipid or proteoglycan. Glycan structures are described by a glycan reference code number, and also illustrated in International PCT Patent Application No. PCT/US2020/016286, filed January 31, 2020. For example *see* Figures 1 through 14 of PCT Patent Application No. PCT/US2020/016286, filed January 31, 2020. Glycans are illustrated using the Symbol Nomenclature for Glycans (SNFG) for illustrating glycans. An explanation of this illustration system is available on the internet at www.ncbi.nlm.nih.gov/glycans/snfg.html. Symbol Nomenclature for Graphical Representation of Glycans as published in Glycobiology 25: 1323-1324, 2015, which is available on the internet at doi.org/10.1093/glycob/cwv091. Additional information showing illustrations of the SNFG system are. Within this system, the term, Hex_i: is interpreted as follows: i indicates the number of green circles (mannose) and the number of yellow circles (galactose). The term, HexNAC_j, uses j to indicate the number of blue squares (GlcNAC's). The term Fuc_d, uses d to indicate the number of red triangles (fucose). The term Neu₅AC_l, uses l to indicate the number of purple diamonds (sialic acid). The glycan reference codes used herein combine these i, j, d, and l terms to make a composite 4-5 number glycan reference code, *e.g.,* 5300 or 5320. As an example, glycans 3200 and 3210 in Figure 1 both include 3 green circles (mannose), 2 blue squares (GlcNAC's), and no purple diamonds (sialic acid) but differ in that glycan 3210 also includes 1 red triangle (fucose).

As used herein, the term "glycopeptide," refers to a peptide having at least one glycan residue bonded thereto. In each embodiment described herein, the glycopeptide may comprise, consist essentially of, or consist of, the amino acid sequence specified by the indicated SEQ ID NO together with one or more glycans, for instance those described herein associated with that SEQ ID NO. For instance, a glycopeptide according to SEQ ID NO: 1, as used herein, can refer to a glycopeptide according to the amino acid sequence of SEQ ID NO: 1 and glycan 5402, wherein the glycan is bonded to residue 1424 of SEQ ID NO: 1. A glycopeptide comprising SEQ ID NO: 1, as used herein, can refer to a glycopeptide comprising the amino acid sequence of SEQ ID NO: 1 and glycan 5402, wherein the glycan is bonded to residue 1424. A glycopeptide consisting essentially of SEQ ID NO: 1, as used herein, can refer to a glycopeptide consisting essentially of the amino acid sequence of SEQ ID NO: 1 and glycan 5402, wherein the glycan is bonded to residue 1424. A glycopeptide consisting of SEQ ID NO: 1, as used herein, can refer to a glycopeptide consisting of the amino acid sequence of SEQ ID NO: 1 and glycan 5402, wherein the glycan is bonded to residue 1424. Similarly usage applies to SEQ ID NOs: 2-30, with the glycans described in sections below.

As used herein, the phrase "glycosylated peptides," refers to a peptide bonded to a glycan.

As used herein, the phrase "glycopeptide fragment" or "glycosylated peptide fragment" or "glycopeptide" refers to a glycosylated peptide (or glycopeptide) having an amino acid sequence that is the same as part (but not all) of the amino acid sequence of the glycosylated protein from which the glycosylated peptide is obtained, e.g., ion fragmentation within a MRM-MS instrument. MRM refers to multiple-reaction-monitoring. Unless specified otherwise, within the specification, "glycopeptide fragments" or "fragments of a glycopeptide" refer to the fragments produced directly by using a mass spectrometer optionally after the glycoprotein has been digested enzymatically to produce the glycopeptides.

As used herein, the phrase "glycoprotein" refers to the glycosylated protein from which the glycosylated peptide is obtained.

As used herein, the phrase "peptide," is meant to include glycopeptides, and not the glycosylated protein from which the glycosylated peptide is obtained, unless stated otherwise.

As used herein, the phrase "multiple reaction monitoring mass spectrometry (MRM-MS)," refers to a highly sensitive and selective method for the targeted quantification of glycans and peptides in biological samples. Unlike traditional mass spectrometry, MRM-MS is highly selective (targeted), allowing researchers to fine tune an instrument to specifically look for certain peptides fragments of interest. MRM allows for greater sensitivity, specificity, speed and quantitation of peptides fragments of interest, such as a potential biomarker. MRM-MS involves using one or more of a triple quadrupole (QQQ) mass spectrometer and a quadrupole time-of-flight (qTOF) mass spectrometer.

As used herein, the phrase "digesting a glycopeptide," refers to a biological process that employs enzymes to break specific amino acid peptide bonds. For example, digesting a glycopeptide includes contacting a glycopeptide with an digesting enzyme, e.g., trypsin to produce fragments of the glycopeptide. In some examples, a protease enzyme is used to digest a glycopeptide. The term "protease" refers to an enzyme that performs proteolysis or breakdown of large peptides into smaller polypeptides or individual amino acids. Examples of a protease include, but are not limited to, one or more of a serine protease, threonine protease, cysteine protease, aspartate protease, glutamic acid protease, metalloprotease, asparagine peptide lyase, and any combinations of the foregoing.

As used herein, the phrase "fragmenting a glycopeptide," refers to the ion fragmentation process which occurs in a MRM-MS instrument. Fragmenting may produce various fragments having the same mass but varying with respect to their charge.

As used herein, the term "subject," refers to a mammal. The non-liming examples of a mammal include a human, non-human primate, mouse, rat, dog, cat, horse, or cow, and the like. Mammals other than humans can be advantageously used as subjects that represent animal models of disease, pre-disease, or a pre-disease condition. A subject can be male or female. However, in the context of diagnosing ovarian cancer, the subject is female unless explicitly specified otherwise. A subject can be one who has been previously identified as having a disease or a condition, and optionally has already undergone, or is undergoing, a therapeutic intervention for the disease or condition. Alternatively, a subject can also be one who has not been previously diagnosed as having a disease or a condition. For example, a subject can be one who exhibits one or more risk factors for a disease or a condition, or a subject who does not exhibit disease risk factors, or a subject who is asymptomatic for a disease or a condition. A subject can also be one who is suffering from or at risk of developing a disease or a condition.

As used herein, the term "patient" refers to a mammalian subject. The mammal can be a human, or an animal including, but not limited to an equine, porcine, canine, feline, ungulate, and primate animal. In one embodiment, the individual is a human. The methods and uses described herein are useful for both medical and veterinary uses. A "patient" is a human subject unless specified to the contrary.

As used herein, the phrase "multiple-reaction-monitoring (MRM) transition," refers to the mass to charge (m/z) peaks or signals observed when a glycopeptide, or a fragment thereof, is detected by MRM-MS. The MRM transition is detected as the transition of the precursor and product ion.

As used herein, the phrase "detecting a multiple-reaction-monitoring (MRM) transition," refers to the process in which a mass spectrometer analyzes a sample using tandem mass spectrometer ion fragmentation methods and identifies the mass to charge ratio for ion fragments in a sample. The phrase also refers to refers to a MS process in which a MRM-MS transition is detected and then compare to a calculated mass to charge ratio (m/z) of a glycopeptide, or fragment thereof, in order to identify the glycopeptide. The absolute value of these identified mass to charge ratios are referred to as transitions. In the context of the methods set forth herein, the mass to charge ratio transitions are the values indicative of glycan, peptide or glycopeptide ion fragments. For some glycopeptides set forth herein, there is a single transition peak or signal. For some other glycopeptides set forth herein, there is more than one transition peak or signal. In some examples, herein, a single transition may be indicative of two more glycopeptides, if those glycopeptides have identical MRM-MS fragmentation patterns. A transition peak or signal includes, but is not limited to, those transitions set forth herein were are associated with a glycopeptide consisting of, or consisting essentially of, an amino acid sequence selected from SEQ ID NOs: 1-76, and combinations thereof, according to Tables 1-5, e.g., Table 1, Table 2, Table 3, Table 4, or Table 5, or a combination thereof. Background information on MRM mass spectrometry can be found in Introduction to Mass Spectrometry: Instrumentation, Applications, and Strategies for Data Interpretation, 4th Edition, J. Throck Watson, O. David Sparkman, ISBN: 978-0-470-51634-8, November 2007.

As used herein, the term "reference value" refers to a value obtained from a population of individual(s) whose disease state is known. The reference value may be in n-dimensional feature space and may be defined by a maximum-margin hyperplane. A reference value can be determined for any particular population, subpopulation, or group of individuals according to standard methods well known to those of skill in the art.

As used herein, the term "population of individuals" means one or more individuals. In one embodiment, the population of individuals consists of one individual. In one embodiment, the population of individuals comprises multiple individuals. As used herein, the term "multiple" means at least 2 (such as at least 4, 6, 8, 10, 12, 14, 16, 18, 20, 22, 24, 26, 28, or 30) individuals. In one embodiment, the population of individuals comprises at least 10 individuals.

As used herein, the term "treatment" or "treating" means any treatment of a disease or condition in a subject, such as a mammal, including: 1) preventing or protecting against the disease or condition, that is, causing the clinical symptoms not to develop; 2) inhibiting the disease or condition, that is, arresting or suppressing the development of clinical symptoms; and/or 3) relieving the disease or condition that is, causing the regression of clinical symptoms. Treating may include administering therapeutic agents to a subject in need thereof.

As used herein, the term "about" indicates and encompasses an indicated value and a range above and below that value. In certain embodiments, the term "about" indicates the designated value ± 10%, ± 5%, or ± 1%. In certain embodiments, the term "about" indicates the designated value ± one standard deviation of that value.

### III. BIOMARKERS

Set forth herein are biomarkers. These biomarkers are useful for a variety of applications, including, but not limited to, diagnosing diseases and conditions. For example, certain biomarkers set forth herein, or combinations thereof, are useful for diagnosing ovarian cancer. In some other examples, certain biomarkers set forth herein, or combinations thereof, are useful for diagnosing and screening patients having cancer, an autoimmune disease, or fibrosis. In some examples, the biomarkers set forth herein, or combinations thereof, are useful for classifying a patient so that the patient receives the appropriate medical treatment. In some other examples, the biomarkers set forth herein, or combinations thereof, are useful for treating or ameliorating a disease or condition in patient by, for example, identifying a therapeutic agent with which to treat a patient. In some other examples, the biomarkers set forth herein, or combinations thereof, are useful for determining a prognosis of treatment for a patient or a likelihood of success or survivability for a treatment regimen.

In some examples, a sample from a patient is analyzed by MS and the results are used to determine the presence, absolute amount, and/or relative amount of a glycopeptide consisting of an amino acid sequence selected from SEQ ID NOs: 1-76 in the sample. In some examples, a sample from a patient is analyzed by MS and the results are used to determine the presence, absolute amount, and/or relative amount of a glycopeptide consisting essentially of an amino acid sequence selected from SEQ ID NOs: 1-76 in the sample. In some examples, a sample from a patient is analyzed by MS and the results are used to determine the presence, absolute amount, and/or relative amount of a glycopeptide consisting of, or consisting essentially of, an amino acid sequence selected from SEQ ID NOs: 1-76 in the sample. In some examples, a sample from a patient is analyzed by MS and the results are used to determine the presence, absolute amount, and/or relative amount of a glycopeptide consisting of, or consisting essentially of, an amino acid sequence selected from SEQ ID NOs: 1-76 in the sample. In some examples, as described below, the presence, absolute amount, and/or relative amount of a glycopeptide is determined by analyzing the MS results. In the invention as claimed, MRM transitions selected from the group consisting of transitions 1, 2 or 3, wherein each MRM transition is indicative of a corresponding alpha-1-antitrypsin (A1AT) glycopeptide in Table 1, are detected. The MS results are analyzed using machine learning.

Set forth herein are biomarkers selected from glycans, peptides, glycopeptides, fragments thereof, and combinations thereof. In some examples, the glycopeptide consists of an amino acid sequence selected from SEQ ID NOs: 1-76. In some examples, the glycopeptide consists essentially of an amino acid sequence selected from SEQ ID NOs: 1-76. In the invention as claimed, MRM transitions selected from the group consisting of transitions 1, 2 or 3, wherein each MRM transition is indicative of a corresponding alpha-1-antitrypsin (A1AT) glycopeptide in Table 1, are detected.

### a. O-Glycosylation

In some examples, the glycopeptides set forth herein include O-glycosylated peptides. These peptides include glycopeptides in which a glycan is bonded to the peptide through an oxygen atom of an amino acid. Typically, the amino acid to which the glycan is bonded is threonine (T) or serine (S). In some examples, the amino acid to which the glycan is bonded is threonine (T). In some examples, the amino acid to which the glycan is bonded is serine (S).

The O-glycosylated peptides may include those peptides from the group selected from Apolipoprotein C-III (APOC3), Alpha-2-HS-glycoprotein (FETUA), and combinations thereof. In certain examples, the O-glycosylated peptide, set forth herein, is an Apolipoprotein C-III (APOC3) peptide. In certain examples, the O-glycosylated peptide, set forth herein, is an Alpha-2-HS-glycoprotein (FETUA).

### b. N-Glycosylation

In some examples, the glycopeptides set forth herein include N-glycosylated peptides. These peptides include glycopeptides in which a glycan is bonded to the peptide through a nitrogen atom of an amino acid. Typically, the amino acid to which the glycan is bonded is asparagine (N) or arginine (R). In some examples, the amino acid to which the glycan is bonded is asparagine (N). In some examples, the amino acid to which the glycan is bonded is arginine (R).

The N-glycosylated peptides may include members selected from the group consisting of Alpha-1-antitrypsin (A1AT), Alpha-1B-glycoprotein (A1BG), Leucine-richAlpha-2-glycoprotein (A2GL), Alpha-2-macroglobulin (A2MG), Alpha-1-antichymotrypsin (AACT), Afamin (AFAM), Alpha-1-acid glycoprotein 1 & 2 (AGP12), Alpha-1-acid glycoprotein 1 (AGP1), Alpha-1-acid glycoprotein 2 (AGP2), Apolipoprotein A-I (APOA1), Apolipoprotein B-100 (APOB), Apolipoprotein D (APOD), Beta-2-glycoprotein-1 (APOH), Apolipoprotein M (APOM), Attractin (ATRN), Calpain-3 (CAN3), Ceruloplasmin (CERU), ComplementFactorH (CFAH), ComplementFactorI (CFAI), Clusterin (CLUS), ComplementC3 (CO3), ComplementC4-A&B (CO4A&CO4B), ComplementcomponentC6 (CO6), ComplementComponentC8AChain (CO8A), Coagulation factor XII (FA12), Haptoglobin (HPT), Histidine-rich Glycoprotein (HRG), Immunoglobulin heavy constant alpha 1&2 (IgA12), Immunoglobulin heavy constant alpha 2 (IgA2), Immunoglobulin heavy constant gamma 2 (IgG2), Immunoglobulin heavy constant mu (IgM), Inter-alpha-trypsin inhibitor heavy chain H1 (ITIH1), Plasma Kallikrein (KLKB 1), Kininogen-1 (KNG1), Serum paraoxonase/arylesterase 1 (PON1), Selenoprotein P (SEPP1), Prothrombin (THRB), Serotransferrin (TRFE), Transthyretin (TTR), Protein unc-13HomologA (UN13A), Vitronectin (VTNC), Zinc-alpha-2-glycoprotein (ZA2G), Insulin-like growth factor-II (IGF2), Apolipoprotein C-I (APOC1), Hemopexin (HEMO), Immunoglobulin heavy constant gamma 1 (IgG1), Immunoglobulin J chain (IgJ), and combinations thereof. In the invention as claimed, MRM transitions selected from the group consisting of transitions 1, 2 or 3, wherein each MRM transition is indicative of a corresponding alpha-1-antitrypsin (A1AT) glycopeptide in Table 1, are detected.

### c. Peptides and Glycopeptides

Set forth herein is a glycopeptide or peptide consisting of an amino acid sequence selected from the group consisting of SEQ ID NOs: 1 - 76, and combinations thereof.

Set forth herein is a glycopeptide or peptide consisting essentially of an amino acid sequence selected from the group consisting of SEQ ID NOs: 1 - 76, and combinations thereof.

Set forth herein is a glycopeptide or peptide consisting of an amino acid sequence selected from the group consisting of SEQ ID NOs: 1, 6, 7, 8, 10, 15, 21, 22, 23, 42, 47, 48, 51, 56, 57, 58, 62, 74, and 76, and combinations thereof.

Set forth herein is a glycopeptide or peptide consisting essentially of an amino acid sequence selected from the group consisting of SEQ ID NOs: 1, 6, 7, 8, 10, 15, 21, 22, 23, 42, 47, 48, 51, 56, 57, 58, 62, 74, and 76, and combinations thereof.

Set forth herein is a glycopeptide or peptide consisting of an amino acid sequence selected from SEQ ID NO: 1, 3, 4, 5, 8, 11, 12, 13, 14, 16, 17, 18, 19, 20, 22, 23, 24, 25, 27, 30, 32, 34, 43, 45, 51, 54, 55, 65, 68, 71, 73, 74, 75, and combinations thereof.

Set forth herein is a glycopeptide or peptide consisting essentially of an amino acid sequence selected from SEQ ID NO: 1, 3, 4, 5, 8, 11, 12, 13, 14, 16, 17, 18, 19, 20, 22, 23, 24, 25, 27, 30, 32, 34, 43, 45, 51, 54, 55, 65, 68, 71, 73, 74, 75, and combinations thereof.

Set forth herein is a glycopeptide or peptide consisting of an amino acid sequence selected from SEQ ID NO: 2, 6, 7, 9, 10, 15, 21, 26, 28, 29, 31, 33, 35, 36, 37, 38, 39, 40, 41, 42, 44, 46, 47, 48, 49, 50, 52, 53, 56, 57, 58, 59, 60, 61, 62, 63, 64, 66, 67, 69, 70, 72, 76, and combinations thereof.

Set forth herein is a glycopeptide or peptide consisting essentially of an amino acid sequence selected from SEQ ID NOs: 2, 6, 7, 9, 10, 15, 21, 26, 28, 29, 31, 33, 35, 36, 37, 38, 39, 40, 41, 42, 44, 46, 47, 48, 49, 50, 52, 53, 56, 57, 58, 59, 60, 61, 62, 63, 64, 66, 67, 69, 70, 72, 76, and combinations thereof.

The glycopeptide may consist essentially of an amino acid sequence selected from SEQ ID NO:1. The glycopeptide may comprise glycan 5402, wherein the glycan is bonded to residue 271. The glycopeptide may be A1AT-GP001_271_5402. Herein A1AT refers to Alpha-1-antitrypsin.

The glycopeptide may consist essentially of an amino acid sequence selected from SEQ ID NO:2. The glycopeptide may comprise glycan 5412 at residue 271. The glycopeptide may be A1AT-GP001_271_5412.

The glycopeptide may consist essentially of an amino acid sequence selected from SEQ ID NO:3. The glycopeptide may comprise glycan 5402 at residue 271. The glycopeptide may be A1AT-GP001_271MC_5402. Herein, "MC" refers to a missed cleavage of a trypsin digestion. A missed cleavage peptide includes the amino acid sequence selected from SEQ ID NO:3 but also includes additional residues which were not cleaved by way of trypsin digestion.

The glycopeptide may consist essentially of an amino acid sequence selected from SEQ ID NO:4. The glycopeptide may comprise glycans 5421 or 5402, or both, at residue 179. The glycopeptide may be A1BG-GP002_179_5421/5402. Herein, when two glycans are recited with a forward slash (/) between them, this means, unless specified otherwise explicitly, that the mass spectrometry method is unable to distinguish between these two glycans, e.g., because they share a common mass to charge ratio. Unless specified to the contrary, 5421/5402 means that either glycan 5421 or 5402 is present. The quantification of the amount of glycans 5421/5402 includes a summation of the detected amount of glycan 5421 as well as the detected amount of glycan 5402. Herein A1BG refers to Alpha-1B-glycoprotein.

The glycopeptide may consist essentially of an amino acid sequence selected from SEQ ID NO:5. The glycopeptide may comprise glycan 5402 at residue 1424. The glycopeptide may be A2MG-GP004_1424_5402. Herein A2MG refers to Alpha-2-macroglobulin.

The glycopeptide may consist essentially of an amino acid sequence selected from SEQ ID NO:6. The glycopeptide may comprise glycan 5411 at residue 1424. The glycopeptide may be A2MG-GP004_1424_5411.

The glycopeptide may consist essentially of an amino acid sequence selected from SEQ ID NO:7. The glycopeptide may comprise glycan 5401 at residue 55. The glycopeptide may be A2MG-GP004_55_5401.

The peptide may comprise an amino acid sequence selected from SEQ ID NO:8. The glycopeptide may comprise glycan 5402 at residue 55. The glycopeptide may be A2MG-GP004_55_5402.

The glycopeptide may consist essentially of an amino acid sequence selected from SEQ ID NO:9. The glycopeptide may comprise glycan 5411 at residue 55. The glycopeptide may be A2MG-GP004_55_5411.

The peptide may comprise an amino acid sequence selected from SEQ ID NO:10. The glycopeptide may comprise glycan 5200 at residue 869. The peptide may be A2MG-GP004_869_5200.

The peptide may comprise an amino acid sequence selected from SEQ ID NO:11. The glycopeptide may comprise glycan 869 at residue 5401. The glycopeptide may be A2MG-GP004_869_5401.

The glycopeptide may consist essentially of an amino acid sequence selected from SEQ ID NO: 12. The glycopeptide may comprise glycan 6301 at residue 869. The glycopeptide may be A2MG-GP004_869_6301.

The glycopeptide may consist essentially of an amino acid sequence selected from SEQ ID NO: 13. The glycopeptide may comprise glycan 5402 at residue 33. The glycopeptide may be AFAM-GP006_33_5402. Herein, AFAM refers to Afamin.

The glycopeptide may consist essentially of an amino acid sequence selected from SEQ ID NO: 14. The glycopeptide may comprise glycan 6503 at residue 72. The glycopeptide may be AGP12-GP007&008_72MC_6503. Herein AGP12 refers to Alpha-1-acid glycoprotein 1&2.

The glycopeptide may consist essentially of an amino acid sequence selected from SEQ ID NO:15. The glycopeptide may comprise glycan 6513 at residue 72. The glycopeptide may be AGP12-GP007&008_72MC_6513.

The glycopeptide may consist essentially of an amino acid sequence selected from SEQ ID NO: 16. The glycopeptide may comprise glycan 7601 at residue 72. The glycopeptide may be AGP12-GP007&008_72MC_7601.

The glycopeptide may consist essentially of an amino acid sequence selected from SEQ ID NO: 17. The glycopeptide may comprise glycan 7602 at residue 72. The glycopeptide may be AGP12-GP007&008_72MC_7602.

The glycopeptide may consist essentially of an amino acid sequence selected from SEQ ID NO: 18. The glycopeptide may comprise glycan 7603 at residue 72. The glycopeptide may be AGP12-GP007&008_72MC_7603.

The glycopeptide may consist essentially of an amino acid sequence selected from SEQ ID NO: 19. The glycopeptide may comprise glycan 7613 at residue 72. The glycopeptide may be AGP12-GP007&008_72MC_7613.

The glycopeptide may consist essentially of an amino acid sequence selected from SEQ ID NO:20. The glycopeptide may comprise glycan 5402 at residue 33. The glycopeptide may be AGP1-GP007_33_5402.

The glycopeptide may consist essentially of an amino acid sequence selected from SEQ ID NO:21. The glycopeptide may comprise glycans 6503 or 6522, or both, at residue 93. The glycopeptide may be AGP1-GP007_93_6503/6522. Herein AGP1 refers to Alpha-1-acid glycoprotein 1.

The glycopeptide may consist essentially of an amino acid sequence selected from SEQ ID NO:22. The glycopeptide may comprise glycan 6513 at residue 93. The glycopeptide may be AGP1-GP007_93_6513.

The glycopeptide may consist essentially of an amino acid sequence selected from SEQ ID NO:23. The glycopeptide may comprise glycans 7603 or 7622, or both, at residue 93. The glycopeptide may be AGP1-GP007_93_7603/7622.

The glycopeptide may consist essentially of an amino acid sequence selected from SEQ ID NO:24. The glycopeptide may comprise glycan 7613 at residue 93. The glycopeptide may be AGP1-GP007_93_7613.

The glycopeptide may consist essentially of an amino acid sequence selected from SEQ ID NO:25. The glycopeptide may comprise glycan 1102 at residue 74. The glycopeptide may be APOC3-GP012_74_1102. Herein APOC3 refers to Apolipoprotein C-III.

The glycopeptide may consist essentially of an amino acid sequence selected from SEQ ID NO:26. The glycopeptide may comprise glycan 1102 at residue 74. The glycopeptide may be APOC3-GP012_74MC_1102.

The glycopeptide may consist essentially of an amino acid sequence selected from SEQ ID NO:27. The glycopeptide may comprise glycan 5401 at residue 253. The glycopeptide may be APOH-GP015_253_5401. Herein APOH refers to Beta-2-glycoprotein1.

The glycopeptide may consist essentially of an amino acid sequence selected from SEQ ID NO:28. The glycopeptide may comprise glycan 5412 at residue 138. The glycopeptide may be CERU-GP023_138_5412. Herein CERU refers to Ceruloplasmin.

The glycopeptide may consist essentially of an amino acid sequence selected from SEQ ID NO:29. The glycopeptide may comprise glycans 5421 or 5402, or both, at residue 138. The glycopeptide may be CERU-GP023_138_5421/5402.

The glycopeptide may consist essentially of an amino acid sequence selected from SEQ ID NO:30. The glycopeptide may comprise glycans 6503 or 6522, or both, at residue 138. The glycopeptide may be CERU-GP023_138_6503/6522.

The peptide may comprise an amino acid sequence selected from SEQ ID NO:31. The glycopeptide may comprise glycans 5421 or 5402, or both, at residue 882. The glycopeptide may be CFAH-GP024_882_5421/5402. Herein CFAH refers to ComplementFactorH.

The glycopeptide may consist essentially of an amino acid sequence selected from SEQ ID NO:32. The glycopeptide may comprise glycan 5200 at residue 85. The glycopeptide may be CO3-GP028_85_5200. Herein CO3 refers to ComplementC3.

The peptide may comprise an amino acid sequence selected from SEQ ID NO:33. The glycopeptide may comprise glycan 6200 at residue 85. The glycopeptide may be C03-GP028_85_6200.

The glycopeptide may consist essentially of an amino acid sequence selected from SEQ ID NO:34. The glycopeptide may comprise glycan 5402 at residue 1328. The glycopeptide may be
CO4A&CO4B-GP029&030_1328_5402. Herein CO4A&CO4B refers to ComplementC4-A&B.

The glycopeptide may consist essentially of an amino acid sequence selected from SEQ ID NO:35. The glycopeptide may comprise glycans 5421 or 5402, or both, at residue 156. The glycopeptide may be FETUA-GP036_156_5402/5421. Herein FETUA refers to Alpha-2-HS-glycoprotein.

The glycopeptide may consist essentially of an amino acid sequence selected from SEQ ID NO:36. The glycopeptide may comprise glycan 6513 at residue 176. The glycopeptide may be
FETUA-GP036_176_6513.

The glycopeptide may consist essentially of an amino acid sequence selected from SEQ ID NO:37. The glycopeptide may comprise glycan 1101 at residue 346. The glycopeptide may be
FETUA-GP036_346_1101.

The glycopeptide may consist essentially of an amino acid sequence selected from SEQ ID NO:38. The glycopeptide may comprise glycans 5412 or 5431, or both, at residue 187. The glycopeptide may be
HEMO-GP042_187_5412/5431. Herein HEMO refers to Hemopexin.

The glycopeptide may consist essentially of an amino acid sequence selected from SEQ ID NO:39. The glycopeptide may comprise glycans 5420 or 5401, or both, at residue 453. The glycopeptide may be
HEMO-GP042_453_5420/5401.

The peptide may comprise an amino acid sequence selected from SEQ ID NO:40. The glycopeptide may comprise glycan 6502 at residue 184. The glycopeptide may be HPT-GP044_184_6502. Herein HPT refers to Haptoglobin.

The glycopeptide may consist essentially of an amino acid sequence selected from SEQ ID NO:41. The glycopeptide may comprise glycan 10803 at residue 207. The glycopeptide may be HPT-GP044_207_10803.

The glycopeptide may consist essentially of an amino acid sequence selected from SEQ ID NO:42. The glycopeptide may comprise glycan 10804 at residue 207. The glycopeptide may be HPT-GP044_207_10804.

The glycopeptide may consist essentially of an amino acid sequence selected from SEQ ID NO:43. The glycopeptide may comprise glycan 11904 at residue 207. The glycopeptide may be HPT-GP044_207_11904.

The glycopeptide may consist essentially of an amino acid sequence selected from SEQ ID NO:44. The glycopeptide may comprise glycan 11914 at residue 207. The glycopeptide may be HPT-GP044_207_11914.

The glycopeptide may consist essentially of an amino acid sequence selected from SEQ ID NO:45. The glycopeptide may comprise glycan 11915 at residue 207. The glycopeptide may be HPT-GP044_207_11915.

The glycopeptide may consist essentially of an amino acid sequence selected from SEQ ID NO:46. The glycopeptide may comprise glycans 5401 or 5420, or both, at residue 241. The glycopeptide may be HPT-GP044_241_5401/5420.

The glycopeptide may consist essentially of an amino acid sequence selected from SEQ ID NO:47. The glycopeptide may comprise glycan 5412 at residue 241. The glycopeptide may be HPT-GP044_241_5412.

The glycopeptide may consist essentially of an amino acid sequence selected from SEQ ID NO:48. The glycopeptide may comprise glycan 6501 at residue 241. The glycopeptide may be HPT-GP044_241_6501.

The glycopeptide may consist essentially of an amino acid sequence selected from SEQ ID NO:49. The glycopeptide may comprise glycan 6502 at residue 241. The glycopeptide may be HPT-GP044_241_6502.

The glycopeptide may consist essentially of an amino acid sequence selected from SEQ ID NO:50. The glycopeptide may comprise glycan 6511 at residue 241. The glycopeptide may be HPT-GP044_241_6511.

The glycopeptide may consist essentially of an amino acid sequence selected from SEQ ID NO:51. The glycopeptide may comprise glycan 6513 at residue 241. The glycopeptide may be HPT-GP044_241_6513.

The glycopeptide may consist essentially of an amino acid sequence selected from SEQ ID NO:52. The glycopeptide may comprise glycan 5501 at residue 144. The glycopeptide may be IgA12-GP046&047_144_5501. Herein IgA12 refers to Immunoglobulin heavy constant alpha 1&2.

The glycopeptide may consist essentially of an amino acid sequence selected from SEQ ID NO:53. The glycopeptide may comprise glycan 4510 at residue 205. The glycopeptide may be IgA2-GP047_205_4510. Herein IgA2 refers to Immunoglobulin heavy constant alpha 2.

The glycopeptide may consist essentially of an amino acid sequence selected from SEQ ID NO:54. The glycopeptide may comprise glycan 5412 at residue 205. The glycopeptide may be IgA2-GP047_205_5412.

The glycopeptide may consist essentially of an amino acid sequence selected from SEQ ID NO:55. The glycopeptide may comprise glycan 5501 at residue 205. The glycopeptide may be IgA2-GP047_205_5510.

The glycopeptide may consist essentially of an amino acid sequence selected from SEQ ID NO:56. The glycopeptide may comprise glycan 3410 at residue 297. The glycopeptide may be IgG1-GP048_297_3410. Herein IgG1 refers to Immunoglobulin heavy constant gamma 1.

The glycopeptide may consist essentially of an amino acid sequence selected from SEQ ID NO:57. The glycopeptide may comprise glycan 4400 at residue 297. The glycopeptide may be IgG1-GP048_297_4400.

The glycopeptide may consist essentially of an amino acid sequence selected from SEQ ID NO:58. The glycopeptide may comprise glycan 4510 at residue 297. The glycopeptide may be IgG1-GP048_297_4510.

The glycopeptide may consist essentially of an amino acid sequence selected from SEQ ID NO:59. The glycopeptide may comprise glycan 5400 at residue 297. The glycopeptide may be IgG1-GP048_297_5400.

The glycopeptide may consist essentially of an amino acid sequence selected from SEQ ID NO:60. The glycopeptide may comprise glycan 5410 at residue 297. The glycopeptide may be IgG1-GP048_297_5410.

The glycopeptide may consist essentially of an amino acid sequence selected from SEQ ID NO:61. The glycopeptide may comprise glycan 5411 at residue 297. The glycopeptide may be IgG1-GP048_297_5411.

The glycopeptide may consist essentially of an amino acid sequence selected from SEQ ID NO:62. The glycopeptide may comprise glycan 5510 at residue 297. The glycopeptide may be IgG1-GP048_297_5510.

The glycopeptide may consist essentially of an amino acid sequence selected from SEQ ID NO:63. The glycopeptide may be
IgGI-GP048_297_nonglycosylated.

The glycopeptide may consist essentially of an amino acid sequence selected from SEQ ID NO:64. The glycopeptide may comprise glycan 3510 at residue 297. The glycopeptide may be
IgG2-GP049_297_3510. Herein IgG2 refers to Immunoglobulin heavy constant gamma 2.

The glycopeptide may consist essentially of an amino acid sequence selected from SEQ ID NO:65. The glycopeptide may comprise glycan 4411 at residue 297. The glycopeptide may be
IgG2-GP049_297_4411.

The glycopeptide may consist essentially of an amino acid sequence selected from SEQ ID NO:66. The glycopeptide may comprise glycan 4510 at residue 297. The glycopeptide may be
IgG2-GP049_297_4510.

The glycopeptide may consist essentially of an amino acid sequence selected from SEQ ID NO:67. The glycopeptide comprise glycan 5401 at residue 71. The glycopeptide may be IgJ-GP052_71_5401. Herein IgJ refers to Immunoglobulin J chain.

The glycopeptide may consist essentially of an amino acid sequence selected from SEQ ID NO:68. The glycopeptide may comprise glycan 6200 at residue 439. The glycopeptide may be IgM-GP053_439_6200. Herein IgM refers to Immunoglobulin heavy constant mu.

The glycopeptide may consist essentially of an amino acid sequence selected from SEQ ID NO:69. The glycopeptide may comprise glycan 4311 at residue 46. The glycopeptide may be IgM-GP053_46_4311.

The glycopeptide may consist essentially of an amino acid sequence selected from SEQ ID NO:70. The glycopeptide may comprise glycan 6503 at residue 205. The glycopeptide may be KNG1-GP057_205_6503. Herein KNG1 refers to Kininogen-1.

The glycopeptide may consist essentially of an amino acid sequence selected from SEQ ID NO:71. The glycopeptide may comprise glycan 5402 at residue 432. The glycopeptide may be TRFE-GP064_432_5402. Herein TRFE refers to Serotransferrin.

The glycopeptide may consist essentially of an amino acid sequence selected from SEQ ID NO:72. The glycopeptide may comprise glycan 5401 at residue 630. The glycopeptide may be TRFE-GP064_630_5401.

The glycopeptide may consist essentially of an amino acid sequence selected from SEQ ID NO:73. The glycopeptide may comprise glycan 6513 at residue 630. The glycopeptide may be TRFE-GP064_630_6513.

The glycopeptide may consist essentially of an amino acid sequence selected from SEQ ID NO:74. The glycopeptide may comprise glycan 5401 at residue 169. The glycopeptide may be VTNC-GP067_169_5401. Herein VTNC refers to Vitronectin.

The glycopeptide may consist essentially of an amino acid sequence selected from SEQ ID NO:75. The glycopeptide may comprise glycan 6503 at residue 242. The glycopeptide may be VTNC-GP067_242_6503.

The glycopeptide may consist essentially of an amino acid sequence selected from SEQ ID NO:76. The glycopeptide may comprise glycan 5421 at residue 86. The glycopeptide may be VTNC-GP067_86_5421.

### IV. METHODS OF USING BIOMARKERS

### A. METHODS FOR DETECTING GLYCOPEPTIDES

Set forth herein is a method for detecting one or more a multiple-reaction-monitoring (MRM) transition, comprising: having obtained a biological sample from a patient, wherein the biological sample comprises one or more glycopeptides; digesting and/or fragmenting a glycopeptide in the sample; and detecting a multiple-reaction-monitoring (MRM) transition selected from the group consisting of transitions 1 - 76. The method may include detecting MRM transitions selected from the group consisting of 1, 6, 7, 8, 10, 15, 21, 22, 23, 42, 47, 48, 51, 56, 57, 58, 62, 74, 76, and combinations thereof. The method may include detecting MRM transitions selected from the group consisting of 1, 3, 4, 5, 8, 11, 12, 13, 14, 16, 17, 18, 19, 20, 22, 23, 24, 25, 27, 30, 32, 34, 43, 45, 51, 54, 55, 65, 68, 71, 73, 74, 75, and combinations thereof. The method may include detecting MRM transitions selected from the group consisting of 2, 6, 7, 9, 10, 15, 21, 26, 28, 29, 31, 33, 35, 36, 37, 38, 39, 40, 41, 42, 44, 46, 47, 48, 49, 50, 52, 53, 56, 57, 58, 59, 60, 61, 62, 63, 64, 66, 67, 69, 70, 72, 76, and combinations thereof. Transitions may include any one or more of the transitions in Tables 1-5. Transitions may include any one or more of the transitions in Tables 1-3. Transitions may include any one or more of the transitions in Table 1. Transitions may include any one or more of the transitions in Table 2. Transitions may include any one or more of the transitions in Table 3. Transitions may include any one or more of the transitions in Table 4. Transitions may include any one or more of the transitions in Table 5. Transitions may be indicative of glycopeptides.

In the methods as claimed, the methods may include fragmenting a glycopeptide in the sample; and detecting a multiple-reaction-monitoring (MRM) transition selected from the group consisting of transitions 1 - 3, and combinations thereof.

Set forth herein is a method of detecting one or more glycopeptides, wherein each glycopeptide is individually in each instance selected from a glycopeptide consisting of an amino acid sequence selected from the group consisting of SEQ ID NOs: 1 - 76, and combinations thereof.

In Set forth herein is a method of detecting one or more glycopeptides, wherein each glycopeptide is individually in each instance selected from a glycopeptide consisting essentially of an amino acid sequence selected from the group consisting of SEQ ID NOs: 1 - 76, and combinations thereof.

Set forth herein is a method of detecting one or more glycopeptides. Set forth herein is a method of detecting one or more glycopeptide fragments. The method may include detecting the glycopeptide group to which the glycopeptide, or fragment thereof, belongs. The glycopeptide group may be selected from Alpha-1-antitrypsin (A1AT), Alpha-1B-glycoprotein (A1BG), Leucine-richAlpha-2-glycoprotein (A2GL), Alpha-2-macroglobulin (A2MG),
Alpha-1-antichymotrypsin (AACT), Afamin (AFAM), Alpha-1-acid glycoprotein 1 & 2 (AGP12), Alpha-1-acid glycoprotein 1 (AGP1), Alpha-1-acid glycoprotein 2 (AGP2), Apolipoprotein A-I (APOA1), Apolipoprotein C-III (APOC3), Apolipoprotein B-100 (APOB), Apolipoprotein D (APOD), Beta-2-glycoprotein-1 (APOH), Apolipoprotein M (APOM), Attractin (ATRN), Calpain-3 (CAN3), Ceruloplasmin (CERU), ComplementFactorH (CFAH), ComplementFactorI (CFAI), Clusterin (CLUS), ComplementC3 (CO3), ComplementC4-A&B (CO4A&CO4B), ComplementcomponentC6 (CO6),
ComplementComponentC8AChain (CO8A), Coagulation factor XII (FA12), Alpha-2-HS-glycoprotein (FETUA), Haptoglobin (HPT), Histidine-rich Glycoprotein (HRG), Immunoglobulin heavy constant alpha 1&2 (IgA12),
Immunoglobulin heavy constant alpha 2 (IgA2),
Immunoglobulin heavy constant gamma 2 (IgG2), Immunoglobulin heavy constant mu (IgM), Inter-alpha-trypsin inhibitor heavy chain H1 (ITIH1), Plasma Kallikrein (KLKB 1), Kininogen-1 (KNG1), Serum paraoxonase/arylesterase 1 (PON1), Selenoprotein P (SEPP1), Prothrombin (THRB), Serotransferrin (TRFE), Transthyretin (TTR), Protein unc-13HomologA (UN13A), Vitronectin (VTNC), Zinc-alpha-2-glycoprotein (ZA2G), Insulin-like growth factor-II (IGF2), Apolipoprotein C-I (APOC1), and combinations thereof.

In the methods as claimed, the method may include detecting a glycopeptide, a glycan on the glycopeptide and the glycosylation site residue where the glycan bonds to the glycopeptide. In certain examples, the method includes detecting a glycan residue. In some examples, the method includes detecting a glycosylation site on a glycopeptide. In some examples, this process is accomplished with mass spectroscopy used in tandem with liquid chromatography.

In the methods as claimed, the biological sample has been obtained from a patient. In some examples, the biological sample is synovial fluid, whole blood, blood serum, blood plasma, urine, sputum, tissue, saliva, tears, spinal fluid, tissue section(s) obtained by biopsy; cell(s) that are placed in or adapted to tissue culture; sweat, mucous, fecal material, gastric fluid, abdominal fluid, amniotic fluid, cyst fluid, peritoneal fluid, pancreatic juice, breast milk, lung lavage, marrow, gastric acid, bile, semen, pus, aqueous humour, transudate, or combinations of the foregoing. In certain examples, the biological sample is selected from the group consisting of blood, plasma, saliva, mucus, urine, stool, tissue, sweat, tears, hair, or a combination thereof. In some of these examples, the biological sample is a blood sample. In some of these examples, the biological sample is a plasma sample. In some of these examples, the biological sample is a saliva sample. In some of these examples, the biological sample is a mucus sample. In some of these examples, the biological sample is a urine sample. In some of these examples, the biological sample is a stool sample. In some of these examples, the biological sample is a sweat sample. In some of these examples, the biological sample is a tear sample. In some of these examples, the biological sample is a hair sample.

In the methods as claimed, the method may also include digesting and/or fragmenting a glycopeptide in the sample. In certain examples, the method includes digesting a glycopeptide in the sample. In certain examples, the method includes fragmenting a glycopeptide in the sample. In some examples, the digested or fragmented glycopeptide is analyzed using mass spectroscopy. In some examples, the glycopeptide is digested or fragmented in the solution phase using digestive enzymes. In some examples, the glycopeptide is digested or fragmented in the gaseous phase inside a mass spectrometer, or the instrumentation associated with a mass spectrometer. In the methods as claimed, the mass spectroscopy results are analyzed using machine learning algorithms. In the methods as claimed, the mass spectroscopy results are the quantification of the glycopeptides, glycans, peptides, and fragments thereof. In the methods as claimed, this quantification is used as an input in a trained model to generate an output probability. The output probability is a probability of being within a given category or classification, e.g., the classification of having ovarian cancer or the classification of not having ovarian cancer. In some other examples, the output probability is a probability of being within a given category or classification, e.g., the classification of having cancer or the classification of not having cancer. In some other examples, the output probability is a probability of being within a given category or classification, e.g., the classification of having an autoimmune disease or the classification of not having an autoimmune disease. In some other examples, the output probability is a probability of being within a given category or classification, e.g., the classification of having fibrosis or the classification of not having an fibrosis.

In the methods as claimed, the method includes introducing the sample, or a portion thereof, into a mass spectrometer.

In the methods as claimed, the method may include fragmenting a glycopeptide in the sample after introducing the sample, or a portion thereof, into the mass spectrometer.

In the methods as claimed, the mass spectroscopy is performed using multiple reaction monitoring (MRM) mode. In some examples, the mass spectroscopy is performed using QTOF MS in data-dependent acquisition. In some examples, the mass spectroscopy is performed using or MS-only mode. In some examples, an immunoassay is used in combination with mass spectroscopy. In some examples, the immunoassay measures CA-125 and HE4.

In the methods as claimed, the method may include digesting a glycopeptide in the sample occurs before introducing the sample, or a portion thereof, into the mass spectrometer.

In the methods as claimed, the method may include fragmenting a glycopeptide in the sample to provide a glycopeptide ion, a peptide ion, a glycan ion, a glycan adduct ion, or a glycan fragment ion.

In the methods as claimed, the method may include digesting and/or fragmenting a glycopeptide in the sample to provide a glycopeptide consisting of an amino acid sequence selected from the group consisting of SEQ ID NOs: 1 - 76, and combinations thereof.

In the methods as claimed, the method may include digesting and/or fragmenting a glycopeptide in the sample to provide a glycopeptide consisting essentially of an amino acid sequence selected from the group consisting of SEQ ID NOs: 1 - 76, and combinations thereof.

In the methods as claimed, the method may include digesting a glycopeptide in the sample to provide a glycopeptide consisting of an amino acid sequence selected from the group consisting of SEQ ID NOs: 1 - 76, and combinations thereof.

In the methods as claimed, the method may include digesting a glycopeptide in the sample to provide a glycopeptide consisting essentially of an amino acid sequence selected from the group consisting of SEQ ID NOs: 1 - 76, and combinations thereof.

In the methods as claimed, the method may include fragmenting a glycopeptide in the sample to provide a glycopeptide consisting of an amino acid sequence selected from the group consisting of SEQ ID NOs: 1 - 76, and combinations thereof.

In the methods as claimed, the method may include fragmenting a glycopeptide in the sample to provide a glycopeptide consisting essentially of an amino acid sequence selected from the group consisting of SEQ ID NOs: 1 - 76, and combinations thereof.

In the methods as claimed, the method may include fragmenting a glycopeptide in the sample to provide a glycopeptide consisting of an amino acid sequence selected from the group consisting of SEQ ID NOs: 1, 3, 4, 5, 8, 11, 12, 13, 14, 16, 17, 18, 19, 20, 22, 23, 24, 25, 27, 30, 32, 34, 43, 45, 51, 54, 55, 65, 68, 71, 73, 74, 75, and combinations thereof.

In the methods as claimed, the method may include fragmenting a glycopeptide in the sample to provide a glycopeptide consisting essentially of an amino acid sequence selected from the group consisting of SEQ ID NOs: 1, 3, 4, 5, 8, 11, 12, 13, 14, 16, 17, 18, 19, 20, 22, 23, 24, 25, 27, 30, 32, 34, 43, 45, 51, 54, 55, 65, 68, 71, 73, 74, 75, and combinations thereof.

In the methods as claimed, the method may include fragmenting a glycopeptide in the sample to provide a glycopeptide consisting of an amino acid sequence selected from the group consisting of SEQ ID NOs: 2, 6, 7, 9, 10, 15, 21, 26, 28, 29, 31, 33, 35, 36, 37, 38, 39, 40, 41, 42, 44, 46, 47, 48, 49, 50, 52, 53, 56, 57, 58, 59, 60, 61, 62, 63, 64, 66, 67, 69, 70, 72, 76, and combinations thereof.

In the methods as claimed, the method may include fragmenting a glycopeptide in the sample to provide a glycopeptide consisting essentially of an amino acid sequence selected from the group consisting of SEQ ID NOs: 2, 6, 7, 9, 10, 15, 21, 26, 28, 29, 31, 33, 35, 36, 37, 38, 39, 40, 41, 42, 44, 46, 47, 48, 49, 50, 52, 53, 56, 57, 58, 59, 60, 61, 62, 63, 64, 66, 67, 69, 70, 72, 76, and combinations thereof.

In the methods as claimed, the method may include fragmenting a glycopeptide in the sample to provide a glycopeptide consisting of an amino acid sequence selected from the group consisting of SEQ ID NOs: 1, 6, 7, 8, 10, 15, 21, 22, 23, 42, 47, 48, 51, 56, 57, 58, 62, 74, and 76, and combinations thereof.

In the methods as claimed, the method may include fragmenting a glycopeptide in the sample to provide a glycopeptide consisting essentially of an amino acid sequence selected from the group consisting of SEQ ID NOs: 1, 6, 7, 8, 10, 15, 21, 22, 23, 42, 47, 48, 51, 56, 57, 58, 62, 74, and 76, and combinations thereof.

In the methods as claimed, the method includes detecting a multiple-reaction-monitoring (MRM) transition selected from the group consisting of transitions 1 - 3. In some examples, the method includes detecting a MRM transition indicative of a glycopeptide or glycan residue, wherein the glycopeptide consists essentially of an amino acid sequence selected from the group consisting of SEQ ID NOs: 1 - 3 and combinations thereof. In some examples, the method includes detecting a MRM transition indicative of a glycopeptide consisting essentially of an amino acid sequence selected from the group consisting of SEQ ID NOs: 1 - 3 and combinations thereof. In some examples, the method includes detecting more than one MRM transition selected from a combination of members from the group consisting of transitions 1 - 3. In some examples, the method includes detecting more than one MRM transition indicative of a combination of glycopeptides having amino acid sequences selected from a combination of SEQ ID NOs: 1 - 3.

In the methods as claimed, the method includes performing mass spectroscopy on the biological sample using multiple-reaction-monitoring mass spectroscopy (MRM-MS).

In the methods as claimed, the method may include digesting a glycopeptide in the sample to provide a glycopeptide consisting essentially of an amino acid sequence selected from the group consisting of SEQ ID NOs: 1 - 76, and combinations thereof. In certain examples, the biological sample is combined with chemical reagents. In certain examples, the biological sample is combined with enzymes. In some examples, the enzymes are lipases. In some examples, the enzymes are proteases. In some examples, the enzymes are serine proteases. In some of these examples, the enzyme is selected from the group consisting of trypsin, chymotrypsin, thrombin, elastase, and subtilisin. In some of these examples, the enzyme is trypsin. In some examples, the methods includes contacting at least two proteases with a glycopeptide in a sample. In some examples, the at least two proteases are selected from the group consisting of serine protease, threonine protease, cysteine protease, aspartate protease. In some examples, the at least two proteases are selected from the group consisting of trypsin, chymotrypsin, endoproteinase, Asp-N, Arg-C, Glu-C, Lys-C, pepsin, thermolysin, elastase, papain, proteinase K, subtilisin, clostripain, and carboxypeptidase protease, glutamic acid protease, metalloprotease, and asparagine peptide lyase.

In the methods as claimed, the method includes detecting a multiple-reaction-monitoring (MRM) transition selected from the group consisting of transitions 1 - 3. In some examples, the method includes detecting a MRM transition indicative of a glycopeptide or glycan residue, wherein the glycopeptide consisting of, or consisting essentially of, an amino acid sequence selected from the group consisting of SEQ ID NOs: 1 - 3 and combinations thereof. In some examples, the method includes detecting a MRM transition indicative of a glycopeptide or glycan residue, wherein the glycopeptide consists essentially of an amino acid sequence selected from the group consisting of SEQ ID NOs: 1 - 3 and combinations thereof. In some examples, the method includes detecting a MRM transition indicative of a glycopeptide consisting essentially of an amino acid sequence selected from the group consisting of SEQ ID NOs: 1 - 3 and combinations thereof. In some examples, the method includes detecting more than one MRM transition selected from a combination of members from the group consisting of transitions 1 - 3. In some examples, the method includes detecting more than one MRM transition indicative of a combination of glycopeptides having amino acid sequences selected from a combination of SEQ ID NOs: 1 - 3.

In the methods as claimed, the method includes performing mass spectroscopy on the biological sample using multiple-reaction-monitoring mass spectroscopy (MRM-MS).

In the methods as claimed, the method may include digesting a glycopeptide in the sample to provide a glycopeptide consisting of an amino acid sequence selected from the group consisting of SEQ ID NOs: 1 - 76, and combinations thereof. In certain examples, the biological sample is contacted with one or more chemical reagents. In certain examples, the biological sample is contacted with one or more enzymes. In some examples, the enzymes are lipases. In some examples, the enzymes are proteases. In some examples, the enzymes are serine proteases. In some of these examples, the enzyme is selected from the group consisting of trypsin, chymotrypsin, thrombin, elastase, and subtilisin. In some of these examples, the enzyme is trypsin. In some examples, the methods includes contacting at least two proteases with a glycopeptide in a sample. In some examples, the at least two proteases are selected from the group consisting of serine protease, threonine protease, cysteine protease, aspartate protease. In some examples, the at least two proteases are selected from the group consisting of trypsin, chymotrypsin, endoproteinase, Asp-N, Arg-C, Glu-C, Lys-C, pepsin, thermolysin, elastase, papain, proteinase K, subtilisin, clostripain, and carboxypeptidase protease, glutamic acid protease, metalloprotease, and asparagine peptide lyase.

The MRM transition may be selected from the transitions, or any combinations thereof, in any one of Tables 1, 2 or 3.

In the methods as claimed, the method may include conducting tandem liquid chromatography-mass spectroscopy on the biological sample.

In the methods as claimed, the method includes multiple-reaction-monitoring mass spectroscopy (MRM-MS) mass spectroscopy on the biological sample.

In the methods as claimed, the method includes detecting a MRM transition using a triple quadrupole (QQQ) and/or a quadrupole time-of-flight (qTOF) mass spectrometer. In certain examples, the method includes detecting a MRM transition using a QQQ mass spectrometer. In certain other examples, the method includes detecting using a qTOF mass spectrometer. In some examples, a suitable instrument for use with the instant methods is an Agilent 6495B Triple Quadrupole LC/MS, which can be found at www.agilent.com/en/products/mass-spectrometry/lc-ms-instruments/triple-quadrupole-lc-ms/6495b-triple-quadrupole-lc-ms.In certain other examples, the method includes detecting using a QQQ mass spectrometer. In some examples, a suitable instrument for use with the instant methods is an Agilent 6545 LC/Q-TOF, which can be found at https://www.agilent.com/en/products/liquid-chromatography-mass-spectrometry-lc-ms/lc-ms-instruments/quadrupole-time-of-flight-lc-ms/6545-q-tof-lc-ms.

In the methods as claimed, the method may include detecting more than one MRM transition using a QQQ and/or qTOF mass spectrometer. In certain examples, the method includes detecting more than one MRM transition using a QQQ mass spectrometer. In certain examples, the method includes detecting more than one MRM transition using a qTOF mass spectrometer. In certain examples, the method includes detecting more than one MRM transition using a QQQ mass spectrometer.

In the methods as claimed, the method may include quantifying one or more glycomic parameters of the one or more biological samples comprises employing a coupled chromatography procedure. In some examples, these glycomic parameters include the identification of a glycopeptide group, identification of glycans on the glycopeptide, identification of a glycosylation site, identification of part of an amino acid sequence which the glycopeptide includes. In some examples, the coupled chromatography procedure comprises: performing or effectuating a liquid chromatography-mass spectrometry (LC-MS) operation. In some examples, the coupled chromatography procedure comprises: performing or effectuating a multiple reaction monitoring mass spectrometry (MRM-MS) operation. In some examples, the methods herein include a coupled chromatography procedure which comprises: performing or effectuating a liquid chromatography-mass spectrometry (LC-MS) operation; and effectuating a multiple reaction monitoring mass spectrometry (MRM-MS) operation. In some examples, the methods include training a machine learning algorithm using one or more glycomic parameters of the one or more biological samples obtained by one or more of a triple quadrupole (QQQ) mass spectrometry operation and/or a quadrupole time-of-flight (qTOF) mass spectrometry operation. In some examples, the methods include training a machine learning algorithm using one or more glycomic parameters of the one or more biological samples obtained by a triple quadrupole (QQQ) mass spectrometry operation. In some examples, the methods include training a machine learning algorithm using one or more glycomic parameters of the one or more biological samples obtained by a quadrupole time-of-flight (qTOF) mass spectrometry operation. In some examples, the methods include quantifying one or more glycomic parameters of the one or more biological samples comprises employing one or more of a triple quadrupole (QQQ) mass spectrometry operation and a quadrupole time-of-flight (qTOF) mass spectrometry operation. In some examples, machine learning algorithms are used to quantify these glycomic parameters. In some examples, including any of the foregoing, the mass spectroscopy is performed using multiple reaction monitoring (MRM) mode. In some examples, the mass spectroscopy is performed using QTOF MS in data-dependent acquisition. In some examples, the mass spectroscopy is performed using or MS-only mode. In some examples, an immunoassay (e.g., ELISA) is used in combination with mass spectroscopy. In some examples, the immunoassay measures CA-125 and HE4 proteins.

In the methods as claimed, the glycopeptide or combination thereof may consist of an amino acid sequence selected from the group consisting of SEQ ID NOs: 1 - 3 and combinations thereof.

In the methods as claimed, the glycopeptide or combination thereof may consist essentially of an amino acid sequence selected from the group consisting of SEQ ID NOs: 1 - 3 and combinations thereof.

The glycopeptide or combination thereof may consist of an amino acid sequence selected from the group consisting of SEQ ID NOs: 1, 3, 4, 5, 8, 11, 12, 13, 14, 16, 17, 18, 19, 20, 22, 23, 24, 25, 27, 30, 32, 34, 43, 45, 51, 54, 55, 65, 68, 71, 73, 74, and 75 and combinations thereof.

The glycopeptide or combination thereof may consist essentially of an amino acid sequence selected from the group consisting of SEQ ID NOs: 1, 3, 4, 5, 8, 11, 12, 13, 14, 16, 17, 18, 19, 20, 22, 23, 24, 25, 27, 30, 32, 34, 43, 45, 51, 54, 55, 65, 68, 71, 73, 74, and 75 and combinations thereof.

The glycopeptide or combination thereof may consist of an amino acid sequence selected from the group consisting of SEQ ID NOs: 1, 6, 7, 8, 10, 15, 21, 22, 23, 42, 47, 48, 51, 56, 57, 58, 62, 74, and 76 and combinations thereof.
The glycopeptide or combination thereof may consist essentially of an amino acid sequence selected from the group consisting of SEQ ID NOs: 1, 6, 7, 8, 10, 15, 21, 22, 23, 42, 47, 48, 51, 56, 57, 58, 62, 74, and 76 and combinations thereof.
The glycopeptide or combination thereof may consist of an amino acid sequence selected from the group consisting of SEQ ID NOs: 2, 6, 7, 9, 10, 15, 21, 26, 28, 29, 31, 33, 35, 36, 37, 38, 39, 40, 41, 42, 44, 46, 47, 48, 49, 50, 52, 53, 56, 57, 58, 59, 60, 61, 62, 63, 64, 66, 67, 69, 70, 72, and 76 and combinations thereof.
The glycopeptide or combination thereof may consist essentially of an amino acid sequence selected from the group consisting of SEQ ID NOs: 2, 6, 7, 9, 10, 15, 21, 26, 28, 29, 31, 33, 35, 36, 37, 38, 39, 40, 41, 42, 44, 46, 47, 48, 49, 50, 52, 53, 56, 57, 58, 59, 60, 61, 62, 63, 64, 66, 67, 69, 70, 72, and 76 and combinations thereof.

The glycopeptide or combination thereof may consist of an amino acid sequence selected from the group consisting of SEQ ID NOs: 1, 6, 7, 8, 10, 15, 21, 22, 23, 42, 47, 48, 51, 56, 57, 58, 62, 74, and 76 and combinations thereof.

The glycopeptide or combination thereof may consist essentially of an amino acid sequence selected from the group consisting of SEQ ID NOs: 1, 6, 7, 8, 10, 15, 21, 22, 23, 42, 47, 48, 51, 56, 57, 58, 62, 74, and 76 and combinations thereof.

In the methods as claimed, the method may include digesting and/or fragmenting a glycopeptide in the sample to provide a glycopeptide consisting of an amino acid sequence selected from the group consisting of SEQ ID NOs: 1 - 76 and combinations thereof.

In the methods as claimed, the method may include digesting and/or fragmenting a glycopeptide in the sample to provide a glycopeptide consisting essentially of an amino acid sequence selected from the group consisting of SEQ ID NOs: 1 - 76 and combinations thereof.

In the methods as claimed, the method may include digesting and/or fragmenting a glycopeptide in the sample to provide a glycopeptide consisting of an amino acid sequence selected from the group consisting of SEQ ID NOs: 1, 6, 7, 8, 10, 15, 21, 22, 23, 42, 47, 48, 51, 56, 57, 58, 62, 74, and 76 and combinations thereof.

In the methods as claimed, the method may include digesting and/or fragmenting a glycopeptide in the sample to provide a glycopeptide consisting essentially of an amino acid sequence selected from the group consisting of SEQ ID NOs: 1, 6, 7, 8, 10, 15, 21, 22, 23, 42, 47, 48, 51, 56, 57, 58, 62, 74, and 76 and combinations thereof.

In the methods as claimed, the method may include detecting one or more MRM transitions indicative of glycans selected from the group consisting of glycan 3200, 3210, 3300, 3310, 3320, 3400, 3410, 3420, 3500, 3510, 3520, 3600, 3610, 3620, 3630, 3700, 3710, 3720, 3730, 3740, 4200, 4210, 4300, 4301, 4310, 4311, 4320, 4400, 4401, 4410, 4411, 4420, 4421, 4430, 4431, 4500, 4501, 4510, 4511, 4520, 4521, 4530, 4531, 4540, 4541, 4600, 4601, 4610, 4611, 4620, 4621, 4630, 4631, 4641, 4650,4700, 4701, 4710, 4711, 4720, 4730, 5200, 5210, 5300, 5301, 5310, 5311, 5320, 5400, 5401, 5402, 5410, 5411, 5412, 5420, 5421, 5430, 5431, 5432, 5500, 5501, 5502, 5510, 5511, 5512, 5520, 5521, 5522, 5530, 5531, 5541, 5600, 5601, 5602, 5610, 5611, 5612, 5620, 5621, 5631, 5650, 5700, 5701, 5702, 5710, 5711, 5712, 5720, 5721, 5730, 5731, 6200, 6210, 6300, 6301, 6310, 6311, 6320, 6400, 6401, 6402, 6410, 6411, 6412, 6420, 6421, 6432, 6500, 6501, 6502, 6503, 6510, 6511, 6512, 6513, 6520, 6521, 6522, 6530, 6531, 6532, 6540, 6541, 6600, 6601, 6602, 6603, 6610, 6611, 6612, 6613, 6620, 6621, 6622, 6623, 6630, 6631, 6632, 6640, 6641, 6642, 6652, 6700, 6701, 6711, 6721, 6703, 6713, 6710, 6711, 6712, 6713, 6720, 6721, 6730, 6731, 6740, 7200, 7210, 7400, 7401, 7410, 7411, 7412, 7420, 7421, 7430, 7431, 7432, 7500, 7501, 7510, 7511, 7512, 7600, 7601, 7602, 7603, 7604, 7610, 7611, 7612, 7613, 7614, 7620, 7621, 7622, 7623, 7632, 7640, 7700, 7701, 7702, 7703, 7710, 7711, 7712, 7713, 7714, 7720, 7721, 7722, 7730, 7731, 7732, 7740, 7741, 7751, 8200, 9200, 9210, 10200, 11200, 12200, and combinations thereof. Herein, these glycans are illustrated in Figures 1-14.

In the methods as claimed, the method may include quantifying a glycan.

In the methods as claimed, the method may include quantifying a first glycan and quantifying a second glycan; and further comprising comparing the quantification of the first glycan with the quantification of the second glycan.

In the methods as claimed, the method may include associating the detected glycan with a peptide residue site, whence the glycan was bonded.

In the methods as claimed, the method may include generating a glycosylation profile of the sample.

In the methods as claimed, the method may include spatially profiling glycans on a tissue section associated with the sample. In some examples, including any of the foregoing, the method includes spatially profiling glycopeptides on a tissue section associated with the sample. In some examples, the method includes matrix-assisted laser desorption ionization time-of-flight mass spectrometry (MALDI-TOF) mass spectroscopy in combination with the methods herein.

In the methods as claimed, the method may include quantifying relative abundance of a glycan and/or a peptide.

In the methods as claimed, the method may include normalizing the amount of a glycopeptide by quantifying a glycopeptide consisting essentially of an amino acid sequence selected from the group consisting of SEQ ID NOs: 1 - 3, and combinations thereof and comparing that quantification to the amount of another chemical species. In some examples, the method includes normalizing the amount of a peptide by quantifying a glycopeptide consisting of an amino acid sequence selected from the group consisting of SEQ ID NOs: 1 - 3, and combinations thereof, and comparing that quantification to the amount of another glycopeptide consisting of an amino acid sequence selected from the group consisting of SEQ ID NOs: 1 - 3. In some examples, the method includes normalizing the amount of a peptide by quantifying a glycopeptide consisting essentially of an amino acid sequence selected from the group consisting of SEQ ID NOs: 1 - 3, and combinations thereof, and comparing that quantification to the amount of another glycopeptide consisting essentially of an amino acid sequence selected from the group consisting of SEQ ID NOs: 1 - 3.

### B. METHODS FOR CLASSIFYING SAMPLES COMPRISING GLYCOPEPTIDES

Set forth herein a method for identifying a classification for a sample, the method comprising: quantifying by mass spectroscopy (MS) one or more glycopeptides in a sample wherein the glycopeptides each, individually in each instance, comprises a glycopeptide consisting essentially of an amino acid sequence selected from the group consisting of, or consisting essentially of, SEQ ID NOs: 1 - 76, and combinations thereof; and inputting the quantification into a trained model to generate a output probability; determining if the output probability is above or below a threshold for a classification; and identifying a classification for the sample based on whether the output probability is above or below a threshold for a classification.

Set forth herein is a method for classifying glycopeptides, comprising: having obtained a biological sample from a patient; digesting and/or fragmenting a glycopeptide in the sample; detecting a multiple-reaction-monitoring (MRM) transition selected from the group consisting of transitions 1 - 76; and classifying the glycopeptides based on the MRM transitions detected. In some examples, a machine learning algorithm is used to train a model using the analyzed the MRM transitions as inputs. In some examples, a machine learning algorithm is trained using the MRM transitions as a training data set. In some examples, the methods herein include identifying glycopeptides, peptides, and glycans based on their mass spectroscopy relative abundance. In some examples, a machine learning algorithm or algorithms select and/or identify peaks in a mass spectroscopy spectrum.

Set forth herein is a method for classifying glycopeptides, comprising: having obtained a biological sample from an individual; digesting and/or fragmenting a glycopeptide in the sample; detecting a multiple-reaction-monitoring (MRM) transition selected from the group consisting of transitions 1 - 76; and classifying the glycopeptides based on the MRM transitions detected. In some examples, a machine learning algorithm is used to train a model using the analyzed the MRM transitions as inputs. In some examples, a machine learning algorithm is trained using the MRM transitions as a training data set. In some examples, the methods herein include identifying glycopeptides, peptides, and glycans based on their mass spectroscopy relative abundance. In some examples, a machine learning algorithm or algorithms select and/or identify peaks in a mass spectroscopy spectrum.

Set forth herein is a method of training a machine learning algorithm using MRM transitions as an input data set. Set forth herein is a method for identifying a classification for a sample, the method comprising quantifying by mass spectroscopy (MS) a glycopeptide in a sample wherein the glycopeptide consisting of, or consisting essentially of, an amino acid sequence selected from the group consisting of SEQ ID NOs: 1 - 76, and combinations thereof; and identifying a classification based on the quantification. In some examples, the quantifying includes determining the presence or absence of a glycopeptide, or combination of glycopeptides, in a sample. In some examples, the quantifying includes determining the relative abundance of a glycopeptide, or combination of glycopeptides, in a sample.

The sample may be a biological sample from a patient having a disease or condition.

In the methods as claimed, the patient has ovarian cancer.

The patient may have cancer.

The patient may have fibrosis.

The patient may have an autoimmune disease.

The disease or condition may be ovarian cancer.

In the methods as claimed, the MS is MRM-MS with a QQQ and/or qTOF mass spectrometer.

In the methods as claimed, the mass spectroscopy is performed using multiple reaction monitoring (MRM) mode. In some examples, the mass spectroscopy is performed using QTOF MS in data-dependent acquisition. In some examples, the mass spectroscopy is performed using or MS-only mode. In some examples, an immunoassay is used in combination with mass spectroscopy. In some examples, the immunoassay measures CA-125 and HE4.

In the methods as claimed, the machine learning algorithm may be selected from the group consisting of a deep learning algorithm, a neural network algorithm, an artificial neural network algorithm, a supervised machine learning algorithm, a linear discriminant analysis algorithm, a quadratic discriminant analysis algorithm, a support vector machine algorithm, a linear basis function kernel support vector algorithm, a radial basis function kernel support vector algorithm, a random forest algorithm, a genetic algorithm, a nearest neighbor algorithm, k-nearest neighbors, a naive Bayes classifier algorithm, a logistic regression algorithm, or a combination thereof. In certain examples, the machine learning algorithm is lasso regression.

In the methods as claimed, the method may include classifying a sample as within, or embraced by, a disease classification or a disease severity classification.

In the methods as claimed, the classification may be identified with 80 % confidence, 85 % confidence, 90 % confidence, 95 % confidence, 99 % confidence, or 99.9999 % confidence.

In the methods as claimed, the method may include quantifying by MS the glycopeptide in a sample at a first time point; quantifying by MS the glycopeptide in a sample at a second time point; and comparing the quantification at the first time point with the quantification at the second time point.

In the methods as claimed, the method may include quantifying by MS a different glycopeptide in a sample at a third time point; quantifying by MS the different glycopeptide in a sample at a fourth time point; and comparing the quantification at the fourth time point with the quantification at the third time point.

In the methods as claimed, the method may include monitoring the health status of a patient.

In the methods as claimed, monitoring the health status of a patient may include monitoring the onset and progression of disease in a patient with risk factors such as genetic mutations, as well as detecting cancer recurrence.

The method may include quantifying by MS a glycopeptide consisting of an amino acid sequence selected from the group consisting of SEQ ID NOs: 1 - 76.

The method may include quantifying by MS a glycopeptide consisting essentially of an amino acid sequence selected from the group consisting of SEQ ID NOs: 1 - 76.

The method may include quantifying by MS a glycopeptide consisting of an amino acid sequence selected from the group consisting of SEQ ID NOs: 1, 6, 7, 8, 10, 15, 21, 22, 23, 42, 47, 48, 51, 56, 57, 58, 62, 74, and 76.

The method may include quantifying by MS a glycopeptide consisting essentially of an amino acid sequence selected from the group consisting of SEQ ID NOs: 1, 6, 7, 8, 10, 15, 21, 22, 23, 42, 47, 48, 51, 56, 57, 58, 62, 74, and 76.

The method may include quantifying by MS one or more glycans selected from the group consisting of glycan 3200, 3210, 3300, 3310, 3320, 3400, 3410, 3420, 3500, 3510, 3520, 3600, 3610, 3620, 3630, 3700, 3710, 3720, 3730, 3740, 4200, 4210, 4300, 4301, 4310, 4311, 4320, 4400, 4401, 4410, 4411, 4420, 4421, 4430, 4431, 4500, 4501, 4510, 4511, 4520, 4521, 4530, 4531, 4540, 4541, 4600, 4601, 4610, 4611, 4620, 4621, 4630, 4631, 4641, 4650,4700, 4701, 4710, 4711, 4720, 4730, 5200, 5210, 5300, 5301, 5310, 5311, 5320, 5400, 5401, 5402, 5410, 5411, 5412, 5420, 5421, 5430, 5431, 5432, 5500, 5501, 5502, 5510, 5511, 5512, 5520, 5521, 5522, 5530, 5531, 5541, 5600, 5601, 5602, 5610, 5611, 5612, 5620, 5621, 5631, 5650, 5700, 5701, 5702, 5710, 5711, 5712, 5720, 5721, 5730, 5731, 6200, 6210, 6300, 6301, 6310, 6311, 6320, 6400, 6401, 6402, 6410, 6411, 6412, 6420, 6421, 6432, 6500, 6501, 6502, 6503, 6510, 6511, 6512, 6513, 6520, 6521, 6522, 6530, 6531, 6532, 6540, 6541, 6600, 6601, 6602, 6603, 6610, 6611, 6612, 6613, 6620, 6621, 6622, 6623, 6630, 6631, 6632, 6640, 6641, 6642, 6652, 6700, 6701, 6711, 6721, 6703, 6713, 6710, 6711, 6712, 6713, 6720, 6721, 6730, 6731, 6740, 7200, 7210, 7400, 7401, 7410, 7411, 7412, 7420, 7421, 7430, 7431, 7432, 7500, 7501, 7510, 7511, 7512, 7600, 7601, 7602, 7603, 7604, 7610, 7611, 7612, 7613, 7614, 7620, 7621, 7622, 7623, 7632, 7640, 7700, 7701, 7702, 7703, 7710, 7711, 7712, 7713, 7714, 7720, 7721, 7722, 7730, 7731, 7732, 7740, 7741, 7751, 8200, 9200, 9210, 10200, 11200, 12200, and combinations thereof. Herein, these glycans are illustrated in Figures 1-14.

The method may include diagnosing a patient with a disease or condition based on the quantification.

In some examples, including any of the foregoing, the method includes diagnosing the patient as having ovarian cancer based on the quantification.

The method may include treating the patient with a therapeutically effective amount of a therapeutic agent selected from the group consisting of a chemotherapeutic, an immunotherapy, a hormone therapy, a targeted therapy, a neoadjuvant therapy, surgery, and combinations thereof.

The method may include diagnosing an individual with a disease or condition based on the quantification.

The method may include diagnosing the individual as having an aging condition.

The method may include treating the individual with a therapeutically effective amount of an anti-aging agent. In some examples, the anti-aging agent is selected from hormone therapy. In some examples, the anti-aging agent is testosterone or a testosterone supplement or derivative. In some examples, the anti-aging agent is estrogen or an estrogen supplement or derivative.

### C. METHODS OF TREATMENT

Set forth herein is a method for treating a patient having a disease or condition, comprising measuring by mass spectroscopy a glycopeptide in a sample from the patient. The patient may be a human. The patient may be a female. The patient may be a female with ovarian cancer. The patient may be a female with ovarian cancer at Stage 1. The patient may be a female with ovarian cancer at Stage 2. The patient may be a female with ovarian cancer at Stage 3. The patient may be a female with ovarian cancer at Stage 4. The female may have an age equal or between 10-20 years. The female may have an age equal or between 20-30 years. The female may have an age equal or between 30-40 years. The female may have an age equal or between 40-50 years. The female may have an age equal or between 50-60 years. The female may have an age equal or between 60-70 years. The female may have an age equal or between 70-80 years. The female may have an age equal or between 80-90 years. The female may have an age equal or between 90-100 years.

Set forth herein is a method for treating a patient having ovarian cancer; the method comprising: having obtained a biological sample from the patient; digesting and/or fragmenting one or more glycopeptides in the sample; and detecting and quantifying one or more multiple-reaction-monitoring (MRM) transitions selected from the group consisting of transitions 1 - 76; inputting the quantification into a trained model to generate an output probability; determining if the output probability is above or below a threshold for a classification; and classifying the patient based on whether the output probability is above or below a threshold for a classification, wherein the classification is selected from the group consisting of: (A) a patient in need of a chemotherapeutic agent; (B) a patient in need of a immunotherapeutic agent; (C) a patient in need of hormone therapy; (D) a patient in need of a targeted therapeutic agent; (E) a patient in need of surgery; (F) a patient in need of neoadjuvant therapy; (G) a patient in need of chemotherapeutic agent, immunotherapeutic agent, hormone therapy, targeted therapeutic agent, neoadjuvant therapy, or a combination thereof, before surgery; (H) a patient in need of chemotherapeutic agent, immunotherapeutic agent, hormone therapy, targeted therapeutic agent, neoadjuvant therapy, or a combination thereof, after surgery; (I) or a combination thereof; administering a therapeutically effective amount of a therapeutic agent to the patient: wherein the therapeutic agent is selected from chemotherapy if classification A or I is determined; wherein the therapeutic agent is selected from immunotherapy if classification B or I is determined; or wherein the therapeutic agent is selected from hormone therapy if classification C or I is determined; or wherein the therapeutic agent is selected from targeted therapy if classification D or I is determined wherein the therapeutic agent is selected from neoadjuvant therapy if classification F or I is determined; wherein the therapeutic agent is selected from chemotherapeutic agent, immunotherapeutic agent, hormone therapy, targeted therapeutic agent, neoadjuvant therapy, or a combination thereof if classification G or I is determined; and wherein the therapeutic agent is selected from chemotherapeutic agent, immunotherapeutic agent, hormone therapy, targeted therapeutic agent, neoadjuvant therapy, or a combination thereof if classification H or I is determined.

The machine learning may be used to identify MS peaks associated with MRM transitions. In some examples, the MRM transitions are analyzed using machine learning. In some examples, the machine learning is used to train a model based on the quantification of the amount of glycopeptides associated with an MRM transition(s). In some examples, the MRM transitions are analyzed with a trained machine learning algorithm. In some of these examples, the trained machine learning algorithm was trained using MRM transitions observed by analyzing samples from patients known to have ovarian cancer.

The patient may be treated with a therapeutic agent selected from targeted therapy. In some examples, the methods herein include administering a therapeutically effective amount of a (poly(ADP)-ribose polymerase) (PARP) inhibitor if combination D is detected. In some examples, the therapeutic agent is selected from Olaparib (Lynparza), Rucaparib (Rubraca), and Niraparib (Zejula).

In some examples, the patient is an adult with platinum-sensitive relapsed high-grade epithelial ovarian, fallopian tube, or primary peritoneal cancer.

In some examples, the therapeutic agent is administered at 150 mg, 250 mg, 300 mg, 350 mg, and 600 mg doses. In some examples, the therapeutic agent is administered twice daily.

Chemotherapeutic agents include, but are not limited to, platinum-based drug such as carboplatin (Paraplatin) or cisplatin with a taxane such as paclitaxel (Taxol) or docetaxel (Taxotere). Paraplatin may be administered at 10mg/mL injectable concentrations (in vials of 50, 150, 450, and 600 mg). For advanced ovarian carcinoma a single agent dose of 360 mg/m² IV for 4 weeks may be administered. Paraplatin may be administered in combination = as 300 mg/m² IV (plus cyclophosphamide 600 mg/m² IV) q4Weeks. Taxol may be administered at 175 mg/m² IV over 3 hours q3Weeks (follow with cisplatin). Taxol may be administered at 135 mg/m² IV over 24 hours q3Weeks (follow with cisplatin). Taxol may be administered at 135-175 mg/m² IV over 3 hours q3Weeks.

Immunotherapeutic agents include, but are not limited to, Zejula (Niraparib). Niraparib may be administered at 300 mg PO qDay.

Hormone therapeutic agents include, but are not limited to, Luteinizing-hormone-releasing hormone (LHRH) agonists, Tamoxifen, and Aromatase inhibitors.

Targeted therapeutic agents include, but are not limited to, PARP inhibitors.

In some examples, including any of the foregoing, the method includes conducting multiple-reaction-monitoring mass spectroscopy (MRM-MS) on the biological sample.

In some examples, including any of the foregoing, the mass spectroscopy is performed using multiple reaction monitoring (MRM) mode. In some examples, the mass spectroscopy is performed using QTOF MS in data-dependent acquisition. In some examples, the mass spectroscopy is performed using or MS-only mode. In some examples, an immunoassay (*e.g.*, ELISA) is used in combination with mass spectroscopy. In some examples, the immunoassay measures CA-125 and HE4.

In some examples, including any of the foregoing, the method includes quantifying one or more glycopeptides consisting of an amino acid sequence selected from the group consisting of SEQ ID NOs: 1 - 76 and combinations thereof.

In some examples, including any of the foregoing, the method includes quantifying one or more glycopeptides consisting essentially of an amino acid sequence selected from the group consisting of SEQ ID NOs: 1, 3, 4, 5, 8, 11, 12, 13, 14, 16, 17, 18, 19, 20, 22, 23, 24, 25, 27, 30, 32, 34, 43, 45, 51, 54, 55, 65, 68, 71, 73, 74, 75, and combinations thereof.

In some examples, including any of the foregoing, the method includes quantifying one or more glycopeptides consisting of an amino acid sequence selected from the group consisting of SEQ ID NOs: 1, 3, 4, 5, 8, 11, 12, 13, 14, 16, 17, 18, 19, 20, 22, 23, 24, 25, 27, 30, 32, 34, 43, 45, 51, 54, 55, 65, 68, 71, 73, 74, 75, and combinations thereof.

In some examples, including any of the foregoing, the method includes quantifying one or more glycopeptides consisting essentially of an amino acid sequence selected from the group consisting of SEQ ID NOs: 2, 6, 7, 9, 10, 15, 21, 26, 28, 29, 31, 33, 35, 36, 37, 38, 39, 40, 41, 42, 44, 46, 47, 48, 49, 50, 52, 53, 56, 57, 58, 59, 60, 61, 62, 63, 64, 66, 67, 69, 70, 72, 76, and combinations thereof.

In some examples, including any of the foregoing, the method includes quantifying one or more glycopeptides consisting of an amino acid sequence selected from the group consisting of SEQ ID NOs: 2, 6, 7, 9, 10, 15, 21, 26, 28, 29, 31, 33, 35, 36, 37, 38, 39, 40, 41, 42, 44, 46, 47, 48, 49, 50, 52, 53, 56, 57, 58, 59, 60, 61, 62, 63, 64, 66, 67, 69, 70, 72, 76, and combinations thereof.

In some examples, including any of the foregoing, the method includes quantifying one or more glycopeptides consisting essentially of an amino acid sequence selected from the group consisting of SEQ ID NOs: 1, 6, 7, 8, 10, 15, 21, 22, 23, 42, 47, 48, 51, 56, 57, 58, 62, 74, 76, and combinations thereof.

In some examples, including any of the foregoing, the method includes quantifying one or more glycopeptides consisting of an amino acid sequence selected from the group consisting of SEQ ID NOs: 1, 6, 7, 8, 10, 15, 21, 22, 23, 42, 47, 48, 51, 56, 57, 58, 62, 74, 76, and combinations thereof.

In some examples, including any of the foregoing, the method includes quantifying one or more glycopeptides consisting essentially of an amino acid sequence selected from the group consisting of SEQ ID NOs: 1 - 76 and combinations thereof.

In some examples, including any of the foregoing, the method includes detecting a multiple-reaction-monitoring (MRM) transition selected from the group consisting of transitions 1 - 76 using a QQQ and/or a qTOF mass spectrometer.

In some examples, including any of the foregoing, the method includes training a machine learning algorithm to identify a classification based on the quantifying step.

In some examples, including any of the foregoing, the method includes using a machine learning algorithm to identify a classification based on the quantifying step.

In some examples, including any of the foregoing, the machine learning algorithm is selected from the group consisting of a deep learning algorithm, a neural network algorithm, an artificial neural network algorithm, a supervised machine learning algorithm, a linear discriminant analysis algorithm, a quadratic discriminant analysis algorithm, a support vector machine algorithm, a linear basis function kernel support vector algorithm, a radial basis function kernel support vector algorithm, a random forest algorithm, a genetic algorithm, a nearest neighbor algorithm, k-nearest neighbors, a naive Bayes classifier algorithm, a logistic regression algorithm, or a combination thereof.

### D. METHODS FOR DIAGNOSING PATIENTS

Set forth herein is a method for diagnosing a patient having a disease or condition, comprising measuring by mass spectroscopy a glycopeptide in a sample from the patient.

The invention provides a method for diagnosing a patient having ovarian cancer; the method comprising: performing mass spectroscopy of the biological sample that has been obtained from a patient, using MRM-MS with a QQQ and/or qTOF spectrometer to detect and quantify one or more MRM transitions selected from transitions 1-3 wherein each MRM transition is indicative of a corresponding alpha-1-antitrypsin (A1AT) glycopeptide in Table 1 wherein the analyzing the detected glycopeptides comprises using a machine learning algorithm; inputting the quantification of the detected glycopeptides or the MRM transitions into a trained model to generate an output probability, determining if the output probability is above or below a threshold for a classification; and identifying a diagnostic classification for the patient based on whether the output probability is above or below a threshold for a classification; and diagnosing the patient as having ovarian cancer based on the diagnostic classification.

Set forth herein is a method for diagnosing a patient having ovarian cancer; the method comprising: obtaining a biological sample from the patient; performing mass spectroscopy of the biological sample using MRM-MS with a QQQ and/or qTOF spectrometer to detect and quantify one or more glycopeptides consisting essentially of an amino acid sequence selected from the group consisting of SEQ ID NOs: 1-76; or to detect and quantify one or more MRM transitions selected from transitions 1, 6, 7, 8, 10, 15, 21, 22, 23, 42, 47, 48, 51, 56, 57, 58, 62, 74, and 76; inputting the quantification of the detected glycopeptides or the MRM transitions into a trained model to generate an output probability, determining if the output probability is above or below a threshold for a classification; and identifying a diagnostic classification for the patient based on whether the output probability is above or below a threshold for a classification; and diagnosing the patient as having ovarian cancer based on the diagnostic classification.

Set forth herein is a method for diagnosing a patient having ovarian cancer; the method comprising: inputting the quantification of detected glycopeptides or MRM transitions into a trained model to generate an output probability, determining if the output probability is above or below a threshold for a classification; and identifying a diagnostic classification for the patient based on whether the output probability is above or below a threshold for a classification; and diagnosing the patient as having ovarian cancer based on the diagnostic classification. In some examples, the method includes obtaining a biological sample from the patient; performing mass spectroscopy of the biological sample using MRM-MS with a QQQ and/or qTOF spectrometer to detect and quantify one or more glycopeptides consisting essentially of an amino acid sequence selected from the group consisting of SEQ ID NOs: 1 - 76; or to detect and quantify one or more MRM transitions selected from transitions 1-76.

Set forth herein is a method for diagnosing a patient having ovarian cancer; the method comprising: obtaining a biological sample from the patient; performing mass spectroscopy of the biological sample using MRM-MS with a QQQ and/or qTOF spectrometer to detect one or more glycopeptides consisting or, or consisting essentially of, an amino acid sequence selected from the group consisting of SEQ ID NOs: 1-76; or to detect one or more MRM transitions selected from transitions 1-76; analyzing the detected glycopeptides or the MRM transitions to identify a diagnostic classification; and diagnosing the patient as having ovarian cancer based on the diagnostic classification.

Set forth herein is a method for diagnosing a patient having ovarian cancer; the method comprising: analyzing detected or quantified glycopeptides or MRM transitions to identify a diagnostic classification; and diagnosing the patient as having ovarian cancer based on the diagnostic classification. In some examples, the method includes obtaining a biological sample from the patient; and performing mass spectroscopy of the biological sample using MRM-MS with a QQQ and/or qTOF spectrometer to detect one or more glycopeptides consisting or, or consisting essentially of, an amino acid sequence selected from the group consisting of SEQ ID NOs: 1-76; or to detect one or more MRM transitions selected from transitions 1-76.

Set forth herein is a method for diagnosing, monitoring, or classifying aging in an individual; the method comprising: obtaining a biological sample from the patient; performing mass spectroscopy of the biological sample using MRM-MS with a QQQ and/or qTOF spectrometer to detect one or more glycopeptides consisting or, or consisting essentially of, an amino acid sequence selected from the group consisting of SEQ ID NOs: 1-76; or to detect one or more MRM transitions selected from transitions 1-76; analyzing the detected glycopeptides or the MRM transitions to identify a diagnostic classification; and diagnosing, monitoring, or classifying the individual as having an aging classification based on the diagnostic classification.**E. DISEASES AND CONDITIONS**

Set forth herein are biomarkers for diagnosing a variety of diseases and conditions.

The diseases and conditions may include cancer. The diseases and conditions may not be limited to cancer.

The diseases and conditions may include fibrosis. The diseases and conditions may not be limited to fibrosis.

The diseases and conditions may include an autoimmune disease. The diseases and conditions may not be limited to an autoimmune disease.

In the methods as claimed, the diseases and conditions include ovarian cancer. The diseases and conditions may not be limited to ovarian cancer.

The condition may be aging. In some examples, the "patient" described herein is equivalently described as an "individual." Set forth are biomarkers for monitoring or diagnosing aging or aging conditions in an individual. In some of these examples, the individual is not necessarily a patient who has a medical condition in need of therapy. In some examples, the individual is a male. In some examples, the individual is a female. In some examples, the individual is a male mammal. In some examples, the individual is a female mammal. In some examples, the individual is a male human. In some examples, the individual is a female human.

In some examples, the individual is 1 year old. In some examples, the individual is 2 years old. In some examples, the individual is 3 years old. In some examples, the individual is 4 years old. In some examples, the individual is 5 years old. In some examples, the individual is 6 years old. In some examples, the individual is 7 years old. In some examples, the individual is 8 years old. In some examples, the individual is 9 years old. In some examples, the individual is 10 years old. In some examples, the individual is 11 years old. In some examples, the individual is 12 years old. In some examples, the individual is 13 years old. In some examples, the individual is 14 years old. In some examples, the individual is 15 years old. In some examples, the individual is 16 years old. In some examples, the individual is 17 years old. In some examples, the individual is 18 years old. In some examples, the individual is 19 years old. In some examples, the individual is 20 years old. In some examples, the individual is 21 years old. In some examples, the individual is 22 years old. In some examples, the individual is 23 years old. In some examples, the individual is 24 years old. In some examples, the individual is 25 years old. In some examples, the individual is 26 years old. In some examples, the individual is 27 years old. In some examples, the individual is 28 years old. In some examples, the individual is 29 years old. In some examples, the individual is 30 years old. In some examples, the individual is 31 years old. In some examples, the individual is 32 years old. In some examples, the individual is 33 years old. In some examples, the individual is 34 years old. In some examples, the individual is 35 years old. In some examples, the individual is 36 years old. In some examples, the individual is 37 years old. In some examples, the individual is 38 years old. In some examples, the individual is 39 years old. In some examples, the individual is 40 years old. In some examples, the individual is 41 years old. In some examples, the individual is 42 years old. In some examples, the individual is 43 years old. In some examples, the individual is 44 years old. In some examples, the individual is 45 years old. In some examples, the individual is 46 years old. In some examples, the individual is 47 years old. In some examples, the individual is 48 years old. In some examples, the individual is 49 years old. In some examples, the individual is 50 years old. In some examples, the individual is 51 years old. In some examples, the individual is 52 years old. In some examples, the individual is 53 years old. In some examples, the individual is 54 years old. In some examples, the individual is 55 years old. In some examples, the individual is 56 years old. In some examples, the individual is 57 years old. In some examples, the individual is 58 years old. In some examples, the individual is 59 years old. In some examples, the individual is 60 years old. In some examples, the individual is 61 years old. In some examples, the individual is 62 years old. In some examples, the individual is 63 years old. In some examples, the individual is 64 years old. In some examples, the individual is 65 years old. In some examples, the individual is 66 years old. In some examples, the individual is 67 years old. In some examples, the individual is 68 years old. In some examples, the individual is 69 years old. In some examples, the individual is 70 years old. In some examples, the individual is 71 years old. In some examples, the individual is 72 years old. In some examples, the individual is 73 years old. In some examples, the individual is 74 years old. In some examples, the individual is 75 years old. In some examples, the individual is 76 years old. In some examples, the individual is 77 years old. In some examples, the individual is 78 years old. In some examples, the individual is 79 years old. In some examples, the individual is 80 years old. In some examples, the individual is 81 years old. In some examples, the individual is 82 years old. In some examples, the individual is 83 years old. In some examples, the individual is 84 years old. In some examples, the individual is 85 years old. In some examples, the individual is 86 years old. In some examples, the individual is 87 years old. In some examples, the individual is 88 years old. In some examples, the individual is 89 years old. In some examples, the individual is 90 years old. In some examples, the individual is 91 years old. In some examples, the individual is 92 years old. In some examples, the individual is 93 years old. In some examples, the individual is 94 years old. In some examples, the individual is 95 years old. In some examples, the individual is 96 years old. In some examples, the individual is 97 years old. In some examples, the individual is 98 years old. In some examples, the individual is 99 years old. In some examples, the individual is 100 years old. In some examples, the individual is more than 100 years old.

### V. MACHINE LEARNING

In some examples, including any of the foregoing, the methods herein include quantifying one or more glycopeptides consisting essentially of an amino acid sequence selected from the group consisting of SEQ ID NOs: 1 - 76 using mass spectroscopy and/or liquid chromatography. In some examples, the quantification results are used as inputs in a trained model. In some examples, the quantification results are classified or categorized with a diagnostic algorithm based on the absolute amount, relative amount, and/or type of each glycan or glycopeptide quantified in the test sample, wherein the diagnostic algorithm is trained on corresponding values for each marker obtained from a population of individuals having known diseases or conditions. In some examples, the disease or condition is ovarian cancer.

In some examples, including any of the foregoing, set forth herein is a method for training a machine learning algorithm, comprising: providing a first data set of MRM transition signals indicative of a sample comprising a glycopeptide consisting essentially of an amino acid sequence selected from the group consisting of SEQ ID NOs: 1-76; providing a second data set of MRM transition signals indicative of a control sample; and comparing the first data set with the second data set using a machine learning algorithm.

In some examples, including any of the foregoing, the method herein include using a sample comprising a glycopeptide consisting of an amino acid sequence selected from the group consisting of SEQ ID NOs: 1-76 is a sample from a patient having ovarian cancer.

In some examples, including any of the foregoing, the method herein include using a sample comprising a glycopeptide consisting essentially of an amino acid sequence selected from the group consisting of SEQ ID NOs: 1-76 is a sample from a patient having ovarian cancer.

In some examples, including any of the foregoing, the method herein include using a control sample, wherein the control sample is a sample from a patient not having ovarian cancer.

In some examples, including any of the foregoing, the method herein include using a sample comprising a glycopeptide consisting essentially of an amino acid sequence selected from the group consisting of SEQ ID NOs: 1-76, which is a pooled sample from one or more patients having ovarian cancer.

In some examples, including any of the foregoing, the method herein include using a control sample, which is a pooled sample from one or more patients not having ovarian cancer.

In some examples, including any of the foregoing, the methods include generating machine learning models trained using mass spectrometry data (e.g., MRM-MS transition signals) from patients having a disease or condition and patients not having a disease or condition. In some examples, the disease or condition is ovarian cancer. In some examples, the methods include optimizing the machine learning models by cross-validation with known standards or other samples. In some examples, the methods include qualifying the performance using the mass spectrometry data to form panels of glycans and glycopeptides with individual sensitivities and specificities. In certain examples, the methods include determining a confidence percent in relation to a diagnosis. In some examples, one to ten glycopeptides consisting essentially of an amino acid sequence selected from the group consisting of SEQ ID NOs: 1 - 76 may be useful for diagnosing a patient with ovarian cancer with a certain confidence percent. In some examples, ten to fifty glycopeptides consisting essentially of an amino acid sequence selected from the group consisting of SEQ ID NOs: 1 - 76 may be useful for diagnosing a patient with ovarian cancer with a higher confidence percent.

In some examples, including any of the foregoing, the methods include performing MRM-MS and/or LC-MS on a biological sample. In some examples, the methods include constructing, by a computing device, theoretical mass spectra data representing a plurality of mass spectra, wherein each of the plurality of mass spectra corresponds to one or more glycopeptides consisting essentially of an amino acid sequence selected from the group consisting of SEQ ID NOs: 1 - 76. In some examples, the methods include comparing, by the computing device, the mass spectra data with the theoretical mass spectra data to generate comparison data indicative of a similarity of each of the plurality of mass spectra to each of the plurality of theoretical target mass spectra associated with a corresponding glycopeptide of the plurality of glycopeptides.

In some examples, machine learning algorithms are used to determine, by the computing device and based on the MRM-MS data, a distribution of a plurality of characteristic ions in the plurality of mass spectra; and determining, by the computing device and based on the distribution, whether one or more of the plurality of characteristic ions is a glycopeptide ion.

In some examples, the methods herein include training a diagnostic algorithm. Herein, training the diagnostic algorithm may refer to supervised learning of a diagnostic algorithm on the basis of values for one or more glycopeptides consisting of, or consisting essentially of, an amino acid sequence selected from the group consisting of SEQ ID NOs: 1 - 76. Training the diagnostic algorithm may refer to variable selection in a statistical model on the basis of values for one or more glycopeptides consisting essentially of an amino acid sequence selected from the group consisting of SEQ ID NOs: 1 - 76. Training a diagnostic algorithm may for example include determining a weighting vector in feature space for each category, or determining a function or function parameters.

In some examples, including any of the foregoing, the machine learning algorithm is selected from the group consisting of a deep learning algorithm, a neural network algorithm, an artificial neural network algorithm, a supervised machine learning algorithm, a linear discriminant analysis algorithm, a quadratic discriminant analysis algorithm, a support vector machine algorithm, a linear basis function kernel support vector algorithm, a radial basis function kernel support vector algorithm, a random forest algorithm, a genetic algorithm, a nearest neighbor algorithm, k-nearest neighbors, a naive Bayes classifier algorithm, a logistic regression algorithm, or a combination thereof. In certain examples, the machine learning algorithm is lasso regression.

In certain examples, the machine learning algorithm is LASSO, Ridge Regression, Random Forests, K-nearest Neighbors (KNN), Deep Neural Networks (DNN), and Principal Components Analysis (PCA). In certain examples, DNN's are used to process mass spec data into analysis-ready forms. In some examples, DNN's are used for peak picking from a mass spectra. In some examples, PCA is useful in feature detection.

In some examples, LASSO is used to provide feature selection.

In some examples, machine learning algorithms are used to quantify peptides from each protein that are representative of the protein abundance. In some examples, this quantification includes quantifying proteins for which glycosylation is not measured.

In some examples, glycopeptide sequences are identified by fragmentation in the mass spectrometer and database search using Byonic software.

In some examples, the methods herein include unsupervised learning to detect features of MRMS-MS data that represent known biological quantities, such as protein function or glycan motifs. In certain examples, these features are used as input for classifying by machine. In some examples, the classification is performed using LASSO, Ridge Regression, or Random Forest nature.

In some examples, the methods herein include mapping input data (*e.g.,* MRM transition peaks) to a value (e.g., a scale based on 0-100) before processing the value in an algorithm. For example, after a MRM transition is identified and the peak characterized, the methods herein include assessing the MS scans in an m/z and retention time window around the peak for a given patient. In some examples, the resulting chromatogram is integrated by a machine learning algorithm that determines the peak start and stop points, and calculates the area bounded by those points and the intensity (height). The resulting integrated value is the abundance, which then feeds into machine learning and statistical analyses training and data sets.

In some examples, machine learning output, in one instance, is used as machine learning input in another instance. For example, in addition to the PCA being used for a classification process, the DNN data processing feeds into PCA and other analyses. This results in at least three levels of algorithmic processing. Other hierarchical structures are contemplated within the scope of the instant disclosure.

In some examples, including any of the foregoing, the methods include comparing the amount of each glycan or glycopeptide quantified in the sample to corresponding reference values for each glycan or glycopeptide in a diagnostic algorithm. In some examples, the methods includes a comparative process by which the amount of a glycan or glycopeptide quantified in the sample is compared to a reference value for the same glycan or glycopeptide using a diagnostic algorithm. The comparative process may be part of a classification by a diagnostic algorithm. The comparative process may occur at an abstract level, *e.g.,* in n-dimensional feature space or in a higher dimensional space.

In some examples, the methods herein include classifying a patient's sample based on the amount of each glycan or glycopeptide quantified in the sample with a diagnostic algorithm. In some examples, the methods include using statistical or machine learning classification processes by which the amount of a glycan or glycopeptide quantified in the test sample is used to determine a category of health with a diagnostic algorithm. In some examples, the diagnostic algorithm is a statistical or machine learning classification algorithm.

In some examples, including any of the foregoing, classification by a diagnostic algorithm may include scoring likelihood of a panel of glycan or glycopeptide values belonging to each possible category, and determining the highest-scoring category. Classification by a diagnostic algorithm may include comparing a panel of marker values to previous observations by means of a distance function. Examples of diagnostic algorithms suitable for classification include random forests, support vector machines, logistic regression (e.g. multiclass or multinomial logistic regression, and/or algorithms adapted for sparse logistic regression). A wide variety of other diagnostic algorithms that are suitable for classification may be used, as known to a person skilled in the art.

In some examples, the methods herein include supervised learning of a diagnostic algorithm on the basis of values for each glycan or glycopeptide obtained from a population of individuals having a disease or condition (*e.g.*, ovarian cancer). In some examples, the methods include variable selection in a statistical model on the basis of values for each glycan or glycopeptide obtained from a population of individuals having ovarian cancer. Training a diagnostic algorithm may for example include determining a weighting vector in feature space for each category, or determining a function or function parameters.

In one embodiment, the reference value is the amount of a glycan or glycopeptide in a sample or samples derived from one individual. Alternatively, the reference value may be derived by pooling data obtained from multiple individuals, and calculating an average (for example, mean or median) amount for a glycan or glycopeptide. Thus, the reference value may reflect the average amount of a glycan or glycopeptide in multiple individuals. Said amounts may be expressed in absolute or relative terms, in the same manner as described herein.

In some examples, the reference value may be derived from the same sample as the sample that is being tested, thus allowing for an appropriate comparison between the two. For example, if the sample is derived from urine, the reference value is also derived from urine. In some examples, if the sample is a blood sample (e.g. a plasma or a serum sample), then the reference value will also be a blood sample (e.g. a plasma sample or a serum sample, as appropriate). When comparing between the sample and the reference value, the way in which the amounts are expressed is matched between the sample and the reference value. Thus, an absolute amount can be compared with an absolute amount, and a relative amount can be compared with a relative amount. Similarly, the way in which the amounts are expressed for classification with the diagnostic algorithm is matched to the way in which the amounts are expressed for training the diagnostic algorithm.

When the amounts of the glycan or glycopeptide are determined, the method may comprise comparing the amount of each glycan or glycopeptide to its corresponding reference value. When the cumulative amount of one, some or all the glycan or glycopeptides are determined, the method may comprise comparing the cumulative amount to a corresponding reference value. When the amounts of the glycan or glycopeptides are combined with each other in a formula to form an index value, the index value can be compared to a corresponding reference index value derived in the same manner.

The reference values may be obtained either within (*i.e.,* constituting a step of) or external to the (*i.e.,* not constituting a step of) methods described herein. In some examples, the methods include a step of establishing a reference value for the quantity of the markers. In other examples, the reference values are obtained externally to the method described herein and accessed during the comparison step of the invention.

In some examples, including any of the foregoing, training of a diagnostic algorithm may be obtained either within (*i.e.,* constituting a step of) or external to (*i.e.,* not constituting a step of) the methods set forth herein. In some examples, the methods include a step of training of a diagnostic algorithm. In some examples, the diagnostic algorithm is trained externally to the method herein and accessed during the classification step of the invention. The reference value may be determined by quantifying the amount of a glycan or glycopeptide in a sample obtained from a population of healthy individual(s). The diagnostic algorithm may be trained by quantifying the amount of a glycan or glycopeptide in a sample obtained from a population of healthy individual(s). As used herein, the term "healthy individual" refers to an individual or group of individuals who are in a healthy state, *e.g.,* patients who have not shown any symptoms of the disease, have not been diagnosed with the disease and/or are not likely to develop the disease. Preferably said healthy individual(s) is not on medication affecting the disease and has not been diagnosed with any other disease. The one or more healthy individuals may have a similar sex, age and body mass index (BMI) as compared with the test individual. The reference value may be determined by quantifying the amount of a glycan or glycopeptide in a sample obtained from a population of individual(s) suffering from the disease. The diagnostic algorithm may be trained by quantifying the amount of a marker in a sample obtained from a population of individual(s) suffering from the disease. More preferably such individual(s) may have similar sex, age and body mass index (BMI) as compared with the test individual. The reference value may be obtained from a population of individuals suffering from ovarian cancer. The diagnostic algorithm may be trained by quantifying the amount of a glycan or glycopeptide in a sample obtained from a population of individuals suffering from ovarian cancer. Once the characteristic glycan or glycopeptide profile of ovarian cancer is determined, the profile of markers from a biological sample obtained from an individual may be compared to this reference profile to determine whether the test subject also has ovarian cancer. Once the diagnostic algorithm is trained to classify ovarian cancer, the profile of markers from a biological sample obtained from an individual may be classified by the diagnostic algorithm to determine whether the test subject is also at that particular stage of ovarian cancer.

### VI. Kits

Set forth herein is a kit comprising a glycopeptide standard, a buffer, and one or more glycopeptides consisting of an amino acid sequence selected from the group consisting of SEQ ID NOs: 1 - 76.

Set forth herein is a kit comprising a glycopeptide standard, a buffer, and one or more glycopeptides consisting essentially of an amino acid sequence selected from the group consisting of SEQ ID NOs: 1 - 76.

Set forth herein is a kit comprising a glycopeptide standard, a buffer, and one or more glycopeptides consisting of an amino acid sequence selected from the group consisting of SEQ ID NOs: 1, 6, 7, 8, 10, 15, 21, 22, 23, 42, 47, 48, 51, 56, 57, 58, 62, 74, and 76, and combinations thereof.

Set forth herein is a kit comprising a glycopeptide standard, a buffer, and one or more glycopeptides consisting essentially of an amino acid sequence selected from the group consisting of SEQ ID NOs: 1, 6, 7, 8, 10, 15, 21, 22, 23, 42, 47, 48, 51, 56, 57, 58, 62, 74, and 76, and combinations thereof.

Set forth herein is a kit comprising a glycopeptide standard, a buffer, and one or more glycopeptides consisting of an amino acid sequence selected from the group consisting of SEQ ID NOs: 1, 3, 4, 5, 8, 11, 12, 13, 14, 16, 17, 18, 19, 20, 22, 23, 24, 25, 27, 30, 32, 34, 43, 45, 51, 54, 55, 65, 68, 71, 73, 74, 75, and combinations thereof.

Set forth herein is a kit comprising a glycopeptide standard, a buffer, and one or more glycopeptides consisting essentially of an amino acid sequence selected from the group consisting of SEQ ID NOs: 1, 3, 4, 5, 8, 11, 12, 13, 14, 16, 17, 18, 19, 20, 22, 23, 24, 25, 27, 30, 32, 34, 43, 45, 51, 54, 55, 65, 68, 71, 73, 74, 75, and combinations thereof.

Set forth herein is a kit comprising a glycopeptide standard, a buffer, and one or more glycopeptides consisting of an amino acid sequence selected from the group consisting of SEQ ID NOs: 2, 6, 7, 9, 10, 15, 21, 26, 28, 29, 31, 33, 35, 36, 37, 38, 39, 40, 41, 42, 44, 46, 47, 48, 49, 50, 52, 53, 56, 57, 58, 59, 60, 61, 62, 63, 64, 66, 67, 69, 70, 72, 76, and combinations thereof.

Set forth herein is a kit comprising a glycopeptide standard, a buffer, and one or more glycopeptides consisting essentially of an amino acid sequence selected from the group consisting of SEQ ID NOs: 2, 6, 7, 9, 10, 15, 21, 26, 28, 29, 31, 33, 35, 36, 37, 38, 39, 40, 41, 42, 44, 46, 47, 48, 49, 50, 52, 53, 56, 57, 58, 59, 60, 61, 62, 63, 64, 66, 67, 69, 70, 72, 76, and combinations thereof.

Set forth herein is a kit for diagnosing or monitoring cancer in an individual wherein the glycan or glycopeptide profile of a sample from said individual is determined and the measured profile is compared with a profile of a normal patient or a profile of a patient with a family history of cancer. The kit may comprise one or more glycopeptides consisting of an amino acid sequence selected from the group consisting of SEQ ID NOs: 1 - 76. The kit may comprise one or more glycopeptides consisting essentially of an amino acid sequence selected from the group consisting of SEQ ID NOs: 1 - 76. The kit may comprise one or more glycopeptides consisting of an amino acid sequence selected from the group consisting of SEQ ID NOs: 1, 6, 7, 8, 10, 15, 21, 22, 23, 42, 47, 48, 51, 56, 57, 58, 62, 74, and 76, and combinations thereof. The kit may comprise one or more glycopeptides consisting essentially of an amino acid sequence selected from the group consisting of SEQ ID NOs: 1, 6, 7, 8, 10, 15, 21, 22, 23, 42, 47, 48, 51, 56, 57, 58, 62, 74, and 76, and combinations thereof. The kit may comprise one or more glycopeptides consisting of an amino acid sequence selected from the group consisting of SEQ ID NOs: 1, 3, 4, 5, 8, 11, 12, 13, 14, 16, 17, 18, 19, 20, 22, 23, 24, 25, 27, 30, 32, 34, 43, 45, 51, 54, 55, 65, 68, 71, 73, 74, 75. The kit may comprise one or more glycopeptides consisting essentially of an amino acid sequence selected from the group consisting of SEQ ID NOs: 1, 3, 4, 5, 8, 11, 12, 13, 14, 16, 17, 18, 19, 20, 22, 23, 24, 25, 27, 30, 32, 34, 43, 45, 51, 54, 55, 65, 68, 71, 73, 74, 75. The kit may comprise one or more glycopeptides consisting of an amino acid sequence selected from the group consisting of SEQ ID NOs: 2, 6, 7, 9, 10, 15, 21, 26, 28, 29, 31, 33, 35, 36, 37, 38, 39, 40, 41, 42, 44, 46, 47, 48, 49, 50, 52, 53, 56, 57, 58, 59, 60, 61, 62, 63, 64, 66, 67, 69, 70, 72, 76, and combinations thereof. The kit may comprise one or more glycopeptides consisting essentially of an amino acid sequence selected from the group consisting of SEQ ID NOs: 2, 6, 7, 9, 10, 15, 21, 26, 28, 29, 31, 33, 35, 36, 37, 38, 39, 40, 41, 42, 44, 46, 47, 48, 49, 50, 52, 53, 56, 57, 58, 59, 60, 61, 62, 63, 64, 66, 67, 69, 70, 72, 76, and combinations thereof.

Set forth herein is a kit comprising the reagents for quantification of the oxidised, nitrated, and/or glycated free adducts derived from glycopeptides.

### VII. Clinical Assays

In some examples, including any of the foregoing, the methods may be used in a clinical setting for diagnosing patients. In some of these examples, the analysis of samples includes the use of internal standards. These standards may include one or more glycopeptides consisting of an amino acid sequence selected from the group consisting of SEQ ID NOs: 1 - 76. These standards may include one or more glycopeptides consisting essentially of an amino acid sequence selected from the group consisting of SEQ ID NOs: 1 - 76. In certain examples, these standards may include one or more glycopeptides consisting of an amino acid sequence selected from the group consisting of SEQ ID NOs: 1, 6, 7, 8, 10, 15, 21, 22, 23, 42, 47, 48, 51, 56, 57, 58, 62, 74, and 76, and combinations thereof. In certain examples, these standards may include one or more glycopeptides consisting essentially of an amino acid sequence selected from the group consisting of SEQ ID NOs: 1, 6, 7, 8, 10, 15, 21, 22, 23, 42, 47, 48, 51, 56, 57, 58, 62, 74, and 76, and combinations thereof. In certain examples, these standards may include one or more glycopeptides consisting of an amino acid sequence selected from the group consisting of SEQ ID NOs: 1, 3, 4, 5, 8, 11, 12, 13, 14, 16, 17, 18, 19, 20, 22, 23, 24, 25, 27, 30, 32, 34, 43, 45, 51, 54, 55, 65, 68, 71, 73, 74, 75, and combinations thereof. In certain examples, these standards may include one or more glycopeptides consisting essentially of an amino acid sequence selected from the group consisting of SEQ ID NOs: 1, 3, 4, 5, 8, 11, 12, 13, 14, 16, 17, 18, 19, 20, 22, 23, 24, 25, 27, 30, 32, 34, 43, 45, 51, 54, 55, 65, 68, 71, 73, 74, 75, and combinations thereof. In certain examples, these standards may include one or more glycopeptides consisting of an amino acid sequence selected from the group consisting of SEQ ID NOs: 2, 6, 7, 9, 10, 15, 21, 26, 28, 29, 31, 33, 35, 36, 37, 38, 39, 40, 41, 42, 44, 46, 47, 48, 49, 50, 52, 53, 56, 57, 58, 59, 60, 61, 62, 63, 64, 66, 67, 69, 70, 72, 76, and combinations thereof. In certain examples, these standards may include one or more glycopeptides consisting essentially of an amino acid sequence selected from the group consisting of SEQ ID NOs: 2, 6, 7, 9, 10, 15, 21, 26, 28, 29, 31, 33, 35, 36, 37, 38, 39, 40, 41, 42, 44, 46, 47, 48, 49, 50, 52, 53, 56, 57, 58, 59, 60, 61, 62, 63, 64, 66, 67, 69, 70, 72, 76, and combinations thereof.

In a clinical setting, samples may be prepared *(e.g.,* by digestion) to include one or more glycopeptides consisting of an amino acid sequence selected from the group consisting of SEQ ID NOs: 1 - 76. In certain examples, these samples may include one or more glycopeptides consisting of an amino acid sequence selected from the group consisting of SEQ ID NOs: 1, 6, 7, 8, 10, 15, 21, 22, 23, 42, 47, 48, 51, 56, 57, 58, 62, 74, and 76, and combinations thereof. In certain examples, these samples may include one or more glycopeptides consisting essentially of an amino acid sequence selected from the group consisting of SEQ ID NOs: 1, 3, 4, 5, 8, 11, 12, 13, 14, 16, 17, 18, 19, 20, 22, 23, 24, 25, 27, 30, 32, 34, 43, 45, 51, 54, 55, 65, 68, 71, 73, 74, 75, and combinations thereof. In certain examples, these samples may include one or more glycopeptides consisting essentially of an amino acid sequence selected from the group consisting of SEQ ID NOs: 2, 6, 7, 9, 10, 15, 21, 26, 28, 29, 31, 33, 35, 36, 37, 38, 39, 40, 41, 42, 44, 46, 47, 48, 49, 50, 52, 53, 56, 57, 58, 59, 60, 61, 62, 63, 64, 66, 67, 69, 70, 72, 76, and combinations thereof.

In a clinical setting, samples may be prepared (*e.g.,* by digestion) to include one or more glycopeptides consisting essentially of an amino acid sequence selected from the group consisting of SEQ ID NOs: 1 - 76. In certain examples, the samples may be prepared (*e.g.,* by digestion) to include one or more glycopeptides consisting essentially of an amino acid sequence selected from the group consisting of SEQ ID NOs: 1, 6, 7, 8, 10, 15, 21, 22, 23, 42, 47, 48, 51, 56, 57, 58, 62, 74, and 76, and combinations thereof. In certain examples, the samples may be prepared (*e.g.,* by digestion) to include one or more glycopeptides consisting essentially of an amino acid sequence selected from the group consisting of SEQ ID NOs: 1, 3, 4, 5, 8, 11, 12, 13, 14, 16, 17, 18, 19, 20, 22, 23, 24, 25, 27, 30, 32, 34, 43, 45, 51, 54, 55, 65, 68, 71, 73, 74, 75, and combinations thereof. In certain examples, the samples may be prepared (*e.g.,* by digestion) to include one or more glycopeptides consisting essentially of an amino acid sequence selected from the group consisting of SEQ ID NOs: 2, 6, 7, 9, 10, 15, 21, 26, 28, 29, 31, 33, 35, 36, 37, 38, 39, 40, 41, 42, 44, 46, 47, 48, 49, 50, 52, 53, 56, 57, 58, 59, 60, 61, 62, 63, 64, 66, 67, 69, 70, 72, 76, and combinations thereof.

In some examples, the amount of a glycan or glycopeptide may be assessed by comparing the amount of one or more glycopeptides consisting of an amino acid sequence selected from the group consisting of SEQ ID NOs: 1 - 76 to the concentration of another biomarker.

In some examples, the amount of a glycan or glycopeptide may be assessed by comparing the amount of one or more glycopeptides consisting essentially of an amino acid sequence selected from the group consisting of SEQ ID NOs: 1 - 76 to the concentration of another biomarker.

In some examples, the amount of a glycan or glycopeptide may be assessed by comparing the amount of one or more glycopeptides consisting of an amino acid sequence selected from the group consisting of SEQ ID NOs: 1 - 76 to the amount of one or more glycopeptides consisting of an amino acid sequence selected from the group consisting of SEQ ID NOs: 1 - 76.

In some examples, the amount of a glycan or glycopeptide may be assessed by comparing the amount of one or more glycopeptides consisting essentially of an amino acid sequence selected from the group consisting of SEQ ID NOs: 1 - 76 to the amount of one or more glycopeptides consisting essentially of an amino acid sequence selected from the group consisting of SEQ ID NOs: 1 - 76.

The kit may include software for computing the normalization of a glycopeptide MRM transition signal.

The kit may include software for quantifying the amount of a glycopeptide consisting of, or consisting essentially of, an amino acid sequence selected from the group consisting of SEQ ID NOs: 1 - 76.

The kit may include software for quantifying the relative amount of a glycopeptide consisting of, or consisting essentially of, an amino acid sequence selected from the group consisting of SEQ ID NOs: 1 - 76.

In some examples, including any of the foregoing, a trained model is stored on a server which is accessed by a clinician performing a method, set forth herein. In some examples, the clinician inputs the quantification of the MRM transition signals from a patient's sample into a trained model which are stored on a server. In some examples, the server is accessed by the internet, wireless communication, or other digital or telecommunication methods.

In some examples, including any of the foregoing, a trained model is stored on a server which is accessed by a clinician performing a method, set forth herein. In some examples, the clinician inputs the quantification of the glycopeptide or glycopeptides consisting of, or consisting essentially of, an amino acid sequence selected from the group consisting of SEQ ID NOs: 1 - 76 from a patient's sample into a trained model which are stored on a server. In some examples, the server is accessed by the internet, wireless communication, or other digital or telecommunication methods.

In some examples, including any of the foregoing, MRM transition signals 1-76 are stored on a server which is accessed by a clinician performing a method, set forth herein. In some examples, the clinician compares the MRM transition signals from a patient's sample to the MRM transition signals 1-76 which are stored on a server. In some examples, the server is accessed by the internet, wireless communication, or other digital or telecommunication methods.

In some examples, including any of the foregoing, a machine learning algorithm, which has been trained using the MRM transition signals 1-76, described herein, is stored on a server which is accessed by a clinician performing a method, set forth herein. In some examples, the machine learning algorithm, accessed remotely on a server, analyzes the MRM transition signals from a patient's sample. In some examples, the server is accessed by the internet, wireless communication, or other digital or telecommunication methods.

### VIII. EXAMPLES

Chemicals and Reagents. Glycoprotein standards purified from human serum/plasma were purchased from Sigma-Aldrich (St. Louis, MO). Sequencing grade trypsin was purchased from Promega (Madison, WI). Dithiothreitol (DTT) and iodoacetamide (IAA) were purchased from Sigma-Aldrich (St. Louis, MO). Human serum was purchased from Sigma-Aldrich (St. Louis, MO).

Sample Preparation. Serum samples and glycoprotein standards were reduced, alkylated and then digested with trypsin in a water bath at 37 °C for 18 hours.

LC-MS/MS Analysis. For quantitative analysis, tryptic digested serum samples were injected into an high performance liquid chromatography (HPLC) system coupled to triple quadrupole (QqQ) mass spectrometer. The separation was conducted on a reverse phase column. Solvents A and B used in the binary gradient were composed of mixtures of water, acetonitrile and formic acid. Typical positive ionization source parameters were utilized after source tuning with vendor supplied standards. The following ranges were evaluated: source spray voltage between 3-5 kV, temperature 250-350 °C, and nitrogen sheath gas flow rate 20-40 psi. The scan mode of instrument used was dMRM.

For the glycoproteomic analysis, enriched serum glycopeptides were analyzed with a Q Exactive^{™} Hybrid Quadrupole-Orbitrap^{™} Mass spectrometer or an Agilent 6495B Triple Quadrupole LC/MS.

MRM Mass Spectroscopy settings, sample preparation, and reagents are set forth in Li, et al., Site-Specific Glycosylation Quantification of 50 serum Glycoproteins Enhanced by Predictive Glycopeptidomics for Improved Disease Biomarker Discovery, Anal. Chem. 2019, 91, 5433-5445; DOI: 10.1021/acs.analchem.9b00776.

### Example 1 - Identifying Glycopeptide Biomarkers

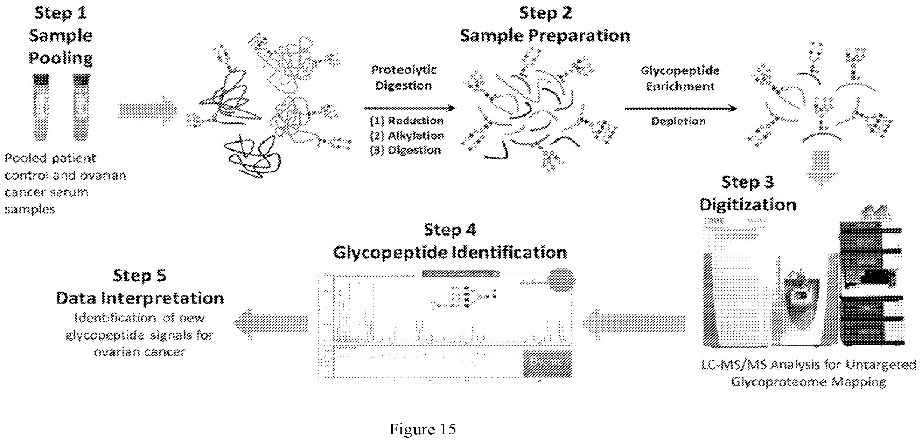

This Example refers to Figures 15 and 17-18.

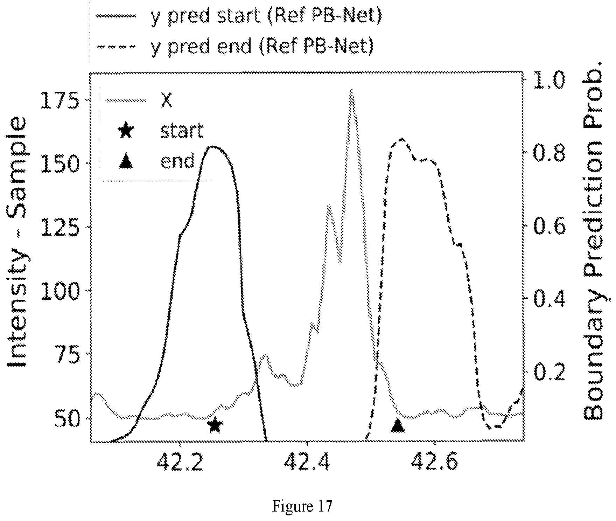

As shown in Figure 15, in step 1, samples from patients having ovarian cancer and samples from patients not having ovarian cancer were provided. In step 2, the samples were digested using protease enzymes to form glycopeptide fragments. In step 3, the glycopeptide fragments were introduced into a tandem LC-MS/MS instrument to analyze the retention time and MRM-MS transition signals associated with the aforementioned samples. In step 4, glycopeptides and glycan biomarkers were identified. Machine learning algorithms selected MRM-MS transition signals from a series of MS spectra and associated those signals with the calculated mass of certain glycopeptide fragments. See Figures 17-18 for retention times analysis for biomarker signals identified by machine learning algorithms.

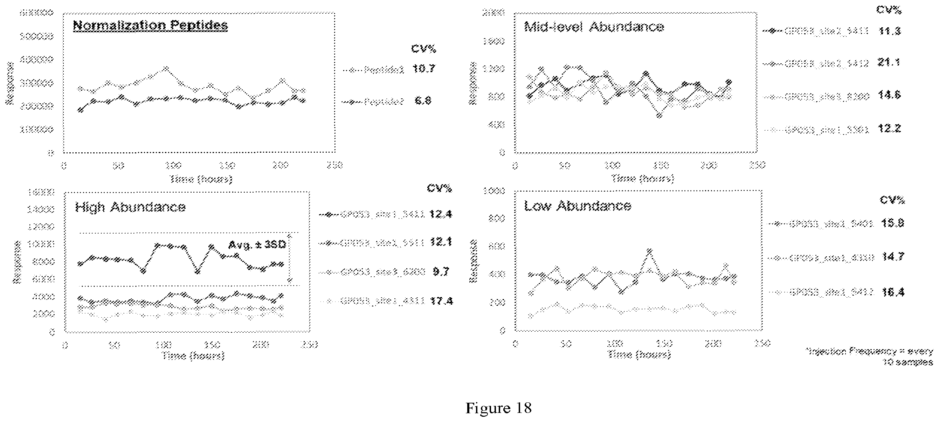

In step 5, the glycopeptides identified in samples from patients having ovarian cancer were compared using machine learning algorithms, including lasso regression, with the glycopeptides identified in samples from patients not having ovarian cancer. This comparison included a comparison of the types, absolute amounts, and relative amounts of glycopeptides. From this comparison, normalization of peptides, and relative abundance of glycopeptides was calculated. See Figure 18 for output results of this comparison.

### Example 2 - Identifying Glycopeptide Biomarkers

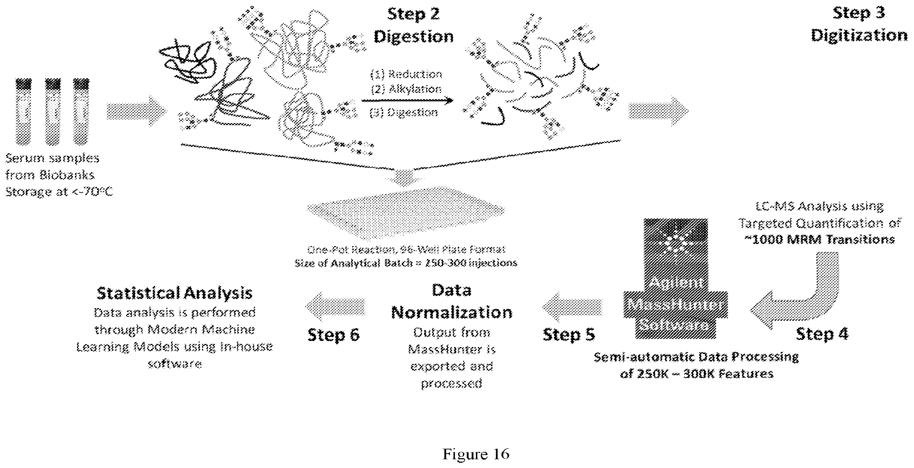

This Example refers to Figure 16.

As shown in Figure 16, in step 1, samples from patients are provided. In step 2, the samples were digested using protease enzymes to form glycopeptide fragments. In step 3, the glycopeptide fragments were introduced into a tandem LC-MS/MS instrument to analyze the retention time and MRM-MS transition signals associated with the sample. In step 4, the glycopeptides were identified using machine learning algorithms which select MRM-MS transition signals and associate those signals with the calculated mass of certain glycopeptide fragments. In step 5, the data is normalized. In step 6, machine learning is used to analyzed the normalized data to identify biomarkers indicative of a patient having ovarian cancer.

### IX. TABLES

**Table 1. Transition Numbers for Glycopeptides from Glycopeptide Groups.**

| **Transition No.** | **Compound Group** | **Compound Name** |
|---|---|---|
| 1 | GP001-P01009\|Alpha-1-antitrypsin\|A1AT | A1AT-GP001_271_5402 |
| 2 | GP001-P01009\|Alpha-1-antitrypsin\|A1AT | A1AT-GP001_271_5412 |
| 3 | GP001-P01009\|Alpha-1-antitrypsin\|A1AT | AlAT-GP001_271MC_5402 |
| 4 | GP002-P04217\|Alpha-1B-glycoprotein\|A1BG | A1BG-GP002_179_5421/5402 |
| 5 | GP004-P01023\|Alpha-2-macroglobulin\|A2MG | A2MG-GP004_1424_5402 |
| 6 | GP004-P01023\|Alpha-2-macroglobulin\|A2MG | A2MG-GP004_1424_5411 |
| 7 | GP004-P01023\|Alpha-2-macroglobulin\|A2MG | A2MG-GP004_55_5401 |
| 8 | GP004-P01023\|Alpha-2-macroglobulin\|A2MG | A2MG-GP004_55_5402 |
| 9 | GP004-P01023\|Alpha-2-macroglobulin\|A2MG | A2MG-GP004_55_5411 |
| 10 | GP004-P01023\|Alpha-2-macroglobulin\|A2MG | A2MG-GP004_869_5200 |
| 11 | GP004-P01023\|Alpha-2-macroglobulin\|A2MG | A2MG-GP004_869_5401 |
| 12 | GP004-P01023\|Alpha-2-macroglobulin\|A2MG | A2MG-GP004_869_6301 |
| 13 | GP006-P43652\|Afamin\|AFAM | AFAM-GP006_33_5402 |
| 14 | GP007&008-P02763&P19652\|Alpha-1-acid glycoprotein 1&2\|AGP12 | AGP12-GP007&008_72MC_6503 |
| 15 | GP007&008-P02763&P19652\|Alpha-1-acid glycoprotein 1&2\|AGP12 | AGP12-GP007&008_72MC_6513 |
| 16 | GP007&008-P02763&P19652\|Alpha-1-acid glycoprotein 1&2\|AGP12 | AGP12-GP007&008_72MC_7601 |
| 17 | GP007&008-P02763&P19652\|Alpha-1-acid glycoprotein 1&2\|AGP12 | AGP12-GP007&008_72MC_7602 |
| 18 | GP007&008-P02763&P19652\|Alpha-1-acid glycoprotein 1&2\|AGP12 | AGP12-GP007&008_72MC_7603 |
| 19 | GP007&008-P02763&P19652\|Alpha-1-acid glycoprotein 1&2\|AGP12 | AGP12-GP007&008_72MC_7613 |
| 20 | GP007-P02763\|Alpha-1-acid glycoprotein 1\|AGP1 | AGP1-GP007_33_5402 |
| 21 | GP007-P02763\|Alpha-1-acid glycoprotein 1\|AGP1 | AGP1-GP007_93_6503/6522 |
| 22 | GP007-P02763\|Alpha-1-acid glycoprotein 1\|AGP1 | AGP1-GP007_93_6513 |
| 23 | GP007-P02763\|Alpha-1-acid glycoprotein 1\|AGP1 | AGP1-GP007_93_7603/7622 |
| 24 | GP007-P02763\|Alpha-1-acid glycoprotein 1\|AGP1 | AGP1-GP007_93_7613 |
| 25 | GP012-P02656\|Apolipoprotein C-III\|APOC3 | APOC3-GP012_74_1102 |
| 26 | GP012-P02656\|Apolipoprotein C-III\|APOC3 | APOC3-GP012_74MC_1102 |
| 27 | GP015-P02749\|Beta-2-glycoprotein \|APOH | APOH-GP015_253_5401 |
| 28 | GP023-P00450\|Ceruloplasmin\|CERU | CERU-GP023_138_5412 |
| 29 | GP023-P00450lCeruloplasminlCERU | CERU-GP023_138_5421/5402 |
| 30 | GP023-P00450\|Ceruloplasmin\|CERU | CERU-GP023_138_6503/6522 |
| 31 | GP024-P08603\|ComplementFactorH\|CFAH | CFAH-GP024_882_5421/5402 |
| 32 | GP028-P01024\|ComplementC3\|CO3 | CO3-GP028_85_5200 |
| 33 | GP028-P01024\|ComplementC3\|CO3 | CO3-GPO28_85_6200 |
| 34 | GP029&030-POCOL4&POCOL5 IComplementC4-A&B\|CO4A&CO4B | CO4A&CO4B-GP029&030_1328_5402 |
| 35 | GP03 6-P02765\|Alpha-2-HS-glycoprotein\|FETUA | FETUA-GP036_156_5402/5421 |
| 36 | GP036-P02765\|Alpha-2-HS-glycoprotein\|FETUA | FETUA-GP036_176_6513 |
| 37 | GP036-PO2765\|Alpha-2-HS-glycoprotein\|FETUA | FETUA-GP036_346_1101 |
| 38 | GP042-P02790\|Hemopexin\|HEMO | BEMO-GPO42_187_5412/5431 |
| 39 | GP042-P02790\|Hemopexin\|HEMO | HEMO-GP042_453_5420/5401 |
| 40 | GP044-P00738\|Haptoglobin\|HPT | HPT-GP044_184_6502 |
| 41 | GP044-P00738\|Haptoglobin\|HPT | HPT-GP044_207_10803 |
| 42 | GP044-P00738\|Haptoglobin\|HPT | HPT-GP044_207 10804 |
| 43 | GP044-P00738\|Haptoglobin\|HPT | HPT-GP044_207_11904 |
| 44 | GP044-P00738\|Haptoglobin\|HPT | HPT-GP044_207_11914 |
| 45 | GP044-P00738\|Haptoglobin\|HPT | HPT-GP044_207_11915 |
| 46 | GP044-P00738\|Haptoglobin\|HPT | HPT-GP044_241_5401/5420 |
| 47 | GP044-P00738\|Haptoglobin\|HPT | HPT-GP044_241_5412 |
| 48 | GP044-P00738\|Haptoglobin\|HPT | HPT-GP044_241_6501 |
| 49 | GP044-P00738\|Haptoglobin\|HPT | HPT-GP044_241_6502 |
| 50 | GP044-P00738\|Haptoglobin\|HPT | HPT-GP044_241_6511 |
| 51 | GP044-P00738\|Haptoglobin\|HPT | HPT-GP044_241_6513 |
| 52 | GP046&047-P01876&P01877\|Immunoglobulin heavy constant alpha 1&2\|IgA12 | IgA12-GP046&047_144_5501 |
| 53 | GP047-P01877\|Immunoglobulin heavy constant alpha 2\|IgA2 | IgA2-GP047_205_4510 |
| 54 | GP047-P01877\|Immunoglobulin heavy constant alpha 2\|IgA2 | IgA2-GP047_205_5412 |
| 55 | GP047-P01877\|Immunoglobulin heavy constant alpha 2\|IgA2 | IgA2-GP047 205_5510 |
| 56 | GP048-P01857\|Immunoglobulin heavy constant gamma 1\|IgG1 | IgG1-GP048_297_3410 |
| 57 | GP048-P01857\|Immunoglobulin heavy constant gamma 1\|IgG1 | IgG1-GP048_297_4400 |
| 58 | GP048-P01857\|Immunoglobulin heavy constant gamma 1\|IgG1 | IgG1-GP048_297_4510 |
| 59 | GP048-P01857\|Immunoglobulin heavy constant gamma 1\|IgG1 | IgG1-GP048_297_5400 |
| 60 | GP048-P01857\|Immunoglobulin heavy constant gamma 1\|IgG1 | IgG1-GP048_297_5410 |
| 61 | GP048-P01857\|Immunoglobulin heavy constant gamma 1\|IgG1 | IgG1-GP048_297_5411 |
| 62 | GP048-P01857\|Immunoglobulin heavy constant gamma 1\|IgG1 | IgG1-GP048_297_5510 |
| 63 | GP048-P01857\|Immunoglobulin heavy constant gamma 1\|IgG1 | IgG1-GP048_297_nonglycosylated |
| 64 | GP049-P01859\|Immunoglobulin heavy constant gamma 2\|IgG2 | IgG2-GP049_297_3510 |
| 65 | GP049-P01859\|Immunoglobulin heavy constant gamma 2\|IgG2 | IgG2-GP049_297_4411 |
| 66 | GP049-P01859\|Immunoglobulin heavy constant gamma 2\|IgG2 | IgG2-GP049_297_4510 |
| 67 | GP052-P01591\|Immunoglobulin J chain\|Ig-J | IgJ-GP052_71_5401 |
| 68 | GP053-P01871\|Immunoglobulin heavy constant mu\|IgM | IgM-GP053_439_6200 |
| 69 | GP053 -P01871 \|Immunoglobulin heavy constant mu\|IgM | IgM-GP053_46_4311 |
| 70 | GP057-P01042\|Kininogen-1\|KNG1 | KNG1-GP057_205_6503 |
| 71 | GP064-P02787\|Serotransferrin\|TRFE | TRFE-GP064_432_5402 |
| 72 | GP064-P02787\|Serotransferrin\|TRFE | TRFE-GP064_630_5401 |
| 73 | GP064-P02787\|Serotransferrin\|TRFE | TRFE-GP064_630_6513 |
| 74 | GP067-P04004\|Vitronectin\|VTNC | VTNC-GP067_169_5401 |
| 75 | GP067-P04004\|Vitronectin\|VTNC | VTNC-GP067_242_6503 |
| 76 | GP067-P04004\|Vitronectin\|VTNC | VTNC-GP067_86_5421 |

**Table 2. Transition Numbers with Precursor Ion and Product Ion (m/z)**

| **Transition No.** | **Precursor Ion** | **Product Ion** |
|---|---|---|
| 1 | 991.2 | 366.1 |
| 2 | 1027.7 | 366.1 |
| 3 | 1149.9 | 366.1 |
| 4 | 1209.5 | 366.1 |
| 5 | 1093.2 | 366.1 |
| 6 | 1057 | 366.1 |
| 7 | 1079 | 366.1 |
| 8 | 1151.7 | 366.1 |
| 9 | 1115.4 | 366.1 |
| 10 | 1158.8 | 1206.9 |
| 11 | 1066.7 | 366.1 |
| 12 | 1322.3 | 366.1 |
| 13 | 1134.1 | 366.1 |
| 14 | 1152.7 | 366.1 |
| 15 | 1181.9 | 366.1 |
| 16 | 1109.1 | 366.1 |
| 17 | 1167.3 | 366.1 |
| 18 | 1225.5 | 366.1 |
| 19 | 1254.7 | 366.1 |
| 20 | 1196.5 | 366.1 |
| 21 | 1195.3 | 366.1 |
| 22 | 1231.8 | 274.1 |
| 23 | 1286.6 | 366.1 |
| 24 | 1323.1 | 366.1 |
| 25 | 1028.8 | 274.1 |
| 26 | 1109.8 | 274.1 |
| 27 | 1055.8 | 366.1 |
| 28 | 1062.2 | 366.1 |
| 29 | 1367.2 | 366.1 |
| 30 | 1189.5 | 366.1 |
| 31 | 1057.7 | 366.1 |
| 32 | 1157.9 | 204.1 |
| 33 | 1211.9 | 366.1 |
| 34 | 1103.8 | 366.1 |
| 35 | 995.4 | 366.1 |
| 36 | 1343.8 | 366.1 |
| 37 | 891.8 | 274.1 |
| 38 | 1253.2 | 366.1 |
| 39 | 1217.9 | 366.1 |
| 40 | 1051 | 366.1 |
| 41 | 1116.4 | 366.1 |
| 42 | 1174.6 | 366.1 |
| 43 | 1247.7 | 366.1 |
| 44 | 1276.9 | 366.1 |
| 45 | 1335.1 | 366.1 |
| 46 | 1237.3 | 366.1 |
| 47 | 1383 | 366.1 |
| 48 | 1019.5 | 366.1 |
| 49 | 1092.3 | 366.1 |
| 50 | 1055.7 | 366.1 |
| 51 | 1201.5 | 366.1 |
| 52 | 1017.3 | 366.1 |
| 53 | 923.5 | 366.1 |
| 54 | 1103.8 | 366.1 |
| 55 | 977.8 | 366.1 |
| 56 | 879 | 204.1 |
| 57 | 884.4 | 204.1 |
| 58 | 1000.7 | 204.1 |
| 59 | 938.4 | 366.1 |
| 60 | 987.1 | 366.1 |
| 61 | 1084.1 | 366.1 |
| 62 | 1054.7 | 366.1 |
| 63 | 595.3 | 640.3 |
| 64 | 935.8 | 204.1 |
| 65 | 1019.4 | 204.1 |
| 66 | 989.9 | 204.1 |
| 67 | 1048.1 | 366.1 |
| 68 | 1248.5 | 1284.7 |
| 69 | 993.8 | 366.1 |
| 70 | 1069.9 | 366.1 |
| 71 | 921.4 | 366.1 |
| 72 | 1108.4 | 366.1 |
| 73 | 1105.6 | 366.1 |
| 74 | 942.4 | 366.1 |
| 75 | 1409.1 | 366.1 |
| 76 | 1148 | 366.1 |

### MS1 and MS2 resolution was 1 unit.

**Table 3. Transition Numbers with Retention Time, ΔRetention Time, Fragmentor and Collision Energy**

| **Transition No.** | **Ret Time (min)** | **Delta Ret Time** | **Fragmentor** | **Collision Energy** |
|---|---|---|---|---|
| 1 | 29.8 | 3 | 380 | 24 |
| 2 | 29.8 | 3 | 380 | 25 |
| 3 | 42.5 | 4 | 380 | 28 |
| 4 | 24 | 2 | 380 | 36 |
| 5 | 43.7 | 3 | 380 | 22 |
| 6 | 43.1 | 3 | 380 | 22 |
| 7 | 39.8 | 3 | 380 | 22 |
| 8 | 41.1 | 3 | 380 | 23 |
| 9 | 39.3 | 3 | 380 | 22 |
| 10 | 25.3 | 2 | 380 | 23 |
| 11 | 26 | 2 | 380 | 22 |
| 12 | 26.2 | 2 | 380 | 24 |
| 13 | 9.3 | 1 | 380 | 30 |
| 14 | 38.2 | 3 | 380 | 28 |
| 15 | 38.2 | 3 | 380 | 29 |
| 16 | 37.3 | 3 | 380 | 30 |
| 17 | 36.5 | 3 | 380 | 29 |
| 18 | 38 | 3 | 380 | 31 |
| 19 | 37.8 | 3 | 380 | 31 |
| 20 | 29 | 2 | 380 | 30 |
| 21 | 17.6 | 1.2 | 380 | 30 |
| 22 | 17.6 | 1.2 | 380 | 31 |
| 23 | 17.5 | 1.2 | 380 | 32 |
| 24 | 17.5 | 1.2 | 380 | 33 |
| 25 | 30.2 | 2 | 380 | 25 |
| 26 | 29.3 | 2 | 380 | 27 |
| 27 | 14.4 | 2 | 380 | 33 |
| 28 | 13 | 2 | 380 | 33 |
| 29 | 13.2 | 2 | 380 | 40 |
| 30 | 13.5 | 2 | 380 | 36 |
| 31 | 12.4 | 1.2 | 380 | 33 |
| 32 | 19.6 | 1.2 | 380 | 30 |
| 33 | 19.5 | 1.2 | 380 | 30 |
| 34 | 13.6 | 1.2 | 380 | 34 |
| 35 | 20.1 | 1.2 | 380 | 24 |
| 36 | 22 | 1.2 | 380 | 34 |
| 37 | 17.1 | 2 | 380 | 21 |
| 38 | 16.2 | 1.2 | 380 | 37 |
| 39 | 21.4 | 1.2 | 380 | 37 |
| 40 | 24.2 | 1.4 | 380 | 26 |
| 41 | 10.8 | 1.2 | 380 | 27 |
| 42 | 11 | 1.2 | 380 | 29 |
| 43 | 10.7 | 1.2 | 380 | 31 |
| 44 | 10.9 | 1.4 | 380 | 32 |
| 45 | 10.9 | 1.4 | 380 | 34 |
| 46 | 20.7 | 1.4 | 380 | 31 |
| 47 | 21.6 | 1.4 | 380 | 35 |
| 48 | 20.6 | 1.2 | 380 | 25 |
| 49 | 21.5 | 1.3 | 380 | 27 |
| 50 | 20.6 | 1.2 | 380 | 30 |
| 51 | 22.2 | 1.2 | 380 | 30 |
| 52 | 38.1 | 2 | 380 | 25 |
| 53 | 10.2 | 1.2 | 380 | 22 |
| 54 | 11.3 | 1.2 | 380 | 27 |
| 55 | 10.2 | 1.2 | 380 | 24 |
| 56 | 5.6 | 1 | 380 | 21 |
| 57 | 5.6 | 1 | 380 | 21 |
| 58 | 5.8 | 1.2 | 380 | 24 |
| 59 | 5.5 | 1 | 380 | 22 |
| 60 | 5.5 | 1 | 380 | 24 |
| 61 | 6 | 1 | 380 | 27 |
| 62 | 5.7 | 1 | 380 | 26 |
| 63 | 15.6 | 1.2 | 380 | 13 |
| 64 | 10.7 | 1.2 | 380 | 22 |
| 65 | 11.3 | 1 | 380 | 25 |
| 66 | 10.6 | 1 | 380 | 24 |
| 67 | 12.4 | 1 | 380 | 26 |
| 68 | 22.2 | 1.2 | 380 | 25 |
| 69 | 4.3 | 1.2 | 380 | 24 |
| 70 | 13.3 | 2 | 380 | 33 |
| 71 | 19.7 | 1.2 | 380 | 22 |
| 72 | 22.5 | 1.2 | 380 | 27 |
| 73 | 24.2 | 1.2 | 380 | 27 |
| 74 | 18.4 | 1.2 | 380 | 30 |
| 75 | 29.3 | 3 | 380 | 41 |
| 76 | 14.5 | 2 | 380 | 35 |

### Cell accelerator voltage was 5.

**Table 4. Glycan Residue Compound Numbers, Molecular Mass, and Glycan Fragment mass-to-charge (m/z) (+2) & (m/z) (+3) ratios**

| **Composition** | **mass** | **m/z (+2)** | **m/z (+3)** |
|---|---|---|---|
| 3200 | 910.327 | 456.1708 | 304.449633 |
| 3210 | 1056.386 | 529.2003 | 353.135967 |
| 3300 | 1113.407 | 557.7108 | 372.142967 |
| 3310 | 1259.465 | 630.7398 | 420.828967 |
| 3320 | 1405.523 | 703.7688 | 469.514967 |
| 3400 | 1316.487 | 659.2508 | 439.8363 |
| 3410 | 1462.544 | 732.2793 | 488.521967 |
| 3420 | 1608.602 | 805.3083 | 537.207967 |
| 3500 | 1519.566 | 760.7903 | 507.5293 |
| 3510 | 1665.624 | 833.8193 | 556.2153 |
| 3520 | 1811.682 | 906.8483 | 604.9013 |
| 3600 | 1722.645 | 862.3298 | 575.2223 |
| 3610 | 1868.703 | 935.3588 | 623.9083 |
| 3620 | 2014.761 | 1008.3878 | 672.5943 |
| 3630 | 2160.89 | 1081.4523 | 721.303967 |
| 3700 | 1925.724642 | 963.869621 | 642.915514 |
| 3710 | 2071.782551 | 1036.898576 | 691.601484 |
| 3720 | 2217.84046 | 1109.92753 | 740.287453 |
| 3730 | 2363.898369 | 1182.956485 | 788.973423 |
| 3740 | 2509.956277 | 1255.985439 | 837.659392 |
| 4200 | 1072.380603 | 537.1976015 | 358.467501 |
| 4210 | 1218.438512 | 610.226556 | 407.153471 |
| 4300 | 1275.459976 | 638.737288 | 426.160625 |
| 4301 | 1566.555392 | 784.284996 | 523.192431 |
| 4310 | 1421.517884 | 711.766242 | 474.846595 |
| 4311 | 1712.613301 | 857.3139505 | 571.8784 |
| 4320 | 1567.575793 | 784.7951965 | 523.532564 |
| 4400 | 1478.539348 | 740.276974 | 493.853749 |
| 4401 | 1769.634765 | 885.8246825 | 590.885555 |
| 4410 | 1624.597257 | 813.3059285 | 542.539719 |
| 4411 | 1915.692673 | 958.8536365 | 639.571524 |
| 4420 | 1770.655166 | 886.334883 | 591.225689 |
| 4421 | 2061.750582 | 1031.882591 | 688.257494 |
| 4430 | 1916.713074 | 959.363837 | 639.911658 |
| 4431 | 2207.808491 | 1104.911546 | 736.943464 |
| 4500 | 1681.618721 | 841.8166605 | 561.546874 |
| 4501 | | 1.0073 | 1.0073 |
| 4510 | 1972.714137 | 987.3643685 | 658.578679 |
| 4511 | 2118.772046 | 1060.393323 | 707.264649 |
| 4520 | 1973.734538 | 987.874569 | 658.918813 |
| 4521 | 2264.829955 | 1133.422278 | 755.950618 |
| 4530 | 2119.792447 | 1060.903524 | 707.604782 |
| 4531 | 2410.887864 | 1206.451232 | 804.636588 |
| 4540 | 2265.850356 | 1133.932478 | 756.290752 |
| 4541 | 2556.945772 | 1279.480186 | 853.322557 |
| 4600 | 1884.698093 | 943.3563465 | 629.239998 |
| 4601 | 2175.79351 | 1088.904055 | 726.271803 |
| 4610 | 2030.756002 | 1016.385301 | 677.925967 |
| 4611 | 2321.851418 | 1161.933009 | 774.957773 |
| 4620 | 2176.813911 | 1089.414256 | 726.611937 |
| 4621 | 2467.909327 | 1234.961964 | 823.643742 |
| 4630 | 2322.87182 | 1162.44321 | 775.297907 |
| 4631 | 2613.967236 | 1307.990918 | 872.329712 |
| 4641 | 2760.025145 | 1381.019873 | 921.015682 |
| 4650 | 2614.987637 | 1308.501119 | 872.669846 |
| 4700 | 2087.777466 | 1044.896033 | 696.933122 |
| 4701 | 2378.872882 | 1190.443741 | 793.964927 |
| 4710 | 2233.835374 | 1117.924987 | 745.619091 |
| 4711 | 2524.930791 | 1263.472696 | 842.650897 |
| 4720 | 2379.893283 | 1190.953942 | 794.305061 |
| 4730 | 2525.951192 | 1263.982896 | 842.991031 |
| 5200 | 1234.433426 | 618.224013 | 412.485109 |
| 5210 | 1380.491335 | 691.2529675 | 461.171078 |
| 5300 | 1437.512799 | 719.7636995 | 480.178233 |
| 5301 | 1728.608215 | 865.3114075 | 577.210038 |
| 5310 | 1583.570708 | 792.792654 | 528.864203 |
| 5311 | 1874.666124 | 938.340362 | 625.896008 |
| 5320 | 1729.628617 | 865.8216085 | 577.550172 |
| 5400 | 1640.592171 | 821.3033855 | 547.871357 |
| 5401 | 1931.687588 | 966.851094 | 644.903163 |
| 5402 | 2222.783005 | 1112.398803 | 741.934968 |
| 5410 | 1786.65008 | 894.33234 | 596.557327 |
| 5411 | 2077.745497 | 1039.880049 | 693.589132 |
| 5412 | 2368.840913 | 1185.427757 | 790.620938 |
| 5420 | 1932.707989 | 967.3612945 | 645.243296 |
| 5421 | 2223.803406 | 1112.909003 | 742.275102 |
| 5430 | 2078.765898 | 1040.390249 | 693.929266 |
| 5431 | 2369.861314 | 1185.937957 | 790.961071 |
| 5432 | 2660.956731 | 1331.485666 | 887.992877 |
| 5500 | 1843.671544 | 922.843072 | 615.564481 |
| 5501 | 2134.766961 | 1068.390781 | 712.596287 |
| 5502 | 2425.862377 | 1213.938489 | 809.628092 |
| 5510 | 1989.729453 | 995.8720265 | 664.250451 |
| 5511 | 2280.824869 | 1141.419735 | 761.282256 |
| 5512 | 2571.920286 | 1286.967443 | 858.314062 |
| 5520 | 2135.787362 | 1068.900981 | 712.936421 |
| 5521 | 2426.882778 | 1214.448689 | 809.968226 |
| 5522 | 2717.978195 | 1359.996398 | 907.000032 |
| 5530 | 2281.84527 | 1141.929935 | 761.62239 |
| 5531 | 2572.940687 | 1287.477644 | 858.654196 |
| 5541 | 2718.998596 | 1360.506598 | 907.340165 |
| 5600 | 2046.750917 | 1024.382759 | 683.257606 |
| 5601 | 2337.846333 | 1169.930467 | 780.289411 |
| 5602 | 2628.94175 | 1315.478175 | 877.321217 |
| 5610 | 2192.808825 | 1097.411713 | 731.943575 |
| 5611 | 2483.904242 | 1242.959421 | 828.975381 |
| 5612 | 2774.999658 | 1388.507129 | 926.007186 |
| 5620 | 2338.866734 | 1170.440667 | 780.629545 |
| 5621 | 2629.962151 | 1315.988376 | 877.66135 |
| 5631 | 2776.020059 | 1389.01733 | 926.34732 |
| 5650 | 2777.040461 | 1389.527531 | 926.687454 |
| 5700 | 2249.830289 | 1125.922445 | 750.95073 |
| 5701 | 2540.925706 | 1271.470153 | 847.982535 |
| 5702 | 2832.021122 | 1417.017861 | 945.014341 |
| 5710 | 2395.888198 | 1198.951399 | 799.636699 |
| 5711 | 2686.983614 | 1344.499107 | 896.668505 |
| 5712 | 2978.079031 | 1490.046816 | 993.70031 |
| 5720 | 2541.946107 | 1271.980354 | 848.322669 |
| 5721 | 2833.041523 | 1417.528062 | 945.354474 |
| 5730 | 2688.004016 | 1345.009308 | 897.008639 |
| 5731 | 2979.099432 | 1490.557016 | 994.040444 |
| 6200 | 1396.48625 | 699.250425 | 466.502717 |
| 6210 | 1542.544159 | 772.2793795 | 515.188686 |
| 6300 | 1599.565622 | 800.790111 | 534.195841 |
| 6301 | 1890.661039 | 946.3378195 | 631.227646 |
| 6310 | 1745.623531 | 873.8190655 | 582.88181 |
| 6311 | 2036.718948 | 1019.366774 | 679.913616 |
| 6320 | 1891.68144 | 946.84802 | 631.56778 |
| 6400 | 1802.644995 | 902.3297975 | 601.888965 |
| 6401 | 2093.740411 | 1047.877506 | 698.92077 |
| 6402 | 2384.835828 | 1193.425214 | 795.952576 |
| 6410 | 1948.702904 | 975.358752 | 650.574935 |
| 6411 | 2239.79832 | 1120.90646 | 747.60674 |
| 6412 | 2530.893737 | 1266.454169 | 844.638546 |
| 6420 | 2094.760813 | 1048.387707 | 699.260904 |
| 6421 | 2385.856229 | 1193.935415 | 796.29271 |
| 6432 | 2823.009554 | 1412.512077 | 942.010485 |
| 6500 | 2005.724367 | 1003.869484 | 669.582089 |
| 6501 | 2296.819784 | 1149.417192 | 766.613895 |
| 6502 | 2587.9152 | 1294.9649 | 863.6457 |
| 6503 | 2879.010617 | 1440.512609 | 960.677506 |
| 6510 | 2151.782276 | 1076.898438 | 718.268059 |
| 6511 | 2442.877693 | 1222.446147 | 815.299864 |
| 6512 | 2733.973109 | 1367.993855 | 912.33167 |
| 6513 | 3025.068526 | 1513.541563 | 1009.36348 |
| 6520 | 2297.840185 | 1149.927393 | 766.954028 |
| 6521 | 2588.935602 | 1295.475101 | 863.985834 |
| 6522 | 2880.031018 | 1441.022809 | 961.017639 |
| 6530 | 2443.898094 | 1222.956347 | 815.639998 |
| 6531 | 2734.99351 | 1368.504055 | 912.671803 |
| 6532 | 3026.088927 | 1514.051764 | 1009.70361 |
| 6540 | 2589.956003 | 1295.985302 | 864.325968 |
| 6541 | 2881.051419 | 1441.53301 | 961.357773 |
| 6600 | 2208.80374 | 1105.40917 | 737.275213 |
| 6601 | 2499.899157 | 1250.956879 | 834.307019 |
| 6602 | 2790.994573 | 1396.504587 | 931.338824 |
| 6603 | 3082.08999 | 1542.052295 | 1028.37063 |
| 6610 | 2354.861649 | 1178.438125 | 785.961183 |
| 6611 | 2645.957065 | 1323.985833 | 882.992988 |
| 6612 | 2937.052482 | 1469.533541 | 980.024794 |
| 6613 | 3228.147898 | 1615.081249 | 1077.0566 |
| 6620 | 2500.919558 | 1251.467079 | 834.647153 |
| 6621 | 2792.014974 | 1397.014787 | 931.678958 |
| 6622 | 3083.110391 | 1542.562496 | 1028.71076 |
| 6623 | 3374.205807 | 1688.110204 | 1125.74257 |
| 6630 | 2646.977466 | 1324.496033 | 883.333122 |
| 6631 | 2938.072883 | 1470.043742 | 980.364928 |
| 6632 | 3229.168299 | 1615.59145 | 1077.39673 |
| 6640 | 2793.035375 | 1397.524988 | 932.019092 |
| 6641 | 3084.130792 | 1543.072696 | 1029.0509 |
| 6642 | 3375.226208 | 1688.620404 | 1126.0827 |
| 6652 | 3521.284117 | 1761.649359 | 1174.76867 |
| 6700 | 2411.883113 | 1206.948857 | 804.968338 |
| 6701 | 2702.978529 | 1352.496565 | 902.000143 |
| 6703 | 3285.169362 | 1643.591981 | 1096.06375 |
| 6710 | 2557.941021 | 1279.977811 | 853.654307 |
| 6711 | 2849.036438 | 1425.525519 | 950.686113 |
| 6711 | 2849.036438 | 1425.525519 | 950.686113 |
| 6712 | 3140.131854 | 1571.073227 | 1047.71792 |
| 6713 | 3431.227271 | 1716.620936 | 1144.74972 |
| 6713 | 3431.227271 | 1716.620936 | 1144.74972 |
| 6720 | 2703.99893 | 1353.006765 | 902.340277 |
| 6721 | 2995.094347 | 1498.554474 | 999.372082 |
| 6721 | 2995.094347 | 1498.554474 | 999.372082 |
| 6730 | 2850.056839 | 1426.03572 | 951.026246 |
| 6731 | 3141.152255 | 1571.583428 | 1048.05805 |
| 6740 | 2996.114748 | 1499.064674 | 999.712216 |
| 7200 | 1558.539073 | 780.2768365 | 520.520324 |
| 7210 | 1704.596982 | 853.305791 | 569.206294 |
| 7400 | 1964.697818 | 983.356209 | 655.906573 |
| 7401 | 2255.793235 | 1128.903918 | 752.938378 |
| 7410 | 2110.755727 | 1056.385164 | 704.592542 |
| 7411 | 2401.851144 | 1201.932872 | 801.624348 |
| 7412 | 2692.94656 | 1347.48058 | 898.656153 |
| 7420 | 2256.813636 | 1129.414118 | 753.278512 |
| 7421 | 2547.909052 | 1274.961826 | 850.310317 |
| 7430 | 2402.871545 | 1202.443073 | 801.964482 |
| 7431 | 2693.966961 | 1347.990781 | 898.996287 |
| 7432 | 2985.062378 | 1493.538489 | 996.028093 |
| 7500 | 2167.777191 | 1084.895896 | 723.599697 |
| 7501 | 2458.872607 | 1230.443604 | 820.631502 |
| 7510 | 2313.8351 | 1157.92485 | 772.285667 |
| 7511 | 2604.930516 | 1303.472558 | 869.317472 |
| 7512 | 2896.025933 | 1449.020267 | 966.349278 |
| 7600 | 2370.856563 | 1186.435582 | 791.292821 |
| 7601 | 2661.95198 | 1331.98329 | 888.324627 |
| 7602 | 2953.047396 | 1477.530998 | 985.356432 |
| 7603 | 3244.142813 | 1623.078707 | 1082.38824 |
| 7604 | 3535.23823 | 1768.626415 | 1179.42004 |
| 7610 | 2516.914472 | 1259.464536 | 839.978791 |
| 7611 | 2808.009889 | 1405.012245 | 937.010596 |
| 7612 | 3099.105305 | 1550.559953 | 1034.0424 |
| 7613 | 3390.200722 | 1696.107661 | 1131.07421 |
| 7614 | 3681.296138 | 1841.655369 | 1228.10601 |
| 7620 | 2662.972381 | 1332.493491 | 888.66476 |
| 7621 | 2954.067798 | 1478.041199 | 985.696566 |
| 7622 | 3245.163214 | 1623.588907 | 1082.72837 |
| 7623 | 3536.258631 | 1769.136616 | 1179.76018 |
| 7632 | 3391.221123 | 1696.617862 | 1131.41434 |
| 7640 | 2955.088199 | 1478.5514 | 986.0367 |
| 7700 | 2573.935936 | 1287.975268 | 858.985945 |
| 7701 | 2865.031352 | 1433.522976 | 956.017751 |
| 7702 | 3156.126769 | 1579.070685 | 1053.04956 |
| 7703 | 3447.222186 | 1724.618393 | 1150.08136 |
| 7710 | 2719.993845 | 1361.004223 | 907.671915 |
| 7711 | 3011.089261 | 1506.551931 | 1004.70372 |
| 7712 | 3302.184678 | 1652.099639 | 1101.73553 |
| 7713 | 3593.280094 | 1797.647347 | 1198.76733 |
| 7714 | 3884.375511 | 1943.195056 | 1295.79914 |
| 7720 | 2866.051754 | 1434.033177 | 956.357885 |
| 7721 | 3157.14717 | 1579.580885 | 1053.38969 |
| 7722 | 3448.242587 | 1725.128594 | 1150.4215 |
| 7730 | 3012.109662 | 1507.062131 | 1005.04385 |
| 7731 | 3303.205079 | 1652.60984 | 1102.07566 |
| 7732 | 3594.300495 | 1798.157548 | 1199.10747 |
| 7740 | 3158.167571 | 1580.091086 | 1053.72982 |
| 7741 | 3449.262988 | 1725.638794 | 1150.76163 |
| 7751 | 3595.320897 | 1798.667749 | 1199.4476 |
| 8200 | 1720.591897 | 861.3032485 | 574.537932 |
| 9200 | 1882.64472 | 942.32966 | 628.55554 |
| 9210 | 2028.702629 | 1015.358615 | 677.24151 |
| 10200 | 2044.697544 | 1023.356072 | 682.573148 |
| 11200 | 2206.750367 | 1104.382484 | 736.590756 |
| 12200 | 2368.80319 | 1185.408895 | 790.608363 |

**Table 5. Glycan Residue Compound Numbers, Molecular Mass, and Classification**

| **Compound** | **Glycan Mass** | **Glycan Composition** | **Class** |
|---|---|---|---|
| 3200 | 910.328 | GlcNAc₂Man₃ | HM |
| 3200 | | | |
| 3210 | 1056.386 | GlcNAc₂Man₃Fuc₁ | HM-F |
| 3210 | | | |
| 3300 | 1113.407 | Hex₃HexNAc₃ | C |
| 3300 | | | |
| 3310 | 1259.465 | Hex₃HexNAc₃Fuc₁ | C-F |
| 3310 | | | |
| 3320 | 1405.523 | Hex₃HexNAc₃Fuc₂ | C-F |
| 3400 | 1316.487 | Hex₃HexNAc₄ | C |
| 3410 | 1462.544 | Hex₃HexNAc₄Fuc₁ | C-F |
| 3410 | | | |
| 3420 | 1608.602 | Hex₃HexNAc₄Fuc₂ | C-F |
| 3500 | 1519.566 | Hex₃HexNAc₅ | C |
| 3510 | 1665.624 | Hex₃HexNAc₅Fuc₁ | C-F |
| 3520 | 1811.682 | Hex₃HexNAc₅Fuc₂ | C-F |
| 3600 | 1722.645 | Hex₃HexNAc₆ | C |
| 3610 | 1868.703 | Hex₃HexNAc₆Fuc₁ | C-F |
| 3620 | 2014.761 | Hex₃HexNAc₆Fuc₂ | C-F |
| 3630 | 2160.819 | Hex₃HexNAc₆Fuc₃ | C-F |
| 3700 | 1925.725 | Hex₃HexNAc₇ | C |
| 3710 | 2071.783 | Hex₃HexNAc₇Fuc₁ | C-F |
| 3720 | 2217.841 | Hex₃HexNAc₇Fuc₂ | C-F |
| 3720 | 2217.841 | Hex₃HexNAc₇Fuc₂ | C-F |
| 3730 | 2363.898 | Hex₃HexNAc₇Fuc₃ | C-F |
| 3740 | 2509.956 | Hex₃HexNAc₇Fuc₄ | C-F |
| 4200 | 1072.381 | GlcNAc₂Man₄ | HM |
| 4200 | | | |
| 4210 | 1218.438 | GlcNAc₂Man₄Fuc₁ | HM-F |
| 4210 | | | |
| 4300 | 1275.460 | Hex₄HexNAc₃ | C/H |
| 4300 | | | |
| 4301 | 1566.555 | Hex₄HexNAc₃Neu5Ac₁ | C-S |
| 4301 | 1566.555 | Hex₄HexNAc₃Neu5Ac₁ | C-S |
| 4301 | | | |
| 4310 | 1421.518 | Hex₄HexNAc₃Fuc₁ | C/H-F |
| 4310 | 1566.555 | Hex₄HexNAc₃Neu5Ac₁ | C-S |
| 4310 | | | |
| 4311 | 1712.613 | Hex₄HexNAc₃Fuc₁Neu5Ac₁ | C-FS |
| 4311 | | | |
| 4320 | | | |
| 4400 | 1478.539 | Hex₄HexNAc₄ | C/H |
| 4400 | | | |
| 4401 | 1769.635 | Hex₄HexNAc₄Neu5Ac₁ | C-S |
| 4410 | 1624.597 | Hex₄HexNAc₄Fuc₁ | C/H-F |
| 4410 | | | |
| 4411 | 1915.693 | Hex₄HexNAc₄Fuc₁Neu5Ac₁ | C-FS |
| 4411 | | | |
| 4420 | 1770.655 | Hex₄HexNAc₄Fuc₂ | C/H-F |
| 4420 | | | |
| 4421 | 2061.751 | Hex₄HexNAc₄Fuc₂Neu5Ac₁ | C-FS |
| 4430 | 1916.713 | Hex₄HexNAc₄Fuc₃ | C/H-F |
| 4431 | 2207.808 | Hex₄HexNAc₄Fuc₃Neu5Ac₁ | C-FS |
| 4431 | 2207.808 | Hex₄HexNAc₄Fuc₃Neu5Ac₁ | C-FS |
| 4531 | 2410.888 | Hex₄HexNAc₅Fuc₃Neu5Ac₁ | C-FS |
| 4541 | 2556.946 | Hex₄HexNAc₅Fuc₄Neu5Ac₁ | C-FS |
| 4600 | 1884.698 | Hex₄HexNAc₆ | C |
| 4601 | 2175.794 | Hex₄HexNAc₆Neu5Ac₁ | C-S |
| 4610 | 2030.756 | Hex₄HexNAc₆Fuc₁ | C-F |
| 4611 | 2321.851 | Hex₄HexNAc₆Fuc₁Neu5Ac₁ | C-FS |
| 4620 | 2176.814 | Hex₄HexNAc₆Fuc₂ | C-F |
| 4621 | 2467.909 | Hex₄HexNAc₆Fuc₂Neu5Ac₁ | C-FS |
| 4630 | 2322.872 | Hex₄HexNAc₆Fuc₃ | C-F |
| 4641 | 2760.025 | Hex₄HexNAc₆Fuc₄Neu5Ac₁ | C-FS |
| 4650 | 2614.988 | Hex₄HexNAc₆Fuc₅ | C-F |
| 4700 | 2087.778 | Hex₄HexNAc₇ | C |
| 4701 | 2378.873 | Hex₄HexNAc₇Neu5Ac₁ | C-S |
| 4710 | 2233.835 | Hex₄HexNAc₇Fuc₁ | C-F |
| 4711 | 2524.931 | Hex₄HexNAc₇Fuc₁Neu5Ac₁ | C-FS |
| 4720 | 2379.893 | Hex₄HexNAc₇Fuc₂ | C-F |
| 4730 | 2525.951 | Hex₄HexNAc₇Fuc₃ | C-F |
| 5200 | | | |
| 5200 | | | |
| 5210 | 1380.491 | GlcNAc₂MansFuc₁ | HM-F |
| 5300 | 1437.513 | Hex₅HexNAc₃ | H |
| 5300 | | | |
| 5301 | 1728.608 | Hex₅HexNAc₃Neu5Ac₁ | H-S |
| 5301 | | | |
| 5310 | 1583.571 | Hex₅HexNAc₃Fuc₁ | H-F |
| 5310 | | | |
| 5311 | 1874.666 | Hex₅HexNAc₃Fuc₁Neu5Ac₁ | H-FS |
| 5311 | | | |
| 5320 | 1729.629 | Hex₅HexNAc₃Fuc₂ | H-F |
| 5320 | | | |
| 5400 | | | |
| 5401 | | | |
| 5401 | | | |
| 5402 | | | |
| 5410 | | | |
| 5411 | | Hex₅HexNAc₄Fuc₁Neu5Ac₁ | C-FS |
| 5411 | | | |
| 5412 | | | |
| 5420 | | | |
| 5421 | | | |
| 5430 | | | |
| 5431 | 2369.861 | Hex₅HexNAc₄Fuc₃Neu5Ac₁ | C/H-FS |
| 5432 | 2660.957 | Hex₅HexNAc₄Fuc₃Neu5Ac₂ | C-FS |
| 5432 | 2660.957 | Hex₅HexNAc₄Fuc₃Neu5Ac₂ | C-FS |
| 5531 | 2572.941 | Hex₅HexNAc₅Fuc₃Neu5Ac₁ | C/H-FS |
| 5541 | 2718.999 | Hex₅HexNAc₅Fuc₄Neu5Ac₁ | C-FS |
| 5631 | 2776.020 | Hex₅HexNAc₆Fuc₃Neu5Ac₁ | C-FS |
| 5650 | 2777.040 | Hex₅HexNAc₆Fuc₅ | C-F |
| 5700 | 2249.830 | Hex₅HexNAc₇ | C |
| 5701 | 2540.926 | Hex₅HexNAc₇Neu5Ac₁ | C-S |
| 5702 | 2832.021 | Hex₅HexNAc₇Neu5Ac₂ | C-S |
| 5710 | 2395.888 | Hex₅HexNAc₇Fuc₁ | C-F |
| 5711 | 2686.984 | Hex₅HexNAc₇Fuc₁Neu5Ac₁ | C-FS |
| 5712 | 2978.079 | Hex₅HexNAc₇Fuc₁Neu5Ac₂ | C-FS |
| 5720 | 2541.946 | Hex₅HexNAc₇Fuc₂ | C-F |
| 5721 | 2833.042 | Hex₅HexNAc₇Fuc₂Neu5Ac₁ | C-FS |
| 5730 | 2688.004 | Hex₅HexNAc₇Fuc₃ | C-F |
| 5730 | 2688.004 | Hex₅HexNAc₇Fuc₃ | C-F |
| 5731 | 2979.099 | Hex₅HexNAc₇Fuc₃Neu5Ac₁ | C-FS |
| 6200 | | | |
| 6200 | | | |
| 6210 | 1542.544 | GlcNAc₂Man₆Fuc₁ | HM-F |
| 6300 | 1599.566 | Hex₆HexNAc₃ | H |
| 6300 | | | |
| 6301 | 1890.661 | Hex₆HexNAc₃Neu5Ac₁ | H-S |
| 6301 | | | |
| 6310 | 1745.623 | Hex₆HexNAc₃Fuc₁ | H-F |
| 6310 | | | |
| 6311 | 2036.719 | Hex₆HexNAc₃Fuc₁Neu5Ac₁ | H-FS |
| 6311 | 2036.719 | Hex₆HexNAc₃Fuc₁Neu5Ac₁ | H-FS |
| 6311 | | | |
| 6320 | 1891.681 | Hex₆HexNAc₃Fuc₂ | H-F |
| 6400 | 1802.645 | Hex₆HexNAc₄ | H |
| 6401 | 2093.740 | Hex₆HexNAc₄Neu5Ac₁ | H-S |
| 6401 | | | |
| 6402 | 2384.836 | Hex₆HexNAc₄Neu5Ac₂ | H-S |
| 6410 | 1948.703 | Hex₆HexNAc₄Fuc₁ | H-F |
| 6410 | | | |
| 6411 | 2239.798 | Hex₆HexNAc₄Fuc₁Neu5Ac₁ | H-FS |
| 6421 | 2385.856 | Hex₆HexNAc₄Fuc₂Neu5Ac₁ | H-FS |
| 6432 | 2823.009 | Hex₆HexNAc₄Fuc₃Neu5Ac₂ | H-FS |
| 6500 | 2005.724 | Hex₆HexNAc₅ | C/H |
| 6500 | | | |
| 6501 | 2296.820 | Hex₆HexNAc₅Neu5Ac₁ | C/H-S |
| 6501 | | | |
| 6502 | 2587.915 | Hex₆HexNAc₅Neu5Ac₂ | C/H-S |
| 6503 | 2879.011 | Hex₆HexNAc₅Neu5Ac₃ | C-S |
| 6510 | 2151.782 | Hex₆HexNAc₅Fuc₁ | C/H-F |
| 6510 | | | |
| 6511 | 2442.878 | Hex₆HexNAc₅Fuc₁Neu5Ac₁ | C/H-FS |
| 6511 | 2442.878 | Hex₆HexNAc₅Fuc₁Neu5Ac₁ | C/H-FS |
| 6511 | | | |
| 6512 | 2733.973 | Hex₆HexNAc₅Fuc₁Neu5Ac₂ | C/H-FS |
| 6513 | 3025.068 | Hex₆HexNAc₅Fuc₁Neu5Ac₃ | C-FS |
| 6520 | | | |
| 6521 | 2588.936 | Hex₆HexNAc₅Fuc₂Neu5Ac₁ | C/H-FS |
| 6522 | 2880.031 | Hex₆HexNAc₅Fuc₂Neu5Ac₂ | C/H-FS |
| 6530 | 2443.898 | Hex₆HexNAc₅Fuc₃ | C/H-F |
| 6530 | 2879.011 | Hex₆HexNAc₅Neu5Ac₃ | C-S |
| 6531 | 2734.993 | Hex₆HexNAc₅Fuc₃Neu5Ac₁ | C/H-FS |
| 6532 | 3026.089 | Hex₆HexNAc₅Fuc₃Neu5Ac₂ | C/H-FS |
| 6603 | 3082.090 | Hex₆HexNAc₆Neu5Ac₃ | C-S |
| 6623 | 3374.206 | Hex₆HexNAc₆Fuc₂Neu5Ac₃ | C-FS |
| 6630 | 3082.090 | Hex₆HexNAc₆Neu5Ac₃ | C-S |
| 6631 | 2938.073 | Hex₆HexNAc₆Fuc₃Neu5Ac₁ | C-FS |
| 6632 | 3229.168 | Hex₆HexNAc₆Fuc₃Neu5Ac₂ | C-FS |
| 6641 | 3084.131 | Hex₆HexNAc₆Fuc₄Neu5Ac₁ | C-FS |
| 6642 | 3375.226 | Hex₆HexNAc₆Fuc₄Neu5Ac₂ | C-FS |
| 6652 | 3521.284 | Hex₆HexNAc₆Fuc₄Neu5Ac₂ | C-FS |
| 6713 | 3431.227 | Hex₆HexNAc₇Fuc₁Neu5Ac₃ | C-FS |
| 6731 | 3141.152 | Hex₆HexNAc₇Fuc₃Neu5Ac₁ | C-FS |
| 6740 | 2996.115 | Hex₆HexNAc₇Fuc₄ | C-F |
| 7200 | 1558.539 | GlcNAc₂Man₇ | HM |
| 7200 | | | |
| 7200 | | | |
| 7210 | 1704.597 | GlcNAc₂Man₇Fuc₁ | HM-F |
| 7400 | 1964.698 | Hex₇HexNAc₄ | H |
| 7400 | | | |
| 7401 | 2255.793 | Hex₇HexNAc₄Neu5Ac₁ | H-S |
| 7410 | 2110.756 | Hex₇HexNAc₄Fuc₁ | H-F |
| 7411 | 2401.851 | Hex₇HexNAc₄Fuc₁Neu5Ac₁ | H-FS |
| 7412 | 2692.946 | Hex₇HexNAc₄Fuc₁Neu5Ac₂ | H-FS |
| 7420 | 2256.814 | Hex₇HexNAc₄Fuc₂ | H-F |
| 7421 | 2547.909 | Hex₇HexNAc₄Fuc₂Neu5Ac₁ | H-FS |
| 7430 | 2402.871 | Hex₇HexNAc₄Fuc₃ | H-F |
| 7431 | 2693.967 | Hex₇HexNAc₄Fuc₃Neu5Ac₁ | H-FS |
| 7432 | 2985.062 | Hex₇HexNAc₄Fuc₃Neu5Ac₂ | H-FS |
| 7500 | 2167.777 | Hex₇HexNAc₅ | H |
| 7500 | 2167.777 | Hex₇HexNAc₅ | H |
| 7511 | 2604.930 | Hex₇HexNAc₅Fuc₁Neu5Ac₁ | H-FS |
| 7512 | 2896.026 | Hex₇HexNAc₅Fuc₁Neu5Ac₂ | H-FS |
| 7601 | 2661.952 | Hex₇HexNAc₆Neu5Ac₁ | C-S |
| 7602 | 2953.047 | Hex₇HexNAc₆Neu5Ac₂ | C-S |
| 7610 | 2516.914 | Hex₇HexNAc₆Fuc₁ | C-F |
| 7610 | | | |
| 7611 | 2808.010 | Hex₇HexNAc₆Fuc₁Neu5Ac₁ | C-FS |
| 7611 | | | |
| 7612 | 3099.105 | Hex₇HexNAc₆Fuc₁Neu5Ac₂ | C-FS |
| 7613 | 3390.201 | Hex₇HexNAc₆Fuc₁Neu5Ac₂ | C-FS |
| 7620 | 2662.972 | Hex₇HexNAc₆Fuc₂ | C-F |
| 7621 | 2954.068 | Hex₇HexNAc₆Fuc₂Neu5Ac₁ | C-FS |
| 7640 | 2955.088 | Hex₇HexNAc₆Fuc₄ | C-F |
| 7713 | 3593.280 | Hex₇HexNAc₇Fuc₁Neu5Ac₃ | C-FS |
| 7731 | 3303.205 | Hex₇HexNAc₇Fuc₃Neu5Ac₁ | C-FS |
| 7740 | 3158.168 | Hex₇HexNAc₇Fuc₄ | C-F |
| 7741 | 3449.263 | Hex₇HexNAc₇Fuc₄Neu5Ac₁ | C-FS |
| 8200 | 1720.592 | GlcNAc₂Man₈ | HM |
| 8200 | | GlcNAc₂Man₈ | |
| 8200 | | | |
| 9200 | 1882.645 | GlcNAc₂Man₉ | HM |
| 9200 | | GlcNAc₂Man₉ | |
| 9200 | | | |
| 9210 | 2028.702 | GlcNAc₂Man₉Fuc₁ | HM-F |
| 9210 | 2028.702 | GlcNAc₂Man₉Fuc₁ | HM-F |
| 10200 | 2044.697 | GlcNAc₂Man₁₀ | HM |
| 10200 | | | |
| 11200 | | | |

### Example 3 - CA 125 ELISA

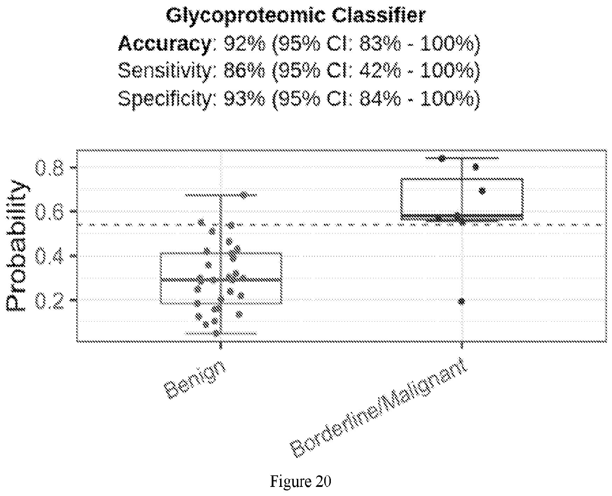

This Example refers to Figure 20.

An protein CA 125 (cancer antigen 125) enzyme-linked immunosorbent assay (ELISA) was performed on patient samples. The patient pool consisted of the first n=41 enrolled women from InterVenn's prospective trial to detect ovarian cancer malignancy (VOCAL). There were n=12 women with borderline or malignant cancer, as well as n=29 women with benign pelvic masses, representing the exact patient population that would be targeted in the clinical setting. Models were trained on previously purchased retrospective samples from commercial biobanks, and applied blind to the VOCAL trial participants.

The results of the ELISA assay are shown in Figure 20.

At a Cutoff = 35; the ELISA assay was observed to diagnose malignant ovarian cancer at the following levels of accuracy, sensitivity and specificity:

| | | |
|---|---|---|
| Accuracy = 56% | Sensitivity = 86% | Specificity = 48% |

The samples had a positive predictive value at 20% Prevalence = 29%
The samples had a negative predictive value at 20% Prevalence = 93%

There are approximately 22,000 new cases of ovarian cancer in the United States every year, which stem from approximately 110,000 pelvic masses (at 20% prevalence). As observed the CA-125 test would correctly identify 18,920 of the malignant cancers and 42,240 of the benign cancers. There would be 45,760 false positives and 3080 false negatives.

### Example 4 - Glycoproteomic Trained Model Test

This Example refers to Figure 21.

A model trained using SEQ ID NOs.: 1-76 was to identify the probability that a given patient sample had ovarian cancer.

The patient pool consisted of the first n=41 enrolled women from InterVenn's prospective trial to detect ovarian cancer malignancy (VOCAL). There were n=12 women with borderline or malignant cancer, as well as n=29 women with benign pelvic masses, representing the exact patient population that would be targeted in the clinical setting. Models were trained on previously purchased retrospective samples from commercial biobanks, and applied blind to the VOCAL trial participants.

The results are shown in Figure 21.

At a Cutoff = 0.54; the model was observed to diagnose malignant ovarian cancer at the following levels of accuracy, sensitivity and specificity:

| | | |
|---|---|---|
| Accuracy = 92% | Sensitivity = 86% | Specificity = 93% |

The samples had a positive predictive value at 20% Prevalence = 75.0%.
The samples had a negative predictive value at 20% Prevalence = 96%.

There are approximately 22,000 new cases of ovarian cancer in the United States every year, which stem from approximately 110,000 pelvic masses (at 20% prevalence).

The glycoproteomic test set forth in this Example 4 would correctly identify 18,920 of the malignant cancers and 81,840 of the benign cancers. There would be 6,160 false positives and 3,080 false negatives.

Compared with CA-125, and in the United States alone, this would amount to 39,600 less incorrect cancer diagnoses per year. These additional correctly-diagnosed patients would all be triaged to the appropriate surgery and surgeon, where they would not have been with the CA-125 test. This results in significantly less stress on patients, as well as on the gynecologic oncologists required to perform surgeries on predicted malignancies.

The embodiments and examples described above are intended to be merely illustrative and non-limiting. Those skilled in the art will recognize or will be able to ascertain using no more than routine experimentation, numerous equivalents of specific compounds, materials and procedures.

## Claims

1. A method for diagnosing a patient having ovarian cancer; the method comprising:
performing mass spectroscopy of a biological sample that has been obtained from the patient, using MRM-MS with a QQQ and/or qTOF spectrometer to detect MRM transitions selected from the group consisting of transitions 1, 2 or 3, wherein each MRM transition is indicative of a corresponding alpha-1-antitrypsin (A1AT) glycopeptide in Table 1, wherein the analyzing the detected glycopeptides comprises using a machine learning algorithm;
inputting the quantification of the MRM transitions into a trained model to generate an output probability,
determining if the output probability is above or below a threshold for a classification; and
identifying a diagnostic classification for the patient based on whether the output probability is above or below a threshold for a classification; and
diagnosing the patient as having ovarian cancer based on the diagnostic classification.

2. The method of claim 1, wherein the method includes digesting a glycopeptide in the sample to provide a glycopeptide consisting essentially of an amino acid sequence selected from the group consisting of SEQ ID NOs: 1-3.

3. The method of any of claim 1 or claim 2 wherein the biological sample is contacted with one or more enzymes, optionally wherein the enzymes are:
(a) lipases; or
(b) proteases, for example serine proteases, optionally wherein the enzyme is selected from the group consisting of trypsin, chymotrypsin, thrombin, elastase, and subtilisin; preferably wherein the enzyme is trypsin.

4. The method of any one of claims 1-3, wherein the MRM transition is detected using a QQQ mass spectrometer.

5. The method of any one of claims 1-4, wherein the method comprises a coupled chromatography procedure which comprises: performing or effectuating a liquid chromatography-mass spectrometry (LC-MS) operation.

6. The method of any one of claims 1-5, wherein the biological sample is:
(a) a plasma sample; or
(b) a serum sample.

7. The method of any one of claims 1-6, wherein:
(a) the MRM transition is indicative of a glycopeptide consisting essentially of an amino acid sequence of SEQ ID NO: 1;
(b) the MRM transition is indicative of a glycopeptide consisting essentially of an amino acid sequence of SEQ ID NO: 2;
(c) the MRM transition is indicative of a glycopeptide consisting essentially of an amino acid sequence of SEQ ID NO: 3.

8. The method of any one of claims 1-7, further comprising quantifying relative abundance of a glycan and/or a peptide.

9. The method of any one of claims 1-8, comprising normalizing the amount of glycopeptide based on the amount of peptide or glycopeptide consisting essentially of an amino acid having SEQ ID NOs:1-3 and combinations thereof and comparing that quantification to the amount of (i) chemical species or (ii) another glycopeptide consisting of an amino acid sequence selected from the group consisting of SEQ ID NOs: 1-3.

10. The method of any one of claims 1-9, wherein the patient has a pelvic mass.

## Patentansprüche

1. Verfahren zur Diagnose einer Patientin mit Eierstockkrebs, wobei das Verfahren umfasst:
Durchführen einer Massenspektroskopie einer biologischen Probe, die von der Patientin gewonnen wurde, unter Verwendung von MRM-MS mit einem QQQ- und/oder qTOF-Spektrometer, um MRM-Übergänge zu detektieren, die aus der Gruppe ausgewählt sind, die aus den Übergängen 1, 2 oder 3 ausgewählt sind, wobei jeder MRM-Übergang ein entsprechendes Alpha-I-Antitrypsin (AIAT)-Glykopeptid aus Tabelle 1 anzeigt, wobei das Analysieren der nachgewiesenen Glykopeptide die Verwendung eines Algorithmus für maschinelles Lernen umfasst;
Eingeben der Quantifizierung der MRM-Übergänge in ein trainiertes Modell, um eine Ausgabewahrscheinlichkeit zu erzeugen,
Bestimmen, ob die Ausgabewahrscheinlichkeit über oder unter einem Schwellenwert für eine Klassifizierung liegt; und
Identifizieren einer diagnostischen Klassifizierung für die Patientin, basierend darauf, ob die Ausgabewahrscheinlichkeit über oder unter einem Schwellenwert für eine Klassifizierung liegt; und
Diagnostizieren, dass die Patientin an Eierstockkrebs leidet, basierend auf der diagnostischen Klassifizierung.

2. Verfahren nach Anspruch 1, wobei das Verfahren das Verdauen eines Glykopeptids in der Probe umfasst, um ein Glykopeptid bereitzustellen, das im Wesentlichen aus einer Aminosäuresequenz besteht, die aus der Gruppe ausgewählt ist, die aus SEQ ID NOs: 1-3 besteht.

3. Verfahren nach Anspruch 1 oder 2, wobei die biologische Probe mit einem oder mehreren Enzymen in Kontakt gebracht wird, wobei die Enzyme optional
(a) Lipasen; oder
(b) Proteasen, beispielsweise Serinproteasen, wobei das Enzym optional aus der Gruppe ausgewählt ist, die aus Trypsin, Chymotrypsin, Thrombin, Elastase und Subtilisin besteht; wobei das Enzym vorzugsweise Trypsin ist.

4. Verfahren nach einem der Ansprüche 1 bis 3, wobei der MRM-Übergang unter Verwendung eines QQQ-Massenspektrometers nachgewiesen wird.

5. Verfahren nach einem der Ansprüche 1 bis 4, wobei das Verfahren ein gekoppeltes Chromatographieverfahren umfasst, das Folgendes umfasst: Durchführen oder Bewirken einer Flüssigchromatographie-Massenspektrometrie (LC-MS)-Operation.

6. Verfahren nach einem der Ansprüche 1 bis 5, wobei die biologische Probe
(a) eine Plasmaprobe; oder
(b) eine Serumprobe ist.

7. Verfahren nach einem der Ansprüche 1 bis 6, wobei
(a) der MRM-Übergang ein Glykopeptid anzeigt, das im Wesentlichen aus einer Aminosäuresequenz von SEQ ID NO: 1 besteht;
(b) der MRM-Übergang ein Glykopeptid anzeigt, das im Wesentlichen aus einer Aminosäuresequenz von SEQ ID NO: 2 besteht;
(c) der MRM-Übergang ein Glykopeptid anzeigt, das im Wesentlichen aus einer Aminosäuresequenz von SEQ ID NO: 3 besteht.

8. Verfahren nach einem der Ansprüche 1 bis 7, das ferner das Quantifizieren der relativen Häufigkeit eines Glykans und/oder eines Peptids umfasst.

9. Verfahren nach einem der Ansprüche 1 bis 8, umfassend das Normalisieren der Menge an Glykopeptid auf der Grundlage der Menge an Peptid oder Glykopeptid, das im Wesentlichen aus einer Aminosäure mit den SEQ ID NOs: 1 bis 3 und Kombinationen davon besteht, und das Vergleichen dieser Quantifizierung mit der Menge an (i) chemischen Spezies oder (ii) einem anderen Glykopeptid, das aus einer Aminosäuresequenz besteht, die aus der Gruppe der SEQ ID NOs:1-3 besteht.

10. Verfahren nach einem der Ansprüche 1 bis 9, wobei die Patientin eine Raumforderung im Becken aufweist.

## Revendications

1. Procédé de diagnostic d'une patiente atteinte d'un cancer de l'ovaire, ledit procédé comprenant :
la réalisation d'une spectroscopie de masse sur un échantillon biologique prélevé chez la patiente, en utilisant la MRM-MS avec un spectromètre QQQ et/ou qTOF pour détecter les transitions MRM choisies dans le groupe constitué par les transitions 1, 2 ou 3, où chaque transition MRM est indicative d'un glycopeptide alpha-1-antitrypsine (A1AT) correspondant dans le Tableau 1, l'analyse des glycopeptides détectés comprenant l'utilisation d'un algorithme d'apprentissage automatique ;
l'entrée de la quantification des transitions MRM dans un modèle entraîné pour générer une probabilité de sortie ;
la détermination pour savoir si la probabilité de sortie est supérieure ou inférieure à un seuil pour une classification, et
l'identification d'une classification diagnostique pour la patiente en fonction de la probabilité de sortie qui va être supérieure ou inférieure à un seuil pour une classification ; et
le diagnostic de la patiente comme ayant un cancer de l'ovaire sur la base de la classification diagnostique.

2. Procédé selon la revendication 1, ledit procédé incluant la digestion d'un glycopeptide dans l'échantillon pour obtenir un glycopeptide constitué essentiellement d'une séquence d'acides aminés choisie dans le groupe constitué par les SEQ ID NO : 1-3.

3. Procédé selon l'une quelconque des revendications 1 et 2, dans lequel l'échantillon biologique est mis en contact avec une ou plusieurs enzymes, où les enzymes sont éventuellement :
(a) des lipases ; ou
(b) des protéases, par exemple, des sérine protéases, où éventuellement, l'enzyme est choisie dans le groupe constitué par la trypsine, la chymotrypsine, la thrombine, l'élastase et la subtilisine ; de préférence, où l'enzyme est la trypsine.

4. Procédé selon l'une quelconque des revendications 1-3, dans lequel la transition MRM est détectée à l'aide d'un spectromètre de masse QQQ.

5. Procédé selon l'une quelconque des revendications 1-4, ledit procédé comprenant une procédure de chromatographie couplée qui comprend : la réalisation ou l'exécution d'une opération de chromatographie en phase liquide couplée à la spectrométrie de masse (LC-MS).

6. Procédé selon l'une quelconque des revendications 1-5, dans lequel l'échantillon biologique est :
(a) un échantillon de plasma ; ou
(b) un échantillon de sérum.

7. Procédé selon l'une quelconque des revendications 1-6, dans lequel
(a) la transition MRM est indicative d'un glycopeptide constitué essentiellement d'une séquence d'acides aminés de SEQ ID NO : 1 ;
(b) la transition MRM est indicative d'un glycopeptide constitué essentiellement d'une séquence d'acides aminés de SEQ ID NO : 2 ;
(c) la transition MRM est indicative d'un glycopeptide constitué essentiellement d'une séquence d'acides aminés de SEQ ID NO : 3.

8. Procédé selon l'une quelconque des revendications 1-7, comprenant en outre la quantification de l'abondance relative d'un glycanne et/ou d'un peptide.

9. Procédé selon l'une quelconque des revendications 1-8, comprenant la normalisation de la quantité de glycopeptide sur la base de la quantité de peptide ou de glycopeptide constitué essentiellement d'une séquence d'acides aminés ayant SEQ ID NO : 1-3 et leurs combinaisons, et la comparaison de cette quantification à la quantité (i) d'espèce chimique ou (ii) d'un autre glycopeptide constitué d'une séquence d'acides aminés choisie dans le groupe constitué par les SEQ ID NO : 1-3.

10. Procédé selon l'une quelconque des revendications 1-9, où la patiente présente une masse pelvienne.
